(19) Europäisches Patentamt
European Patent Office
Office européen des brevets

(11) EP 2 382 325 B1

(12) **EUROPEAN PATENT SPECIFICATION**

(45) Date of publication and mention of the grant of the patent:
**19.08.2015 Bulletin 2015/34**

(21) Application number: **09803912.6**

(22) Date of filing: **22.12.2009**

(51) Int Cl.:
***C12Q 1/68*** *(2006.01)*

(86) International application number:
**PCT/IB2009/055909**

(87) International publication number:
**WO 2010/073218 (01.07.2010 Gazette 2010/26)**

(54) **METHYLATION BIOMARKERS FOR PREDICTING RELAPSE FREE SURVIVAL**

METHYLIERUNGSBIOMARKER ZUR VORHERSAGE EINES RÜCKFALLFREIEN ÜBERLEBENS

MARQUEURS BIOLOGIQUES DE MÉTHYLATION POUR PRÉDIRE LA SURVIE SANS RECHUTE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **23.12.2008 US 140272 P**

(43) Date of publication of application:
**02.11.2011 Bulletin 2011/44**

(73) Proprietors:
- **Koninklijke Philips N.V.**
  **5656 AE Eindhoven (NL)**
- **COLD SPRING HARBOR LABORATORY**
  **Cold Spring Harbor, NY 11724 (US)**

(72) Inventors:
- **KAMALAKARAN, Sitharthan**
  **NL-5656 AE Eindhoven (NL)**
- **VARADAN, Vinay**
  **NL-5656 AE Eindhoven (NL)**
- **HICKS, James, B.**
  **NL-5656 AE Eindhoven (NL)**

(74) Representative: **van Velzen, Maaike Mathilde**
**Philips**
**Intellectual Property & Standards**
**P.O. Box 220**
**5600 AE Eindhoven (NL)**

(56) References cited:
**EP-A1- 1 840 224** **EP-A2- 1 612 281**
**WO-A2-02/077272** **WO-A2-2008/083251**

- **BROCK MALCOLM V ET AL: "DNA methylation markers and early recurrence in stage I lung cancer." THE NEW ENGLAND JOURNAL OF MEDICINE 13 MAR 2008, vol. 358, no. 11, 13 March 2008 (2008-03-13), pages 1118-1128, XP002575979 ISSN: 1533-4406**
- **CUSTODIO ANA BELÉN ET AL: "Prognostic and predictive markers of benefit from adjuvant chemotherapy in early-stage non-small cell lung cancer." JOURNAL OF THORACIC ONCOLOGY : OFFICIAL PUBLICATION OF THE INTERNATIONAL ASSOCIATION FOR THE STUDY OF LUNG CANCER JUL 2009, vol. 4, no. 7, July 2009 (2009-07), pages 891-910, XP9131531 ISSN: 1556-1380**
- **TAKASHI ARAI ET AL: "Association of GSTP1 CpG Islands Hypermethylation with Poor Prognosis in Human Breast Cancers", BREAST CANCER RESEARCH AND TREATMENT, KLUWER ACADEMIC PUBLISHERS, BO, vol. 100, no. 2, 22 June 2006 (2006-06-22) , pages 169-176, XP019433517, ISSN: 1573-7217, DOI: 10.1007/S10549-006-9241-9**
- **CRISTOFANILLI ET AL: "3-36 Circulating Tumor-Specific DNA: A Marker for Monitoring Efficacy of Adjuvant Therapy in Cancer Patients", BREAST DISEASES: A YEAR BOOK QUARTERLY, MOSBY, ST. LOUIS, MO, US, vol. 16, no. 3, 1 October 2005 (2005-10-01), page 283, XP005209921, ISSN: 1043-321X, DOI: 10.1016/S1043-321X(05)80234-6**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 382 325 B1

## Description

FIELD OF THE INVENTION

This invention pertains in general to the field of statistical data processing.

More particularly the invention relates to methylation classification correlated to clinical pathological information, for indicating likelihood of recurrence of cancer.

BACKGROUND OF THE INVENTION

DNA methylation, a type of chemical modification of DNA that can be inherited and subsequently removed without changing the original DNA sequence, is the most well studied epigenetic mechanism of gene regulation. There are areas in DNA where a cytosine nucleotide occurs next to a guanine nucleotide in the linear sequence of bases called CpG islands.

It is known that DNA methylation of these islands, present in the promoter region, can act as a mechanism for gene silencing. Methods exist for experimentally finding the differential methylation, such as differential methylation hybridization, methylation specific sequencing, HELP assay, bisulfite sequencing, CpG island arrays etc.

CpG islands are generally heavily methylated in normal cells. However, during tumorigenesis, hypomethylation occurs at these islands, which may result in the expression of certain repeats. In addition, this hypomethylation correlates to DNA breaks and genome instability. These hypomethylation events also correlate to the severity of some cancers. Under certain circumstances, which may occur in pathologies such as cancer, imprinting, development, tissue specificity, or X chromosome inactivation, gene associated islands may be heavily methylated. Specifically, in cancer, methylation of islands proximal to tumor suppressors is a frequent event, often occurring when the second allele is lost by deletion (Loss of Heterozygosity, LOH). Some tumor suppressors commonly seen with methylated islands are p16, Rassf1a, BRCA1.

WO 2008/083251 discloses DNA methylation markers based on epigenetic stem cell signatures in cancer.

Arai, T. et al., (Breast Cancer Res Treat (2006) 100: 169-176), discloses Association of GSTP1 CpG islands hypermethylatin with poor prognosis in human breast cancers.

Cristofanilli, M. (Breast Diseases: A year book quarterly , Mosby, St. Louis, MO, US, Vol. 16, No. 3, 1 October 2005) discloses Circulating Tumor-Specific DNA. RASSFF1A is discloses as marker for monitoring efficacy of adjuvant therapy in cancer patients.

There are reported epigenetic markers for colorectal and prostate cancer. For example, Epigenomics AG (Berlin, Germany) has the Septin 9 as a marker for colorectal cancer screening in blood plasma. A method for using methylation sites to predict differential therapy responses in cancer and recommending an appropriate therapy has been disclosed in US20050021240A1. However, the results predicted by this method are limited.

Methods known within the art involves the use of immuno-histopathological variables such as tumor size, ER/PR status, lymph node negativity, etc. to define a clinical prognostic index such as the Nottingham Prognostic Index (NPI). The problem with such an index is that it has been shown to be very conservative, thus typically causing patients to receive aggressive therapy even when a low risk of disease recurrence exists.

An alternate method known within the art involves measurement of the expression levels of a large number of genes, typically around 70, and calculating a risk score based on the relative expression levels of the genes. These prognostic tests are not very specific and also remain very costly in terms of tissue handling requirements. Using RNA is difficult because RNA degrades much faster and needs more careful handling.

Hence, an improved method for obtaining statistically processed methylation data correlated to clinical pathological information would be advantageous and in particular a method allowing for increased flexibility, cost-effectiveness, and/or statistically correct prognosis data would be advantageous.

Accordingly, the present invention preferably seeks to mitigate, alleviate or eliminate one or more of the above-identified deficiencies in the art and disadvantages singly or in any combination and solves at least the above-mentioned problems by providing a method, and a device according to the appended patent claims.

A methylation classification list comprising loci DNA, for which loci the methylation status of the DNA is indicative of likelihood of recurrence of breast cancer, is provided. The methylation classification list comprises at least SEQ ID NO: 135.

An advantage of the methylation classification list is that it allows for clinical prognostic tests of breast cancer that could be widely used in clinical practice.

A method for obtaining a methylation classification list comprising statistically processed methylation data correlated to clinical pathological information is provided. The method comprises at least the steps of providing tumour DNA from breast cancer patients with a known clinical pathological history. Then, the methylation status of the tumour DNA is analyzed, resulting in a methylation classification list. The list comprises a selection of the statistically processed methylation data, wherein the selection is suitable for predicting probability of relapse free survival of a subject. This is advantageous, since DNA methylation may be much more easily measured in the clinical setting compared to data such as gene expression, thus enabling a highly useful clinical prognostic test. A further advantage is that clinicians are able to robustly stratify patients into good or poor prognostic groups and thus make appropriate therapy choices using the discovered DNA methylation markers.

The invention concerns a method for predicting probability of relapse free survival of a subject diagnosed with breast cancer, as defined in claim 1. The method comprises creating a marker panel comprising at least one post from the methylation classification list, providing DNA from the subject, analysing the methylation status of the parts of the DNA from the subject, corresponding to the marker panel. The result is a local methylation classification list, comprising statistically processed methylation data. The local methylation classification list is statistically analysed, which gives a predicted probability of relapse free survival for the subject.

In another aspect, an apparatus for predicting probability of relapse free survival of a subject, who has been diagnosed with cancer, as defined in claim 2, is provided. The apparatus comprises a first unit, creating a marker panel comprising at least SEQ ID NO: 135 in the methylation classification list. The apparatus also comprises a second unit, providing DNA from the subject and a third unit, analysing the methylation status of the parts of the DNA from the subject, corresponding to the marker panel. The output is a local methylation classification list comprising statistically processed methylation data. The apparatus further comprises a fourth unit, statistically analysing the local methylation classification list providing a predicted probability of relapse free survival for the subject. The units are operatively connected to each other.

Use of the methylation classification list, for predicting probability of relapse free survival of a subject diagnosed with breast cancer is disclosed.

BRIEF DESCRIPTION OF THE DRAWINGS

Other advantages of which the invention is capable of will be apparent and elucidated from the following description, reference being made to the accompanying drawings, in which

Fig. 1 is a schematic overview of a method ;

Fig. 2 is a schematic overview of a method ;

Fig. 3 is a block scheme of an apparatus ; and

Fig. 4 is showing example graphs of Kaplan-Meier curves.

DESCRIPTION OF EMBODIMENTS

Several embodiments of the present invention will be described in more detail below with reference to the accompanying drawings in order for those skilled in the art to be able to carry out the invention.

In Fig. 1, a method (10) for obtaining a methylation classification list (12) comprising statistically processed methylation data correlated to clinical pathological information is disclosed. The method comprises creating the methylation classification list (12), based on statistical analysis (120) of DNA (11) provided (110) from tumours of cancer patients with a known clinical pathological history. The tumours may e.g. be 89 tumours, wherein 83 of which have associated clinical pathological records such as relapse incidentals or survival data, for an extended period of time, e.g. 10 years. The method will be described in further detail below.

In an embodiment according to Fig. 2, a method (20) for predicting probability of relapse free survival of a subject diagnosed with cancer is provided. The method comprises the following steps. First, a marker panel (23) is created (230). The marker panel (23) comprises at least one post from the methylation classification list (12). Then, DNA (24) is provided (240) from the subject. The methylation status of the parts of the DNA (24) from the subject, corresponding to the marker panel (23) is analyzed (250) resulting in a local methylation classification list (25) comprising statistically processed methylation data. Next, the local methylation classification list (25) is statistically analysed (260), thus giving a predicted probability (26) of relapse free survival for the subject.

Based on the methylation classification list, a marker panel is created by selecting at least one post from the methylation classification list. The selection of loci for the classification is based on the Kaplan-Meier Survivial estimate that is detailed below. In order to select the particular loci for the test from the table, a variety of criteria are used, such as P-value of the difference between methylation status and the likelihood of relapse. Tope performing loci are preferred;

Combination of two loci can be made by accounting for synergy between two loci in making a better prediction of relapse than single loci alone;

Performance and ease of methylation assay will be taken in to account in choosing one loci over the other; and

Other information such as tumor grade or size can be put into the classification scheme, but are not present in the table.

Next, DNA is provided, i.e. by performing extraction from the subject, e.g. from blood, tissue, urine, saliva etc. Extraction is performed according to methods well known to a person skilled in the art, such as ethanol precipitation or by using a DNeasy Blood & Tissue Kit from Qiagen. This results in subject DNA.

Then, the methylation status of each sequence of subject DNA, corresponding to the sequences in the marker panel is analysed using a method well known to the skilled artisan, such as differential methylation hybridization, methylation specific sequencing, HELP assay, bisulphite sequencing, or using a CpG island microarray. The result is a methylation list.

The methylation list is compared to the marker panel and the posts in the methylation list matching posts in the marker panel are selected. The methylation status of the selected posts, i.e. DNA sequences, is checked using a local methylation classification, further described below, thus creating a local methylation classification list. The local methylation classification list is then subject to a diagnostic multivariate analysis, further described below. The result of the multivariate analysis is a predicted probability of relapse free survival for the subject.

METHYLATION CLASSIFICATION

In order to find the loci with highest prognosis potential, a methylation classification list is constructed in the following manner. Extraction of DNA is performed according to methods well known to a person skilled in the art, such as ethanol precipitation or by using a DNeasy Blood & Tissue Kit from Qiagen. This results in classification DNA.

The methylation status of each sequence of classification DNA, each locus, is decided using a method well known to the skilled artisan, such as differential methylation hybridization, methylation specific sequencing, HELP assay, bisulphite sequencing, or using a CpG island microarray. The resulting methylation list, based on the classification DNA, is subject to methylation classification.

The methylation classification is performed with the Kaplan-Meier estimator of the survival function, as described below.

Of the 159,436 loci resulting from the 89 tumours, each locus is sorted binary, i.e. associated to a good or a bad prognosis. This is done by first classifying the methylation status of the specific locus as non-methylated, partially methylated or methylated. These three possible states of the locus correspond to three possible groupings of subjects.

The Kaplan-Meier estimator, well known to a person skilled in the art, uses the time to relapse for each patient within the above groupings and calculates the survival probability, *S(t),* which is, the probability that a patient within the grouping would survive without a relapse for a given length of time. Assuming there were N patients in a specific grouping and the observed time to recurrence for each of the N samples was:

$$t_1 \leq t_2 \leq t_3 \ldots \leq t_N .$$

Corresponding to each time $t_i$ is $n_i$, the number of patients at risk of relapse just prior to $t_i$, and $d_i$, the number of patients who experienced relapse at time $t_i$. The Kaplan-Meier survival function is then defined as:

$$S(t) = \prod_{t_i \leq t} \frac{n_i - d_i}{n_i}$$

This Kaplan-Meier estimator is used to derive the recurrence-free survival function for each of the three groupings defined by each methylation locus. These survival functions, when plotted against time, give us survival curves. The survival curve has time on the x-axis and probability of recurrence-free survival on the y-axis. Thus, one survival curve is drawn for each grouping generated using the methylation status of a particular locus.

Fig. 4 is showing example graphs of Kaplan-Meier curves. Fig. 4A is an example of a graph with Topol 144777, Fig. 4B is an example of a graph with JMJD2C 67675, Fig. 4C is an example of a graph with DLG1 31375 and Fig. 4D is an example of a graph with Goosecoid 103370. The top curve in each graph represents methylation status 0 and the bottom curve represents methylation status -1. The Kaplan-Meier survival curve has time measured in months on the x-axis and probability of recurrence-free survival on the y-axis. Each patient stratification group is represented by one Kaplan-Meier curve, which captures the rate at which patients in this group tend to relapse. Thus, patient group represented by a curve that falls steeply suggests that patients in this group are at high risk for relapse, whereas patients that are in a group with a relatively flat curve are at lower risk of relapse. Given any two Kaplan-Meier curves, we can interpret differences in the curves at any given time to estimate the difference in risk of relapse for patients in the two groups. Again, the lower the value of a Kaplan-Meier curve at any given time, suggests a higher risk of relapse for patients belonging to the group represented by the curve.

We then check for statistically significant differences between the three Kaplan-Meier survival curves for each locus using the log-rank or Mantel-Haenszel test of the difference in Kaplan-Meier curves. The log-rank test statistic compares estimates of the survival functions of any two groups at each observed event time. It is constructed by computing the observed and expected number of events in one of the groups at each observed event time and then adding these to obtain an overall summary across all time points where there is an event. Let $j = 1,..,. J$ be the distinct times of observed relapse of cancer in any group. For each time, $j$, let $N_{1j}$ and $N_{2j}$ be the number of patients at risk of relapse in each group respectively. Let $N_j = N_{1j} + N_{2j}$. Let $O_{1j}$ and $O_{2j}$ be the number of relapses in the groups at time $j$ respectively, and $O_j = O_{1j} + O_{2j}$. Given that $O_j$ events happened across both groups at time $j$, the null hypothesis that the grouping was purely random, would have a hyper geometric distribution with:

mean equal to

$$E_j = O_j \frac{N_{1j}}{N_j}$$

and variance

$$V_j = \frac{O_j \left(N_{1j}/N_j\right)\left(1 - N_{1j}/N_j\right)\left(N_j - O_j\right)}{N_j - 1}$$

The logrank statistic then compares each $O_j$ to its expectation under the null hypothesis and is defined as:

$$Z = \frac{\sum_{j=1}^{J}\left(O_j - E_j\right)}{\sqrt{\sum_{j=1}^{J} V_j}}$$

The above Z-value can then be converted into a p-value, which is the probability that the survival functions are different purely by chance, by using the chi-squared statistic:

$$p = \Pr\left(\chi^2(1) \geq Z\right)$$

The p-value as calculated above gives the probability that the observed difference in the two survival curves is purely by chance. It is well known to a person skilled in the art that a p-value of 0.05 or lower is interpreted to suggest that one can be practically certain that the observed difference between the two curves is definitely not due to pure chance. This would suggest that any locus that achieves a p-value (statistical significance) of at least 0.05 or lower, is potentially a good biomarker for stratification of patients into good or poor prognosis groups. We evaluate all 159,436 loci in the above fashion. The loci with a statistical significance of at least 0.05 or lower are stored in a list, shown in table 1, along with their ability to stratify subjects into good or poor prognosis groups. The resulting methylation classification list is provided as SEQ ID NO: 1 to SEQ ID NO: 252. While the p-value is used as a means of including loci in the list, once a particular locus is included, the key elements are the survival curves associated with that locus. These survival curves provide the means to ascertain a patient's risk of relapse at any given point after initial diagnosis, and thus would be used in the embodiment of a diagnostic, as described in the diagnostic multivariate analysis section below.

*Table 1*. Statistically significant list of methylation loci that can individually stratify patients into good and bad prognosis groups.

| SEQ ID NO: | Chromosome | Start | End | P-value | Chi-square value | Gene | Gene Name |
|---|---|---|---|---|---|---|---|
| 1 | chr1 | 1064313 | 1064455 | 0,000386239 | 12,59761578 | AK128271 | Hypothetical protein FU46577. |
| 2 | chr1 | 1741929 | 1742020 | 0,000623577 | 11,70424305 | NADK | NAD kinase |
| 3 | chr1 | 3181325 | 3181447 | 5,19E-08 | 29,64604699 | | |
| 4 | chr1 | 20557064 | 20557187 | 0,000534876 | 11,98997202 | CaMKIINalpha/ BC020630 | calcium/calmodulin-dependent protein kinase II/PRO1489 (CaMKIINalpha protein). |
| 5 | chr1 | 21855222 | 21855381 | 2,12E-05 | 18,07960521 | CR619608/USP 48 | Ubiquitin specific protease 48./ubiquitin specific protease 48 |
| 6 | chr1 | 25318507 | 25318605 | 0,000458721 | 12,27640425 | Clorf63 | NPD014 protein isoform 2 |
| 7 | chr1 | 29625402 | 29625654 | 0,000252817 | 13,3911284 | | |
| 8 | chr1 | 38066662 | 38066799 | 3,63E-06 | 21,45255346 | INPP5B | inositol polyphosphate-5-phosphatase, 75kDa |
| 9 | chr1 | 56756288 | 56756461 | 0,000935047 | 10,95195838 | PPAP2B | phosphatidic acid phosphatase type 2B |
| 10 | chr1 | 65143624 | 65143729 | 0,000505234 | 12,09624827 | | |
| 11 | chr1 | 92062935 | 92063033 | 0,000853848 | 11,12036797 | TGFBR3 | transforming growth factor, beta receptor III |
| 12 | chr1 | 93009675 | 93009792 | 0,000371587 | 12,6699137 | AB208980 | MSTP030 (Ribosomal protein L5). |
| 13 | chr1 | 1,07E+08 | 107395391 | 0,000687924 | 11,52159376 | AB023193/NTN G1 | Splice isoform 2 of Q9Y2I2/netrin G1 |
| 14 | chr1 | 1,14E+08 | 113645473 | 4,65E-06 | 20,97835927 | MAGI3/MAGI3 | membrane-associated guanylate kinase-related 3/membrane-associated guanylate kinase-related 3 |
| 15 | chr1 | 1,42E+08 | 142421107 | 0,001050064 | 10,73714195 | PDE4DIP/PDE4 DIP | phosphodiesterase 4D interacting protein isoform/phosphodiesterase 4D interacting protein isoform |
| 16 | chr1 | 1,45E+08 | 145046154 | 0,000902258 | 11,01811403 | FU39739 | hypothetical protein LOC388685 |
| 17 | chr1 | 1,51E+08 | 150762785 | 5,87E-05 | 16,14555739 | JTB | jumping translocation breakpoint |
| 18 | chr1 | 1,58E+08 | 158230271 | 0,000330063 | 12,89158516 | | |
| 19 | chr1 | 1,58E+08 | 158239282 | 0,00030571 | 16,18574971 | | |
| 20 | chr1 | 1,73E+08 | 172908461 | 0,000248164 | 13,42597512 | RFWD2 | ring finger and WD repeat domain 2 isoform a |
| 21 | chr1 | 1,77E+08 | 176855380 | 0,000407058 | 12,49951524 | QSCN6 | quiescin Q6 isoform b |
| 22 | chr1 | 1,77E+08 | 176932542 | 0,000510124 | 12,07828732 | LHX4 | LIM homeobox 4 |

(continued)

| SEQ ID NO: | Chromosome | Start | End | P-value | Chi-square value | Gene | Gene Name |
|---|---|---|---|---|---|---|---|
| 23 | chr1 | 1,9E+08 | 189822582 | 0,000566915 | 11,881591 | HRPT2 | parafibromin |
| 24 | chr1 | 2,12E+08 | 211644651 | 0,00090527 | 11,01193681 | KCNK2 | potassium channel, subfamily K, member 2 |
| 25 | chr1 | 2,2E+08 | 220340980 | 0,000370567 | 12,67505329 | TP53BP2 | tumor protein p53 binding protein, 2 |
| 26 | chr1 | 2,22E+08 | 222376502 | 0,000513992 | 12,06420764 | KIAA0792 | hypothetical protein LOC9725 |
| 27 | chr1 | 2,23E+08 | 222616934 | 0,000982028 | 10,86114849 | | |
| 28 | chr1 | 2,23E+08 | 223434254 | 7,21E-05 | 15,75446713 | BC005171 | Chaperone, ABC1 activity of bc1 complex like (S. pombe). |
| 29 | chr1 | 2,33E+08 | 233093690 | 0,000722537 | 11,43035956 | MGC72083 | hypothetical protein LOC440736 |
| 30 | chr1 | 2,42E+08 | 242178360 | 0,001036209 | 10,76172264 | AK001019 | Hypothetical protein FU10157. |
| 31 | chr1 | 2,42E+08 | 242178493 | 0,000406559 | 12,50180374 | AK001019 | Hypothetical protein FU10157. |
| 32 | chr2 | 5781856 | 5781991 | 0,000192958 | 13,89844523 | SOX11 | SRY-box 11 |
| 33 | chr2 | 9298072 | 9298297 | 0,000451361 | 12,30659295 | DDEF2 | development and differentiation enhancing factor 2 |
| 34 | chr2 | 10213943 | 10214077 | 0,000147157 | 14,40824202 | RRM2 | ribonucleotide reductase M2 polypeptide |
| 35 | chr2 | 16033110 | 16033212 | 0,000631137 | 11,68182256 | MYCNOS/AF32 0053 | v-myc myelocytomatosis viral related oncogene, neuroblastoma derived (avian) opposite strand/N-MYC. |
| 36 | chr2 | 31717290 | 31717412 | 0,000972653 | 10,87891257 | | |
| 37 | chr2 | 32176570 | 32176759 | 0,000620444 | 11,71361603 | LOC84661 | dpy-30-like protein |
| 38 | chr2 | 37370187 | 37370286 | 0,000801102 | 11,23867769 | CEBPZ/PRO18 3 | CCAAT/enhancer binding protein zeta/hypothetical protein LOC55471 isoform 1 |
| 39 | chr2 | 38741040 | 38741184 | 0,000273705 | 13,24227999 | AY236962 | Stromal RNA regulating factor. |
| 40 | chr2 | 39014423 | 39014605 | 0,001010661 | 10,80793274 | BC060778/MO PT | DHX57 protein./protein containing single MORN motif in testis |
| 41 | chr2 | 46436609 | 46436740 | 1,79E-05 | 18,39971876 | BC051338 | EPAS1 protein. |
| 42 | chr2 | 48756549 | 48756680 | 0,000700308 | 11,48843 | ALF/ALF | TFIIA-alpha/beta-like factor isoform 1/TFIIA-alpha/beta-like factor isoform 1 |
| 43 | chr2 | 73372031 | 73372177 | 3,98E-06 | 21,27457405 | CCT7/C2orf7 | chaperonin containing TCP1, subunit 7 isoform a/chromosome 2 open reading frame 7 |

(continued)

| SEQ ID NO: | Chromosome | Start | End | P-value | Chi-square value | Gene | Gene Name |
|---|---|---|---|---|---|---|---|
| 44 | chr2 | 95253332 | 95253453 | 9,06E-05 | 15,32328453 | CR749650 | Hypothetical protein DKFZp686D2168. |
| 45 | chr2 | 95614686 | 95614925 | 0,000718539 | 11,4406703 | AK024144 | Hypothetical protein FLJ14082. |
| 46 | chr2 | 96232849 | 96232973 | 0,000310083 | 13,00848366 | DUSP2/AF331843 | dual specificity phosphatase 2/Phosphatase. |
| 47 | chr2 | 99565510 | 99565712 | 0,000443709 | 12,33850961 | REV1L | REV1-like |
| 48 | chr2 | 1,06E+08 | 105820719 | 0,000309255 | 13,01349189 | NCK2 | NCK adaptor protein 2 isoform A |
| 49 | chr2 | 1,6E+08 | 160297839 | 0,000709552 | 11,46405687 | BAZ2B | bromodomain adjacent to zinc finger domain, 2B |
| 50 | chr2 | 1,61E+08 | 161089859 | 0,000241664 | 13,47576785 | RBMS1 | RNA binding motif, single stranded interacting protein 1 |
| 51 | chr2 | 2,4E+08 | 240059794 | 0,000602184 | 11,76921502 | BC039904 | HDAC4 protein. |
| 52 | chr3 | 5204530 | 5204673 | 9,03E-05 | 15,32960597 | EDEM1 | ER degradation enhancer, mannosidase alpha-like |
| 53 | chr3 | 12680778 | 12680878 | 0,000425083 | 12,41857971 | RAF1 | v-raf-1 murine leukemia viral oncogene homolog |
| 54 | chr3 | 14141364 | 14141481 | 0,000933138 | 10,95574521 | BC003125/CHCHD4 | Hypothetical protein FLJ14560./coiled-coil-helix-coiled-coil-helix domain |
| 55 | chr3 | 24511219 | 24511396 | 0,000551997 | 11,93126397 | THRB/THRB | thyroid hormone receptor, beta/thyroid hormone receptor, beta |
| 56 | chr3 | 26639582 | 26639712 | 0,000146625 | 14,41506532 | LRRC3B | leucine rich repeat containing 3B |
| 57 | chr3 | 33457722 | 33457849 | 0,000344073 | 12,81379493 | UBP1 | upstream binding protein 1 (LBP-1a) |
| 58 | chr3 | 39423133 | 39423352 | 0,000374847 | 12,65358155 | X15005/X1500 5 | 40S ribosomal protein SA(p40)(34/67 kDa laminin receptor) (Colon carcinoma laminin-binding protein) (NEM/1CHD4) (Multidrug resistance-associated protein MGr1-Ag)./40S ribosomal protein SA (p40)(34/67 kDa laminin receptor) (Colon carcinoma laminin-binding protein) (NEM/1CHD4) (Multidrug resistance-associated protein MGr1-Ag). |
| 59 | chr3 | 46012237 | 46012342 | 0,000236958 | 13,51267433 | FYCO1/FYCO1 | FYVE and coiled-coil domain containing 1/FYVE and coiled-coil domain containing 1 |
| 60 | chr3 | 46593412 | 46593530 | 2,53E-05 | 17,74017338 | TDGF1 | teratocarcinoma-derived growth factor 1 |
| 61 | chr3 | 48463194 | 48463299 | 4,78E-05 | 16,53330372 | TREX1/TREX1 | three prime repair exonuclease 1 isoform d/three prime repair exonuclease 1 isoform d |

(continued)

| SEQ ID NO: | Chromosome | Start | End | P-value | Chi-square value | Gene | Gene Name |
|---|---|---|---|---|---|---|---|
| 62 | chr3 | 49424481 | 49424611 | 0,000167272 | 14,16708091 | RHOA/TCTA/RHOA | ras homolog gene family, member A/T-cell leukemia translocation altered gene/ras homolog gene family, member A |
| 63 | chr3 | 49566612 | 49566744 | 0,000674555 | 11,55807856 | BSN | bassoon |
| 64 | chr3 | 57516816 | 57517032 | 0,000620841 | 11,7124272 | 2'-PDE | 2'-phosphodiesterase |
| 65 | chr3 | 61522701 | 61522802 | 0,00097199 | 10,88017587 | BC047734 | PTPRG protein. |
| 66 | chr3 | 1,03E+08 | 102763354 | 0,00013999 | 14,50227829 | BC035967 | FLJ20432 protein (HBV pre-S2 trans-regulated protein 2). |
| 67 | chr3 | 1,22E+08 | 121508929 | 0,000982227 | 10,8607719 | | |
| 68 | chr3 | 1,23E+08 | 123195137 | 0,000474985 | 12,2113969 | | |
| 69 | chr3 | 1,24E+08 | 123766114 | 0,00077556 | 11,29882892 | PARP9/BC039580/AY780792 | B aggressive lymphoma gene/Splice isoform 2 of Q8IXQ6/Rhysin 2. |
| 70 | chr3 | 1,24E+08 | 124115294 | 0,000158828 | 14,26455622 | | |
| 71 | chr3 | 1,29E+08 | 128806597 | 0,000650859 | 11,62458127 | | |
| 72 | chr3 | 1,3E+08 | 130362802 | 0,001055342 | 10,72786464 | KIAA1160/KIAA1160 | hypothetical protein LOC57461/hypothetica l protein LOC57461 |
| 73 | chr3 | 1,43E+08 | 142980179 | 0,000423578 | 12,42520484 | GRK7 | G-protein-coupled receptor kinase 7 |
| 74 | chr3 | 1,55E+08 | 155322572 | 0,000875873 | 11,07313919 | AB073386 | Hypothetical protein HMFN1864. |
| 75 | chr3 | 1,99E+08 | 198513786 | 6,60E-05 | 15,9229383 | DLG1/DLG1 | discs, large homolog 1 (Drosophila)/discs, large homolog 1 (Drosophila) |
| 76 | chr4 | 1774869 | 1775089 | 0,000427789 | 12,40672921 | | |
| 77 | chr4 | 1810318 | 1810481 | 0,000662831 | 11,59068096 | | |
| 78 | chr4 | 1,25E+08 | 124676421 | 1,14E-05 | 19,25579282 | SPRY1 | sprouty homolog 1, antagonist of FGF signaling |
| 79 | chr4 | 1,47E+08 | 147215955 | 0,000527391 | 12,01623602 | LOC152485 | hypothetical protein LOC152485 |
| 80 | chr4 | 1,75E+08 | 174629580 | 0,000780177 | 11,28780827 | HMGB2 | high-mobility group box 2 |

(continued)

| SEQ ID NO: | Chromosome | Start | End | P-value | Chi-square value | Gene | Gene Name |
|---|---|---|---|---|---|---|---|
| 81 | chr5 | 271188 | 271302 | 0,000127692 | 14,67554968 | LOC133957/BC041016/LOC133957 | hypothetical protein LOC133957/SDHA protein./hypothetical protein LOC133957 |
| 82 | chr5 | 1062804 | 1063000 | 2,18E-05 | 18,02734989 | NKD2 | naked cuticle homolog 2 |
| 83 | chr5 | 52320929 | 52321041 | 0,000817865 | 11,20024511 | ITGA2/ITGA2 | integrin alpha 2 precursor/integrin alpha 2 precursor |
| 84 | chr5 | 57791657 | 57791770 | 0,000582971 | 11,82958226 | PLK2 | polo-like kinase 2 |
| 85 | chr5 | 60031303 | 60031428 | 0,000759889 | 11,33673246 | BC019075 | Hypothetical protein FLJ10304(DEPDC1B protein). |
| 86 | chr5 | 70256621 | 70256723 | 0,000870432 | 11,08469342 | SMN2 | survival of motor neuron 2, centromeric isoform |
| 87 | chr5 | 72287001 | 72287092 | 0,000488126 | 12,16048738 | FCHO2 | FCH domain only 2 |
| 88 | chr5 | 1,3E+08 | 130358943 | 0,000667411 | 11,57787641 | | |
| 89 | chr5 | 1,34E+08 | 134210075 | 0,000335682 | 12,85999469 | FU37562 | hypothetical protein LOC134553 |
| 90 | chr5 | 1,38E+08 | 138117162 | 0,000214628 | 13,69847922 | BC000385/BC000385 | CTNNA1 protein./CTNNA1 protein. |
| 91 | chr5 | 1,73E+08 | 172598230 | 0,00075599 | 11,34628548 | NKX2-5 | NK2 transcription factor related, locus 5 |
| 92 | chr5 | 1,77E+08 | 176876872 | 0,000458614 | 12,27683927 | DDX41 | DEAD-box protein abstrakt |
| 93 | chr5 | 1,77E+08 | 177344133 | 0,000654362 | 11,61459616 | | |
| 94 | chr6 | 237511 | 237619 | 0,000356749 | 12,74611554 | DUSP22 | dual specificity phosphatase 22 |
| 95 | chr6 | 31964939 | 31965081 | 0,001020908 | 10,78925848 | | |
| 96 | chr6 | 34311500 | 34311607 | 0,000812493 | 11,21247403 | HMGA1 | high mobility group AT-hook 1 isoform b |
| 97 | chr6 | 41148102 | 41148203 | 0,000156673 | 14,29026186 | C6orf130/NFYA/C6orf130 | hypothetical protein LOC221443/nuclear transcription factor Y, alpha isoform 1/hypothetical protein LOC221443 |
| 98 | chr6 | 1,29E+08 | 128882815 | 0,0004801 | 12,19141439 | PTPRK | protein tyrosine phosphatase, receptor type, K |
| 99 | chr6 | 1,39E+08 | 138525192 | 0,000472925 | 12,21950603 | | |
| 100 | chr6 | 1,46E+08 | 146177821 | 0,000230614 | 13,56360562 | FBXO30 | F-box only protein 30 |

(continued)

| SEQ ID NO: | Chromosome | Start | End | P-value | Chi-square value | Gene | Gene Name |
|---|---|---|---|---|---|---|---|
| 101 | chr6 | 1,61E+08 | 161383312 | 0,000572481 | 11,86339358 | AK094629/MAP3K4/MAP3K4 | Hypothetical protein FLJ37310./mitogen-activated protein kinase kinase kinase 4/m itogen-activated protein kinase kinase kinase 4 |
| 102 | chr7 | 1479500 | 1479665 | 0,0001496 | 14,37724038 | | |
| 103 | chr7 | 4455011 | 4455116 | 2,27E-05 | 17,94884655 | | |
| 104 | chr7 | 5983224 | 5983379 | 0,000814954 | 11,2068621 | | |
| 105 | chr7 | 6466188 | 6466295 | 0,000609951 | 11,74536137 | | |
| 106 | chr7 | 55291210 | 55291340 | 0,001053899 | 10,73039629 | | |
| 107 | chr7 | 72093180 | 72093418 | 0,00088048 | 14,07008724 | | |
| 108 | chr7 | 72171559 | 72171806 | 5,69E-05 | 16,20284108 | NSUN5 | NOL1/NOP2/Sun domain family, member 5 isoform 1 |
| 109 | chr7 | 1,02E+08 | 101692023 | 0,000401812 | 12,52374784 | | |
| 110 | chr7 | 1,02E+08 | 101751738 | 0,000611902 | 11,73941723 | RASA4 | RAS p21 protein activator 4 |
| 111 | chr7 | 1,23E+08 | 122983379 | 0,000325854 | 12,91560316 | WASL | Wiskott-Aldrich syndrome-like |
| 112 | chr7 | 1,23E+08 | 122983556 | 0,000209449 | 13,74436089 | WASL | Wiskott-Aldrich syndrome-like |
| 113 | chr7 | 1,29E+08 | 128845655 | 0,000167042 | 14,1696641 | NRF1/NRF1 | nuclear respiratory factor 1/nuclear respiratory factor 1 |
| 114 | chr8 | 25958801 | 25958910 | 0,000118967 | 14,80899151 | | |
| 115 | chr8 | 43115669 | 43115790 | 0,000182403 | 14,0041922 | | |
| 116 | chr8 | 86206522 | 86206644 | 0,000957259 | 10,90846366 | BC070092 | Hypothetical protein FU25261. |
| 117 | chr8 | 1,42E+08 | 142388242 | 0,000225669 | 13,60428885 | | |
| 118 | chr8 | 1,45E+08 | 144750913 | 0,000648976 | 11,62997072 | TIGD5/EEF1D | tigger transposable element derived 5/eukaryotic translation elongation factor 1 delta |
| 119 | chr8 | 1,46E+08 | 145588117 | 0,000813467 | 11,21025169 | BC031570 | ADCK5 protein. |
| 120 | chr9 | 6748739 | 6748894 | 2,40E-05 | 17,84501582 | JMJD2C | jumonji domain containing 2C |
| 121 | chr9 | 36181413 | 36181517 | 0,000173112 | 14,1025146 | CLTA | clathrin, light polypeptide A isoform b |
| 122 | chr9 | 42334145 | 42334248 | 0,0001454 | 14,43086267 | | |

(continued)

| SEQ ID NO: | Chromosome | Start | End | P-value | Chi-square value | Gene | Gene Name |
|---|---|---|---|---|---|---|---|
| 123 | chr9 | 67245311 | 67245440 | 9,51E-06 | 23,12594975 | | |
| 124 | chr9 | 86993066 | 86993159 | 0,00110196 | 10,64789881 | FLJ45537/FLJ45 537 | hypothetical protein LOC401535/hypothetic al protein LOC401535 |
| 125 | chr9 | 93796287 | 93796379 | 0,000583418 | 11,82815412 | BC064363 | BarH-like homeobox 1. |
| 126 | chr9 | 95348626 | 95348979 | 0,000691745 | 11,51129603 | U43148 | Tumor suppressor patched short isoform (Fragment). |
| 127 | chr9 | 1,05E+08 | 104606311 | 0,000599408 | 11,77781574 | NIPSNAP3B | nipsnap homolog 3B |
| 128 | chr9 | 1,07E+08 | 107125545 | 0,000920123 | 10,98177362 | BC020973/BC0 20973 | RAD23-like protein B./RAD23-like protein B. |
| 129 | chr9 | 1,07E+08 | 107329991 | 0,001101579 | 10,64853945 | KLF4 | Kruppel-like factor 4 (gut) |
| 130 | chr9 | 1,11E+08 | 111473181 | 0,000569006 | 11,8747353 | BC040897/BC0 48318/BC0408 97 | Chromosome 9 open reading frame 29./BA16L21.2.1./Chro mosome 9 open reading frame 29. |
| 131 | chr9 | 1,12E+08 | 112329042 | 1,51E-05 | 18,7205768 | KIAA1958 | hypothetical protein LOC158405 |
| 132 | chr9 | 1,29E+08 | 129018769 | 0,000114139 | 14,88712944 | IER5L/AK12379 7 | immediate early response 5-like/Hypothetical protein FLJ41803. |
| 133 | chr9 | 1,3E+08 | 129895443 | 0,001090482 | 10,6672655 | BC039728 | Lung seven transmembrane receptor 1 (G protein-coupled receptor 107). |
| 134 | chr9 | 1,36E+08 | 136322760 | 0,000411339 | 12,47996971 | LHX3/LHX3 | LIM homeobox protein 3 isoform a/LIM homeobox protein 3 isoform b |
| 135 | chr9 | 1,37E+08 | 137412116 | 5,34E-07 | 28,88601644 | TUBB2 | tubulin, beta, 2 |
| 136 | chr10 | 1769223 | 1769407 | 0,000323229 | 16,07430111 | AF034837 | Adenosine deaminase, RNA-specific, B2 (RED2 homolog rat). |
| 137 | chr10 | 12277961 | 12278087 | 0,000115355 | 14,86713766 | NUDT5/C10orf 7/C10orf7 | nudix-type motif 5/D123 gene product/D123 gene product |
| 138 | chr10 | 12431830 | 12431927 | 0,000105684 | 15,03234547 | CAMK1D | calcium/calmodulin-dependent protein kinase ID |
| 139 | chr10 | 39063751 | 39063883 | 0,001081456 | 10,68264099 | | |
| 140 | chr10 | 47793890 | 47794031 | 0,000223518 | 13,62227584 | | |
| 141 | chr10 | 72647384 | 72647661 | 0,000900361 | 11,02201506 | | |

(continued)

| SEQ ID NO: | Chromosome | Start | End | P-value | Chi-square value | Gene | Gene Name |
|---|---|---|---|---|---|---|---|
| 142 | chr10 | 76835693 | 76835926 | 9,85E-05 | 15,1649962 | BC007494 | ZNF503 protein. |
| 143 | chr10 | 81731874 | 81732028 | 0,000215209 | 13,69340113 | | |
| 144 | chr10 | 89612230 | 89612351 | 0,000538766 | 11,9764678 | PTEN | phosphatase and tensin homolog |
| 145 | chr10 | 1,25E+08 | 124703983 | 0,000789103 | 11,26669002 | C10orf88 | hypothetical protein LOC80007 |
| 146 | chr10 | 1,3E+08 | 129814818 | 0,000202783 | 13,80512059 | MKI67 | antigen identified by monoclonal antibody Ki-67 |
| 147 | chr11 | 524896 | 524996 | 0,000450179 | 12,31148697 | HRAS/AK02449 5 | v-Ha-ras Harvey rat sarcoma viral oncogene/Hypothetical protein DKFZp761L1518 (Fragment). |
| 148 | chr11 | 47556581 | 47556766 | 8,41E-06 | 19,84187439 | KBTBD4/NDUFS 3 | kelch repeat and BTB (POZ) domain containing 4/NADH dehydrogenase (ubiquinone) Fe-S protein 3, |
| 149 | chr11 | 61104518 | 61104615 | 0,000939106 | 10,94393371 | SYT7 | synaptotagmin VII |
| 150 | chr11 | 65063180 | 65063335 | 0,000118217 | 14,8209104 | | |
| 151 | chr11 | 65443997 | 65444104 | 0,000932125 | 10,9577585 | Bles03/DRAP1 | basophilic leukemia expressed protein BLES03/DR1-associated protein 1 |
| 152 | chr11 | 72745477 | 72745741 | 8,93E-05 | 15,35039228 | | |
| 153 | chr11 | 74914419 | 74914510 | 0,000614221 | 11,73237768 | GDPD5 | glycerophosphodiester phosphodiesterase domain |
| 154 | chr11 | 1,18E+08 | 118395164 | 0,000238539 | 13,50019151 | RPS25/TRAPPC 4 | ribosomal protein S25/trafficking protein particle complex 4 |
| 155 | chr12 | 6449822 | 6449993 | 0,000606268 | 11,75663324 | VAMP1 | vesicle-associated membrane protein 1 isoform 1 |
| 156 | chr12 | 48248023 | 48248161 | 0,000627616 | 11,6922319 | BC011794/MC RS1 | Hypothetical protein DKFZp686N07218./m ic rospherule protein 1 isoform 2 |
| 157 | chr12 | 93045312 | 93045455 | 0,000614585 | 11,73127392 | PLXNC1 | plexin C1 |
| 158 | chr12 | 1,09 E+08 | 109182166 | 0,000840629 | 11,14931181 | ATP2A2/ATP2A 2 | ATPase, Ca++ transporting, cardiac muscle, slow/ATPase, Ca++ transporting, cardiac muscle, slow |
| 159 | chr12 | 1,1E+08 | 109935041 | 0,000588157 | 11,81308979 | CUTL2 | cut-like 2 |
| 160 | chr12 | 1,19E+08 | 119017702 | 0,000682187 | 11,53716216 | RAB35 | RAB35, member RAS oncogene family |
| 161 | chr12 | 1,19E+08 | 119396577 | 0,000286209 | 13,15855584 | DNCL1 | cytoplasmic dynein light polypeptide |

(continued)

| SEQ ID NO: | Chromosome | Start | End | P-value | Chi-square value | Gene | Gene Name |
|---|---|---|---|---|---|---|---|
| 162 | chr12 | 1,32E+08 | 131949269 | 0,000696339 | 11,49899183 | | |
| 163 | chr13 | 18081618 | 18081741 | 0,000624287 | 11,70212617 | | |
| 164 | chr13 | 79813402 | 79813505 | 0,000929702 | 10,96258238 | SPRY2 | sprouty 2 |
| 165 | chr14 | 18960232 | 18960350 | 0,000554978 | 11,9212304 | | |
| 166 | chr14 | 23771465 | 23771584 | 0,000724239 | 11,4259881 | GMPR2/NEDD8 /GMPR2 | guanosine monophosphate reductase 2 isoform 2/neural precursor cell expressed, developmentally/guan osine monophosphate reductase 2 isoform 2 |
| 167 | chr14 | 36201449 | 36201600 | 0,000430049 | 12,39689029 | PAX9 | paired box gene 9 |
| 168 | chr14 | 36736922 | 36737036 | 0,000292995 | 13,11465582 | MIPOL1 | mirror-image polydactyly 1 |
| 169 | chr14 | 50480941 | 50481049 | 0,000827923 | 11,17756613 | PYGL/PYGL | glycogen phosphorylase, liver/glycogen phosphorylase, liver |
| 170 | chr14 | 64077190 | 64077336 | 0,000353102 | 12,76533476 | | |
| 171 | chr14 | 64639425 | 64639524 | 0,000222298 | 13,63254434 | MAX | MAX protein isoform e |
| 172 | chr14 | 94305550 | 94305691 | 0,000176277 | 14,06843945 | GSC | goosecoid |
| 173 | chr15 | 21006802 | 21006907 | 0,000336817 | 12,85367485 | | |
| 174 | chr15 | 38550795 | 38550931 | 0,000414841 | 12,46413445 | D4ST1 | dermatan 4 sulfotransferase 1 |
| 175 | chr15 | 65904935 | 65905087 | 0,000478806 | 12,19644629 | | |
| 176 | chr15 | 73535116 | 73535243 | 0,001016458 | 10,79734447 | BC066364 | Hypothetical protein. |
| 177 | chr15 | 80122439 | 80122600 | 0,000465946 | 12,24724475 | | |
| 178 | chr15 | 89338732 | 89338920 | 0,000743772 | 11,37654749 | PRC1/PRC1 | protein regulator of cytokinesis 1 isoform 2/protein regulator of cytokinesis 1 isoform 2 |
| 179 | chr15 | 94701203 | 94701299 | 0,001052049 | 10,73364738 | | |
| 180 | chr15 | 99147210 | 99147469 | 0,000402469 | 12,52069456 | LOC440313 | hypothetical protein LOC440313 |
| 181 | chr16 | 43459 | 43567 | 0,000789488 | 11,26578584 | POLR3K/AF289 572/C16orf33 | DNA directed RNA polymerase III polypeptide K/Hypothetical protein./U11/U12 snRNP 25K protein |
| 182 | chr16 | 52214 | 52493 | 0,000185368 | 13,9738827 | | |

(continued)

| SEQ ID NO: | Chromosome | Start | End | P-value | Chi-square value | Gene | Gene Name |
|---|---|---|---|---|---|---|---|
| 183 | chr16 | 363973 | 364064 | 0,000349228 | 12,78597469 | MRPL28 | mitochondrial ribosomal protein L28 |
| 184 | chr16 | 674025 | 674140 | 0,000529605 | 12,00842892 | AF370420/AK1 24887 | PP14397./Hypothetical protein FLJ42897. |
| 185 | chr16 | 3244236 | 3244405 | 0,000753382 | 11,35270486 | | |
| 186 | chr16 | 19442583 | 19442684 | 0,000713539 | 11,45364455 | CP110/MIR16 | CP110 protein/membrane interacting protein of RGS16 |
| 187 | chr16 | 29372997 | 29373131 | 0,000128311 | 14,66642755 | BC062756/GIY D2 | Splice isoform 2 of Q9H3K6/GIY-YIG domain containing 2 isoform 1 |
| 188 | chr16 | 31099416 | 31099544 | 0,000264906 | 13,30353853 | FUS | fusion (involved in t(12;16) in malignant liposarcoma) |
| 189 | chr16 | 54783597 | 54783802 | 0,000411643 | 12,47858849 | BC030027/BC0 30027 | GNAO1 protein./GNAO1 protein. |
| 190 | chr16 | 72959992 | 72960138 | 0,000596519 | 11,7868082 | AK124154 | Hypothetical protein FU42160. |
| 191 | chr16 | 78192209 | 78192354 | 0,000914831 | 10,99246447 | MAF | v-maf musculoaponeurotic fibrosarcoma oncogene |
| 192 | chr16 | 86721939 | 86722067 | 0,000787949 | 11,26940745 | | |
| 193 | chr17 | 655150 | 655270 | 6,50E-05 | 15,95085222 | | |
| 194 | chr17 | 24431919 | 24432040 | 6,16E-05 | 16,05151947 | AK124161 | Hypothetical protein FU42167. |
| 195 | chr17 | 38719661 | 38719776 | 0,000375239 | 12,65162484 | MGC20235 | hypothetical protein LOC113277 |
| 196 | chr17 | 45829715 | 45829856 | 0,000929038 | 10,9639059 | PRO1855/PRO1 855 | hypothetical protein LOC55379/hypothetical protein LOC55379 |
| 197 | chr17 | 55325368 | 55325476 | 0,000151179 | 14,35746578 | TUBD1/BC0533 65 | delta-tubulin/RPS6KB1 protein. |
| 198 | chr17 | 55458830 | 55458961 | 0,000192051 | 13,90730157 | | |
| 199 | chr17 | 56841553 | 56841710 | 0,000433357 | 12,3825826 | LOC388407 | hypothetical protein LOC388407 |
| 200 | chr17 | 58134948 | 58135077 | 0,000693525 | 11,5065186 | | |
| 201 | chr17 | 63798271 | 63798372 | 0,000426527 | 12,41224765 | SLC16A6 | solute carrier family 16, member 6 |
| 202 | chr17 | 70520458 | 70520560 | 0,000263538 | 13,31324642 | ICT1 | immature colon carcinoma transcript 1 |
| 203 | chr17 | 70712975 | 70713102 | 0,001054702 | 10,72898622 | PCNT1 | pericentrin 1 |

(continued)

| SEQ ID NO: | Chromosome | Start | End | P-value | Chi-square value | Gene | Gene Name |
|---|---|---|---|---|---|---|---|
| 204 | chr17 | 71893558 | 71893671 | 0,000843093 | 11,14388038 | SPHK1 | sphingosine kinase 1 |
| 205 | chr17 | 75689829 | 75689939 | 4,60E-05 | 16,60634652 | GAA/GAA | acid alpha-glucosidase preproprotein/acid alpha-glucosidase preproprotein |
| 206 | chr17 | 76988422 | 76988525 | 1,29E-06 | 23,43828162 | | |
| 207 | chr17 | 78001554 | 78001664 | 0,000266529 | 13,29208193 | BC003595 | FLJ00406 protein (Fragment). |
| 208 | chr17 | 78248629 | 78248871 | 0,000399573 | 12,53418719 | RAB40B | RAB40B, member RAS oncogene family |
| 209 | chr18 | 31964095 | 31964188 | 2,50E-05 | 17,76720291 | BC039498/STATIP1 | SLC39A6 protein./elongator protein 2 |
| 210 | chr18 | 75371945 | 75372141 | 0,000239191 | 13,49507221 | | |
| 211 | chr19 | 876428 | 876538 | 0,000609455 | 11,74687509 | ARID3A | AT rich interactive domain 3A (BRIGHT-like) |
| 212 | chr19 | 2177443 | 2177554 | 0,000941284 | 10,93964155 | | |
| 213 | chr19 | 2242109 | 2242204 | 0,000471819 | 12,22387338 | AY358234 | EPWW6493. |
| 214 | chr19 | 4149224 | 4149351 | 0,000290339 | 13,13171343 | | |
| 215 | chr19 | 5958898 | 5959039 | 0,000101606 | 15,10662833 | | |
| 216 | chr19 | 6162973 | 6163221 | 7,08E-05 | 15,79054424 | | |
| 217 | chr19 | 13074482 | 13074572 | 0,000820608 | 11,19403341 | LYL1 | lymphoblastic leukemia derived sequence 1 |
| 218 | chr19 | 19175104 | 19175214 | 0,000608579 | 11,74955156 | TRA16 | TR4 orphan receptor associated protein TRA16 |
| 219 | chr19 | 37859582 | 37859892 | 0,000773148 | 11,30461291 | BC045605/AK127646 | Hypothetical protein DKFZp434L0718./Hypo thetical protein FU45744. |
| 220 | chr19 | 43557208 | 43557314 | 0,000235626 | 13,52324944 | PSMD8 | proteasome 26S non-ATPase subunit 8 |
| 221 | chr19 | 51951696 | 51951852 | 0,000899906 | 11,02295246 | | |
| 222 | chr19 | 53814488 | 53814583 | 0,000697731 | 11,49527994 | AB100373/RPL18/SPHK2 | Sphingosine kinase 2./ribosomal protein L18/sphingosine kinase type 2 isoform |
| 223 | chr19 | 57464757 | 57464869 | 2,28E-05 | 17,9437302 | LOC90321 | hypothetical protein LOC90321 |
| 224 | chr19 | 59396620 | 59396876 | 0,000198838 | 13,84202995 | RPS9/RPS9 | ribosomal protein S9/ribosomal protein S9 |

(continued)

| SEQ ID NO: | Chromosome | Start | End | P-value | Chi-square value | Gene | Gene Name |
|---|---|---|---|---|---|---|---|
| 225 | chr19 | 59666829 | 59666948 | 0,00066746 | 11,57774161 | LENG9 | leukocyte receptor cluster (LRC) member 9 |
| 226 | chr19 | 60542523 | 60542731 | 0,000540702 | 11,96978379 | BC044889 | SUV420H2 protein. |
| 227 | chr20 | 2801205 | 2801366 | 5,60E-05 | 16,23309088 | PTPRA | protein tyrosine phosphatase, receptor type, A |
| 228 | chr20 | 21442251 | 21442378 | 0,000258032 | 13,35283597 | NKX2-2 | NK2 transcription factor related, locus 2 |
| 229 | chr20 | 33793182 | 33793282 | 0,00048638 | 12,16717135 | RNPC2 | RNA-binding region containing protein 2 isoform |
| 230 | chr20 | 39091433 | 39091534 | 1,72E-05 | 18,47745076 | TOP1 | DNA topoisomerase I |
| 231 | chr20 | 43874589 | 43874738 | 5,68E-05 | 16,20747328 | UBE2C/UBE2C | ubiquitin-conjugating enzyme E2C isoform 4/ubiquitin-conjugating enzyme E2C isoform 4 |
| 232 | chr20 | 55757454 | 55757557 | 8,52E-05 | 15,43886697 | | |
| 233 | chr20 | 57948390 | 57948510 | 0,000788526 | 11,26804908 | PPP1R3D/C20orf177 | protein phosphatase 1, regulatory subunit 3D/hypothetical protein LOC63939 |
| 234 | chr20 | 60246637 | 60246847 | 0,000702659 | 11,48220046 | OSBPL2 | oxysterol-binding protein-like protein 2 isoform |
| 235 | chr21 | 32906806 | 32907051 | 0,000275888 | 13,22739119 | C21orf59 | hypothetical protein LOC56683 |
| 236 | chr21 | 46911964 | 46912056 | 0,000586578 | 11,81809635 | | |
| 237 | chr22 | 15863530 | 15863720 | 0,000979733 | 10,86548193 | | |
| 238 | chr22 | 19103828 | 19104117 | 0,001091193 | 10,66606081 | | |
| 239 | chr22 | 19263715 | 19263960 | 0,000260841 | 13,33253083 | | |
| 240 | chr22 | 19661225 | 19661412 | 0,000912645 | 10,99689882 | LZTR1 | leucine-zipper-like transcription regulator 1 |
| 241 | chr22 | 42155460 | 42155650 | 0,000200341 | 13,82788225 | | |
| 242 | chr22 | 42676139 | 42676235 | 0,000804941 | 11,22980531 | CGI-51/CGI-51 | CGI-51 protein/CGI-51 protein |
| 243 | chr22 | 45249954 | 45250077 | 0,000385757 | 12,59994853 | | |
| 244 | chr22 | 48920032 | 48920227 | 0,000359446 | 12,73203053 | | |
| 245 | chr22 | 49002030 | 49002159 | 0,000541863 | 11,96578914 | CR456515 | MAPK12 protein. |
| 246 | chrX | 106516 | 106713 | 1,21E-07 | 31,85069213 | | |

(continued)

| SEQ ID NO: | Chromosome | Start | End | P-value | Chi-square value | Gene | Gene Name |
|---|---|---|---|---|---|---|---|
| 247 | chrX | 1514977 | 1515236 | 0,000216997 | 13,67786623 | | |
| 248 | chrX | 21434980 | 21435328 | 0,000241991 | 13,47323255 | RP11-450P7.3 | hypothetical protein LOC257240 |
| 249 | chrX | 23560930 | 23561041 | 0,000635499 | 11,66900762 | SAT/SAT | spermidine/spermine N1-acetyltransferase/spermidine/spermine N1-acetyltransferase |
| 250 | chrX | 39721718 | 39721822 | 0,000513949 | 12,06436286 | | |
| 251 | chrX | 1,03E+08 | 103074078 | 0,000803032 | 11,23421147 | H2BFWT | H2B histone family, member W, testis-specific |
| 252 | chrX | 1,53E+08 | 152741253 | 0,000292553 | 13,11747938 | | |

The inventors have found that SEQ ID NO's: 135, 78, 230, 82, 120, 60, 75, 63 and 173 are advantageous. The loci of said sequences are surprisingly good biomarkers for stratification of patients into good or poor prognosis groups.

*Local Methylation Classification*

From the methylation classification list (12), a local methylation classification list (25) may be obtained according to the following. The methylation status is determined according to any method known in the art. Extraction of DNA is performed according to methods well known to a person skilled in the art, such as ethanol precipitation or by using a DNeasy Blood & Tissue Kit from Qiagen. From the extracted DNA, the methylation status of each sequence of classification DNA, each locus, is decided using a method well known to the skilled artisan, such as differential methylation hybridization, methylation specific sequencing, HELP assay, bisulphite sequencing, The results from these will be the methylation status of each of the assayed loci given in the form of a binary variable - 0 or 1.

In an example, Markers 1, 2, 5, 10 are selected from the methylation classification list. Then, DNA from the patient sample is evaluated and the methylation status for each of these loci corresponding to markers 1, 2, 5 and 10 is decided. The results are shown in table 2.

*Table 2*. Methylation status for each of these loci corresponding to markers 1, 2, 5 and 10.

| MARKER | METHYLATION VALUE |
|---|---|
| Marker 1 | 0 |
| Marker 2 | 1 |
| Marker 5 | 1 |
| Marker 10 | 1 |

The methylation status values are then input into the risk model, detailed in section "Diagnostic Multivariate Analysis" and finally there is an output that gives the probability of relapse risk for the patient based on the measurement of methylation at these loci.

Any kind of markers may be selected from SEQ ID NO: 1 to SEQ ID NO: 252. The methylation status at each of those markers may then be measured and input into the classification model, which will give an output similar to the list shown in table 2.

*Diagnostic Multivariate Analysis*

According to the invention, the diagnostic assay includes SEQ ID NO: 135 from the list of loci submitted, thus making it a univariate diagnostic assay. In this embodiment, upon diagnosis with breast cancer, a given patient will immediately undergo the diagnostic test as described above and the methylation level of the specific locus will be estimated. Depending on whether the methylation level is unmethylated, partially methylated or methylated, the patient would be placed in the appropriate grouping, thus suggesting that the patient's relapse-free survival function is similar to the one derived for that particular grouping and that specific locus in the list above.

For example, the survival function for locus i in the methylated state is $S_{i=Methylated}(t)$. The risk of relapse for the patient with this methylation status may be estimated from the above survival function as:

$$R(t) = S_{i=Methylated}(t)$$

Therefore, if one wishes to give the patient a risk of relapse in 5 years, the above risk function is evaluated at $t$ = 5 years.

Further, the diagnostic assay could include several loci from the list as independent risk factors. These independent risk factors would be measured as described above and their individual methylation levels ascertained. The risk functions for each of the factors is then be extracted similar to the example described in the previous embodiment. These independent risks can then be combined using any number of approaches, one of which could be as follows.

Let $R_i$ be the probability of relapse in 5 years for a given patient based on the methylation level $m_j$ of locus $i$, in a diagnostic test containing $K$ loci. The total risk of relapse for the given patient may be calculated as:

$$R(m_1, m_2, \ldots m_K) = \frac{R_1 R_2 \ldots R_K}{R_1 R_2 \ldots R_K + (1-R_1)(1-R_2)\ldots(1-R_K)}$$

In another embodiment, the risk assessment from individual loci in the diagnostic assay can be further combined with other risk factors such as age, tumor size, hormone status, etc. The risks from these individual factors can be combined just as above, assuming independence, or depending on further analysis, the factors can be combined in other ways to identify synergies amongst different risk factors, thus including that in the multivariate diagnosis.

In Fig. 3, an apparatus (30) for predicting probability of relapse free survival of a subject, who has been diagnosed with cancer, is disclosed.

The apparatus comprises a first unit (330), creating a marker panel (23) comprising at least one post from the methylation classification list according to any of claims 1 to 3. The apparatus further comprises a second unit (340), providing DNA (24) from the subject and a third unit (350), analyzing the methylation status of the parts of the DNA (24) from the subject, corresponding to the marker panel (23) resulting in a local methylation classification list (25) comprising statistically processed methylation data. The apparatus also comprises a fourth unit (360), statistically analyzing the local methylation classification list (25), thus obtaining a predicted probability (26) of relapse free survival for the subject. The units are operatively connected to each other.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A method (20) for predicting probability of relapse free survival of a subject diagnosed with breast cancer, said method comprising:

    creating (230) a marker panel (23) comprising the methylation classification list comprising the sequence SEQ ID NO: 135;
    providing (240) DNA (24) from the subject;
    analysing (250) the methylation status of the parts of the DNA (24) from the subject, corresponding to the marker panel (23) resulting in a local methylation classification list (25) comprising statistically processed methylation data;
    statistically analysing (260) the local methylation classification list (25), whereby at least the methylation of SEQ ID NO:135 is used as marker of the prognosis, thus obtaining a predicted probability (26) of relapse free survival for the subject.

2. An apparatus (30) for predicting probability of relapse free survival of a subject, who has been diagnosed with breast cancer, said apparatus being configured to perform the method according to claim 1, said apparatus comprising
    a first unit (330), creating a marker panel (23) comprising at least one post from the methylation classification list according to any of claim 1;
    a second unit (340), providing DNA (24) from the subject;
    a third unit (350), analysing the methylation status of the parts of the DNA (24) from the subject, corresponding to the marker panel (23) resulting in a local methylation classification list (25) comprising statistically processed methylation data;
    a fourth unit (360), statistically analysing the local methylation classification list (25), whereby at least the methylation of SEQ ID NO:135 is used as marker of the prognosis, thus obtaining a predicted probability (26) of relapse free survival for the subject.

## Patentansprüche

1. Verfahren (20) zum Vorhersagen der Wahrscheinlichkeit des rezidiv-freien Überlebens eines Patienten mit diagnostiziertem Brustkrebs, wobei das Verfahren Folgendes umfasst:

    Schaffen (230) eines Marker-Panels (23) mit der Methylierungsklassifikationsliste mit der Sequenz SEQ ID NO: 135;
    Bereitstellen (240) von DNA (24) des Patienten;
    Analysieren (250) des Methylierungsstatus von den Teilen der DNA (24) des Patienten, die dem Marker-Panel (23) entsprechen, so dass sich eine lokale Methylierungsklassifikationsliste (25) ergibt, die statistisch verarbeitete Methylierungsdaten umfasst;
    statistisches Analysieren (260) der lokalen Methylierungsklassifikationsliste (25), wobei mindestens die Methylierung von SEQ ID NO: 135 als Marker für die Prognose verwendet wird, um so eine vorhergesagte Wahrscheinlichkeit (26) des rezidiv-freien Überlebens des Patienten zu erlangen.

2. Gerät (30) zum Vorhersagen der Wahrscheinlichkeit des rezidiv-freien Überlebens eines Patienten mit diagnostiziertem Brustkrebs, wobei das genannte Gerät dafür eingerichtet ist, das Verfahren nach Anspruch 1 durchzuführen, wobei das Gerät Folgendes umfasst:

    eine erste Einheit (330) zum Schaffen eines Marker-Panels (23) mit mindestens einer Position aus der Methylierungsklassifikationsliste nach Anspruch 1;
eine zweite Einheit (340) zum Bereitstellen von DNA (24) des Patienten;
eine dritte Einheit (350) zum Analysieren des Methylierungsstatus von den Teilen der DNA (24) des Patienten, die dem Marker-Panel (23) entsprechen, so dass sich eine lokale Methylierungsklassifikationsliste (25) ergibt, die statistisch verarbeitete Methylierungsdaten umfasst;
eine vierte Einheit (360) zum statistischen Analysieren der lokalen Methylierungsklassifikationsliste (25), wobei mindestens die Methylierung von SEQ ID NO: 135 als Marker für die Prognose verwendet wird, um so eine vorhergesagte Wahrscheinlichkeit (26) des rezidiv-freien Überlebens des Patienten zu erlangen.

## Revendications

**1.** Procédé (20) de prévision de la probabilité de survie sans récidive d'un sujet chez lequel un cancer du sein a été diagnostiqué, ledit procédé comprenant :

la création (230) d'un groupe de marqueurs (23) comprenant la liste de classification des méthylations comprenant la séquence SEQ ID N° 135 ;
la fourniture (240) d'ADN (24) du sujet ;
l'analyse (250) de l'état de méthylation des parties de l'ADN (24) du sujet qui correspondent au groupe de marqueurs (23) pour obtenir une liste de classification des méthylations locales (25) comprenant des données de méthylation traitées sur le plan statistique ;
l'analyse statistique (260) de la liste de classification des méthylations locales (25), au moins la méthylation de SEQ ID N° 135 étant utilisée comme marqueur du pronostic, afin d'obtenir une probabilité théorique (26) de survie sans récidive du sujet.

**2.** Appareil (30) de prévision de la probabilité de survie sans récidive d'un sujet chez lequel un cancer du sein a été diagnostiqué, ledit appareil étant conçu pour effectuer le procédé selon la revendication 1, ledit appareil comprenant une première unité (330) qui crée un groupe de marqueurs (23) comprenant au moins un poste de la liste de classification des méthylations selon la revendication 1 ;
une deuxième unité (340) qui fournit l'ADN (24) du sujet ;
une troisième unité (350) qui analyse l'état de méthylation des parties de l'ADN (24) du sujet qui correspondent au groupe de marqueurs (23) pour obtenir une liste de classification des méthylations locales (25) comprenant des données de méthylation traitées sur le plan statistique ;
une quatrième unité (360) qui analyse sur le plan statistique la liste de classification des méthylations locales (25), au moins la méthylation de SEQ ID N° 135 étant utilisée comme marqueur du pronostic, afin d'obtenir une probabilité théorique (26) de survie sans récidive du sujet.

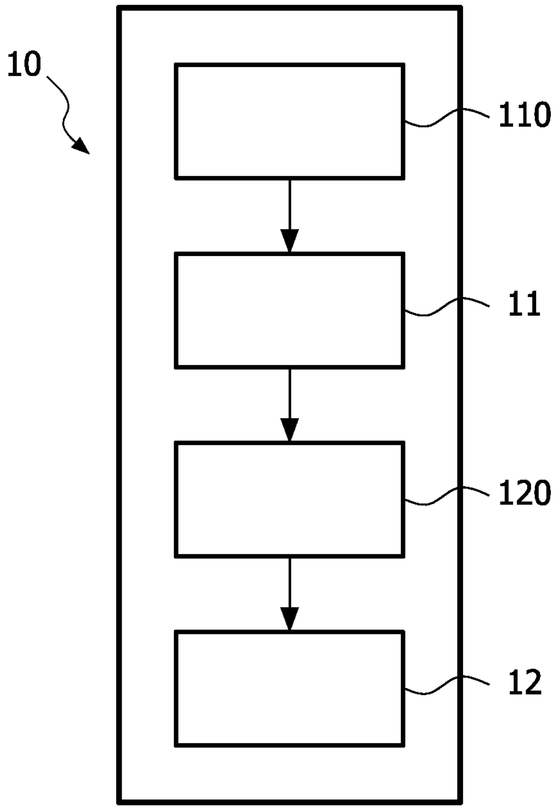


FIG. 1

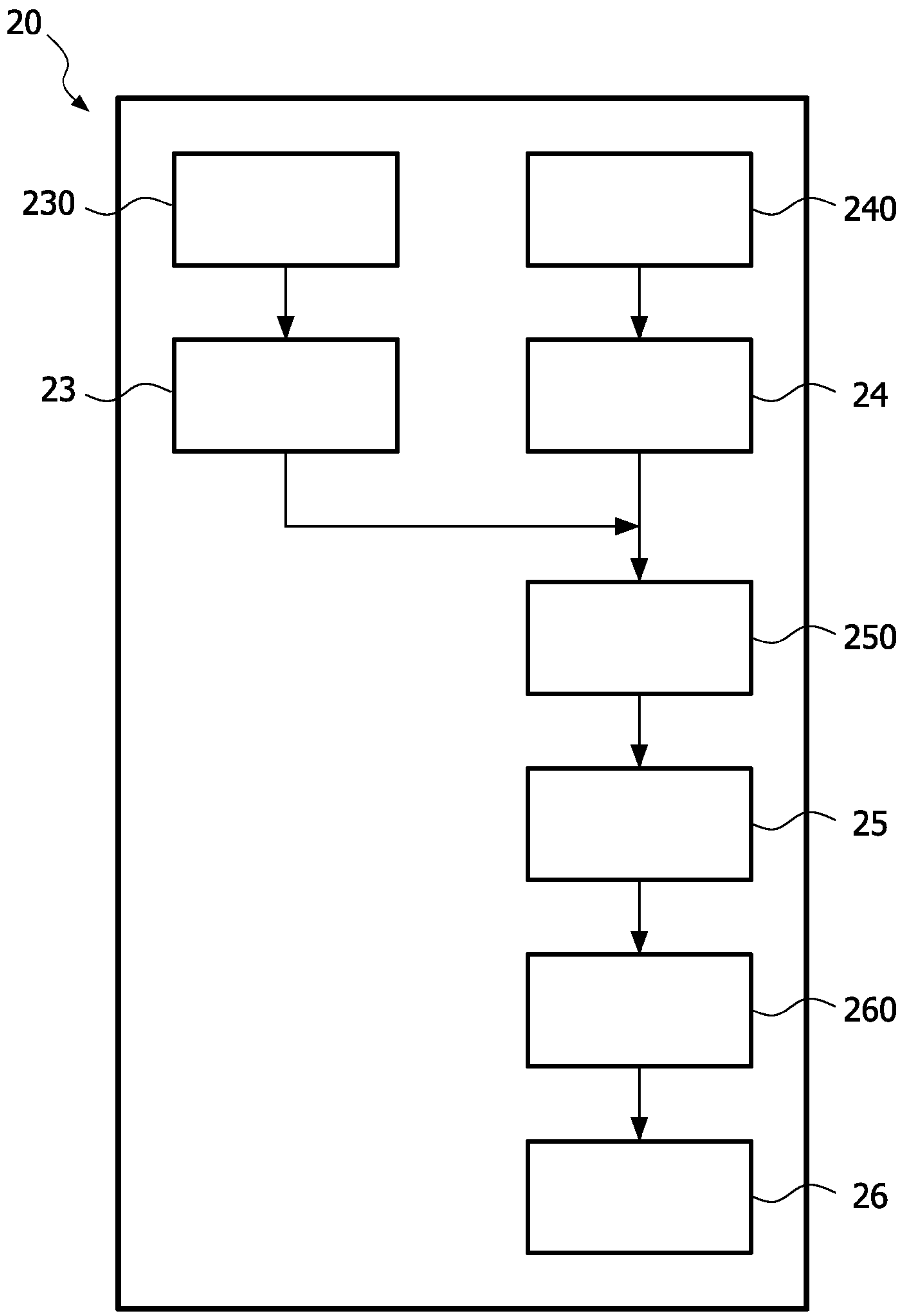


FIG. 2

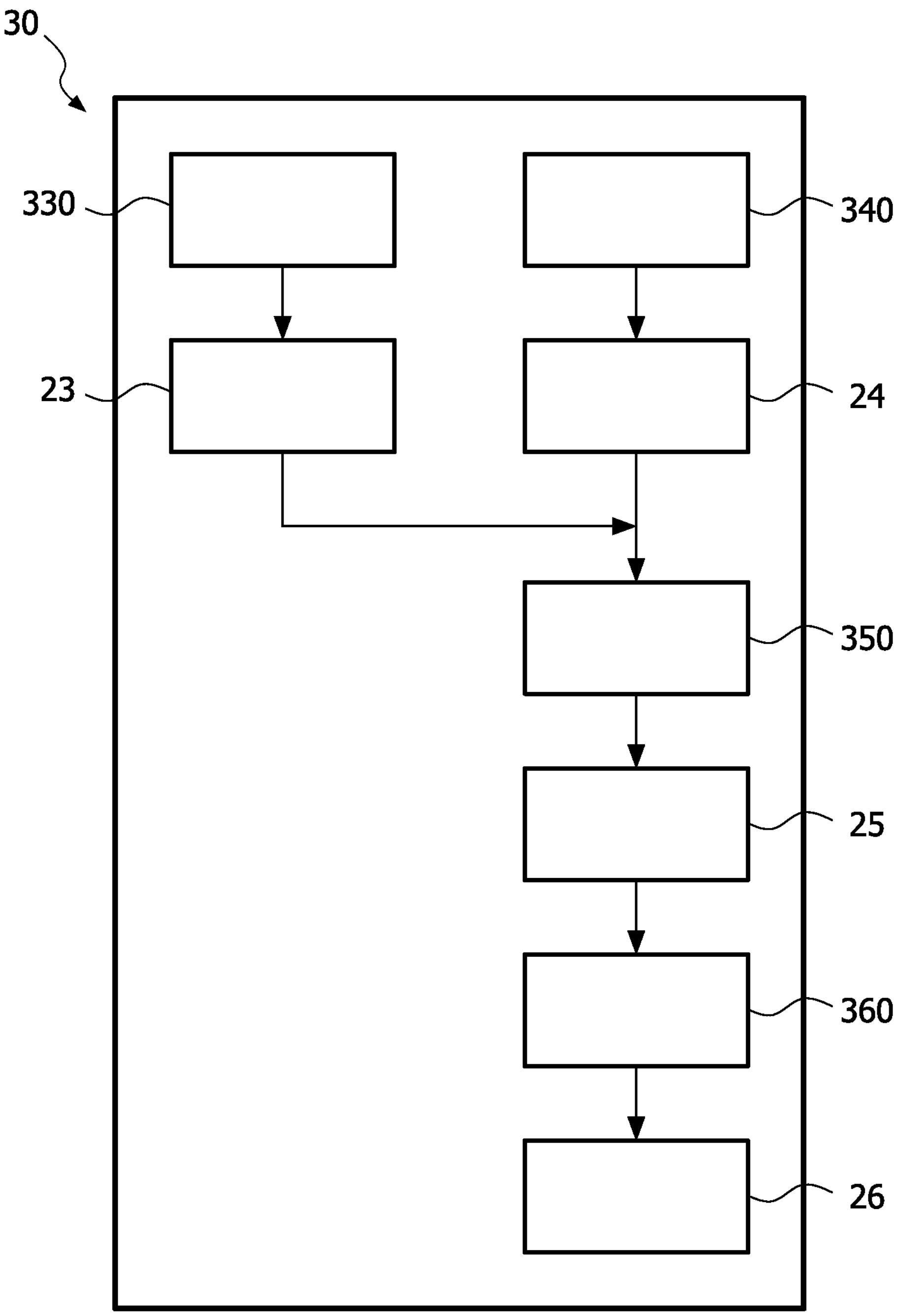


FIG. 3

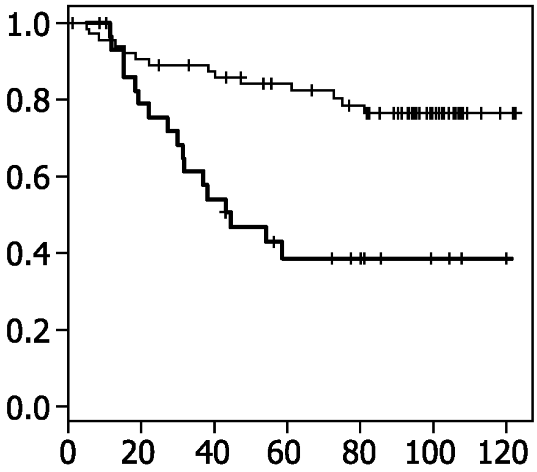


FIG. 4A

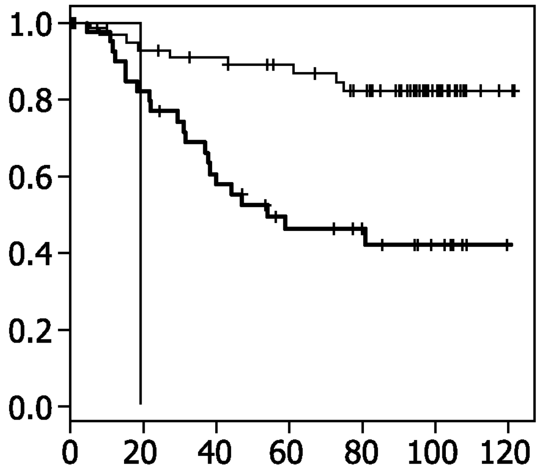


FIG. 4B

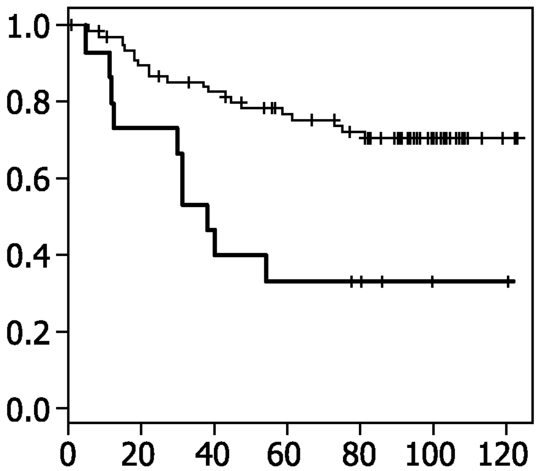


FIG. 4C

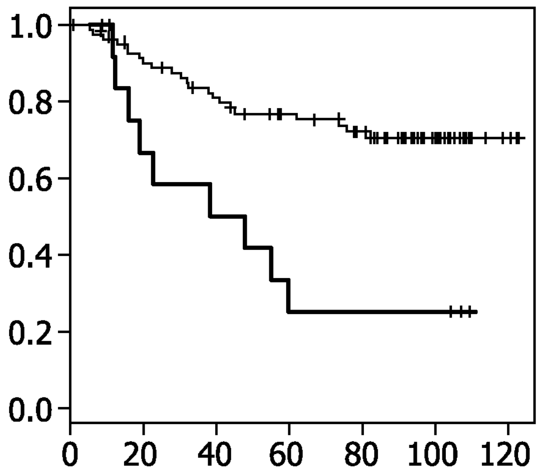


FIG. 4D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2008083251 A
- US 20050021240 A1

### Non-patent literature cited in the description

- **ARAI, T. et al.** *Breast Cancer Res Treat,* 2006, vol. 100, 169-176
- **CRISTOFANILLI, M.** *Breast Diseases: A year book quarterly,* 01 October 2005, vol. 16 (3

SEQUENCE LISTING

<110> Koninklijke Philips Electronics N.V.

<120> METHYLATION BIOMARKERS FOR PREDICTING RELAPSE FREE SURVIVAL

<130> PH011909

<160> 252

<170> PatentIn version 3.3

<210> 1
<211> 242
<212> DNA
<213> Homo sapiens

<400> 1

```
cacagggctc gggcgacttc agcgaggctc tgtctgagag tctctgcccc ggaggccgaa      60
gaacggagtc cacggcaggg agccaaacac cagcgcagca ctgtccccag aagtgcccgc     120
tgcctccccg agcggagacg tgacctggct tggggccgca cgcaggggga agcgtatcca     180
acaccgcagg cccggcccct ccccacgcgc gccagacggt ccccaccctg cgcctgtgca     240
tg                                                                    242
```

<210> 2
<211> 191
<212> DNA
<213> Homo sapiens

<400> 2

gctcactcac cgttcgctgc cgcgggcgcc tgcgggggcg tctacatgcc ggaccgagcc 60

cgcaggcccc gccgccgcgc cgccgccagc cgtcgctacc tggcccttgg cgccctggcc 120

gcctgttgcc ccatggccgc ccggaccccg gcgccggcgc cgccgagcag caatgcgccg 180

cgcccgccca c 191

<210> 3
<211> 222
<212> DNA
<213> Homo sapiens

<400> 3

ggccggtgtt ggagcatccg tctctgcgtc cccacctgat gacaagctcc ggggagcagg 60

agtgctggcc aagcccacgc acggaccaag cccacgtcag actccgccag aaaggcaaga 120

agggcctccg catggcgtga cggatgggtt tatgggggca ttccatccca cccggacgct 180

cggagggcag ggagacagga agatcaggcg accaaaagtg ca 222

<210> 4
<211> 223
<212> DNA
<213> Homo sapiens

<400> 4

cgcttgctcc ggccgatctg gcccagcttg ggcggccgct tgttctgccc ggcgccgaag 60

aagttgttgg tgtcctgcag gcggcaggag aagatctggc ccacgtcgcc gccgtcgccg 120

taggggctca gcttctcgtc gccgtagggc agcacctccg acatggtcgc gtccgggggc 180

tccgccgcgg cgcctcctcc gcccgcgtcc ccgcccgcgg cgg 223

<210> 5
<211> 259
<212> DNA
<213> Homo sapiens

<400> 5

cgtaccacta ctctaacaaa ccagtgcctc gaaagctatt tcttgcctcc ggtacttccc 60

ttttaaccat gaagttttca cgctagaggc tactcaacgc acacacaggc ccgcccagac 120

tgtggagcgg gacctgttta gagtagaaac cgagtcgcca aagaaaccag tgttggctct 180

ttagagggag atttaacttg agcctagacc ggttccgaac ccctttcccg agccctcctg 240

acgtcatggg accttccgg 259

<210> 6
<211> 198
<212> DNA
<213> Homo sapiens

<400> 6

ggtgaacccg tagcgcctct ctcggtaacg gcggctgcgg gatcgcgacc ggctccgcga 60

gtatgaccgc gagtagcgcc tgtacttccg ctggtggcgc cttcgggaac gggacctgga 120

cttgctcctc cgactcctgg acgaaaaccg gctcgagacg cgggaatggg accgagagct 180

tctggaaaaa gagcggct 198

<210> 7
<211> 352
<212> DNA
<213> Homo sapiens

<400> 7

tcagcgctga cctctcccct ccccctccgc tgtgtccatc gcagtccgcc ggccccctcc 60

cagcgtctct cgaccccttc ccccttccc tgtactccag ccgcggctga ttcctgctgt 120

ctgggtgggg tccctgtaag gctcaggcgg ctgactgggg aggcgatccc aggaggcaca 180

gtggggaggg gatagcaggg cggaaagagg ctgaatcaca aggaggcggg ggcggcggct 240

cggtcccagg gccatctcag ggaccgtgtc gggggtgct gactctgccc atcaagggga 300

gccggacatt tatccgccaa agcccgtccc tcgcggtgag ggtcgctcct gg 352

<210> 8
<211> 237
<212> DNA
<213> Homo sapiens

<400> 8

ggagcccgcc ccctgtggcc gggcctcctc caatccccac cgtttcttcc gggacggata 60

cctgcctccg ccaattgtcg cgtaccctgg ccaggtaact agggccgagt tacaaccgcg 120

cggcgttggc atctccagct ccagctctgc gcacctatac cgagacagcc acaggaattc 180

aggatcccgg gtcgcagaat cgaagcctgt gcaggaacaa atgggggcag actgagc 237

<210> 9
<211> 273
<212> DNA
<213> Homo sapiens

<400> 9

cttcccctac gctaaaagga cgaacccaca ccttccgcgg gattccgccc gggcttccaa 60

cgtggagacg acttttactg agctggttta acagttgccc acgaacgctt aagcacgccc 120

agacccactc aagtacaggc caagaagtcg gtgggatcgg ggagggcggg ggcttcgggc 180

ctgggcggcg caaaggctcc ccaggaaggc tgcgggggcc ccccggagct gacggcgcgg 240

cgcggcgcgg cgctgcgcgg ccccggactg ggc 273

<210> 10
<211> 205
<212> DNA
<213> Homo sapiens

<400> 10

ctccttcctg gggccgaaga agaaaacctt tctgcgctgc caccccggcc gggagctggc 60

ggtcaagttc gcggtacaag gagctaggga cgtcagccct ccagcccgcg cctgtatctc 120

ggggtggtat aaacaggatg tcagacagcg cgctccgggc ttgtttatga aaacttgccg 180

tgcgcgctgg gggaggggc ggcct 205

<210> 11
<211> 198
<212> DNA
<213> Homo sapiens

<400> 11

aagtttgtcc taaccaaatg aatacaagaa gagagccttc aaaataaacc ggggcggggg 60

ggggccgagc tgcagcttgc ggctcagccc gcaaggcacc ctgagggcgc ggccagcggg 120

gaacgcaaac cgcgtccgtg cacgagtccg ggagccacag cctgggccac ggcaattgac 180

tgcgaaccca caacaaac 198

<210> 12
<211> 217
<212> DNA
<213> Homo sapiens

<400> 12

cacccccctag cgccgctggg cctgcaggtc tctgtcgagc agcggacgcc ggtctctgtt 60

ccgcaggatg gtgagtggat gcctcggtct cggggcttta gatgcatgga ggttcccttt 120

tcttgcccgt atgccagcct agggcccgtc tcgcgcgtcg caggggccgg atggcgttag 180

attgctagct ctgacttggt cgaggtgcag ttcccag 217

<210> 13
<211> 249
<212> DNA
<213> Homo sapiens

<400> 13

atgtgcagtg acatttctga cttgagtgga ggaatgcgcg aaggaaaccc gggcgaaatt 60

ggtattagag gatcttcttg gcgccaatgc taattgtcct gatttatgcc ccttctgact 120

aagctcaacg tcttcatctg aaataaaatg aaagcggtgc caagcggcat agaaccgcct 180

ggccgctggt tctgatcccc gggaaccaga aaatcgtcct gcggcttgaa aaaaaaaaaa 240

agaggaaaa 249

<210> 14
<211> 197
<212> DNA
<213> Homo sapiens

<400> 14

ccccgcctca gcctttcccg ccgctcgggc gctgagccca gggaccagcc ggcggcagtg 60

accccgcccg aagccccttc tggaacctcc tggggacaat tcccttttcc tttcttcagc 120

ctaacccgcg gtggccctcg cggagtccgg tcagccccgg ggagcgcagc cgggaggggc 180

gtccaagggg cgtctcg 197

<210> 15

<211> 277
<212> DNA
<213> Homo sapiens

<400> 15

gtggggtccc cactctgcag gggcgcgccc ctgccccgcg cgccgactcc gggtaccaga 60
gccgtcagcc ccgcccctcg ggcagggggc gggcgtggaa acgcctaaga ggatgctgag 120
tgacagaggc gcaggccgcg ctccgaccct gccatgtctc tccccagctg tctctcccgc 180
cagccgccgc gtcctcaacg tcctcccctt ttagcagact gggctcccgg agcagcagcg 240
gccggccgcg cagacgcaaa gcctcgggga tggagga 277

<210> 16
<211> 215
<212> DNA
<213> Homo sapiens

<400> 16

cagccccagc cgggctcgcg gcagcgaaag caaaatccga tctctctccc gggggagcaa 60
aatggacgaa aggcgacccc ccaggcaggg gcgctgggtg ccttgggaat ccgaggaatc 120
ccttctcttt gccagtcgga ggggactaga tggagccgaa ggggccggag atgggccgag 180
cgctgccccc cgggggtcct cggcgccggg cgcag 215

<210> 17
<211> 223
<212> DNA
<213> Homo sapiens

<400> 17

caccccctcc tgcgctgtcc atggataaga tctcccccag ccccgaggcc gggaacgatc 60
ctgtcctcca gtgacctcct tttccctccc cttacctcct ccttaccaga gctttaaggt 120
gaaagcacag agcaaccagc agaggtggcg gccctggggg aggccaggcc tcccggcacc 180
cgcaagcatg gagcgccaag tgtcgcacct gtcggaacag agg 223

<210> 18
<211> 209
<212> DNA
<213> Homo sapiens

<400> 18

gcgccgaatc ctaaccacta gaccaccagg gagaggcggc ttcagggccc ggcttgcgcc 60

tcctggcccc agcgcctctg gccccagcac tgggcgctac cttggcgcct gtcccctgct 120

tcgctttaaa acaaacaccg caaccagccc ccgcgacgcc ggcggcaacc ggcctgccct 180

cacgagcagc tctggccctg ttcgcagca 209

<210> 19
<211> 214
<212> DNA
<213> Homo sapiens

<400> 19

ggcgcaggcg ttcgcgccgg gtcccagcca tgccagcggc gacgcgcccc ggcgcgccgt 60

caggatggcc gagcggtcta aggcgctgcg ttcaggtcgc agtctcccct ggaggcgtgg 120

gttcgaatcc cactcctgac aagccgacct tttggcccgc ccgccggagg gcaacgccca 180

tggcaaccct ggagcacctt tggccgcttc ctgc 214

<210> 20
<211> 205
<212> DNA
<213> Homo sapiens

<400> 20

ggaacgtagc gggcggaaac tctcgaaaag cgactgcgca ggcgcgcgcc gggcgcggac 60

gctcacgggt cacccagggt atccgcccca agcggagaat atcggcgcaa gcgcggagta 120

gccgtgtcac gcatgcgtgc gaacgcacca cattccggaa cgggaagaag gagctgtctc 180

tctcttctga ggggtaggca ggtgc 205

<210> 21
<211> 216
<212> DNA
<213> Homo sapiens

<400> 21

tctcatcgct ccgcgggagc ccaggaactg ccctacaaca gccgaaatcc gggtcgtcta 60

ttccaacgga ctcctcttcc gttggatacg gggccgtga gaggccaaac aacctccgcg 120

aggctacccc cgcagcgacc gctcggcggg cgctccattc ctttaccgga ctgtacacct 180

cgcctcctgg ggcctgaggc gggaacccgg gtcgcg 216

<210> 22

<211> 218
<212> DNA
<213> Homo sapiens

<400> 22

```
gcgccgggag agtcctgccc ctgtggcttg cacgctcagc agggcgcccc gggcaccctg      60
gagagaccaa ggagttcctc gctccgcttg ccaaggggtt gtacttccgc ctgcgacttc     120
tttagtggtg cggtagctac atcttcgggg tttactttaa gtaagggccg gctcatccag     180
ggatggtgac tacctacaaa ctagggcgca cgagcgcg                             218
```

<210> 23
<211> 254
<212> DNA
<213> Homo sapiens

<400> 23

```
agtgttccgc aaatccttgc cgaattccat cccggccaaa gcagggaccc gggggagcct      60
ctggtcgcac ccgagacagt tcgttaaata ctcagtgcac gtgctgggga tggaagtgct     120
gacccactgc tcaggtacgc gcgcacctga cgccgcttgg tgcgcgccgc ggggtactgg     180
gtacgtgctc cgcgcgcgcg cgcccggcaa agttccccgt acgtgcgccg tacgtgggcg     240
cggtgcccca gcgc                                                       254
```

<210> 24
<211> 202
<212> DNA
<213> Homo sapiens

<400> 24

```
agcgagccgg gaagggagag cagcggaggg ctgcagagct gcgggcgccc ggaccgtgcc      60
acacaccccc cgcggggcac ggagggcatt gcggggggag acacacaaag acatgcgaag     120
aggggctgaa cgaggctcgc gcacaaagac gccggggcgc acggcagctg gggctgcacc     180
caccgccccc gcgccgcgcc gt                                              202
```

<210> 25
<211> 221
<212> DNA
<213> Homo sapiens

<400> 25

cgcgccgctg ggccaggcgg agagaggcgg acggacttca ggggaggccc gggccacgcc 60

gcggaaacta cccgactccc tggaggcggc caggaccgac cctgtcccga caaaatggag 120

ttccccgtgt ggcttcagct cgcggcgcgt tcccagagct cctcagtgat ccggctttcg 180

gattgttcgc ctttcatctc atttgccgtt gtccaaattc t 221

<210> 26
<211> 225
<212> DNA
<213> Homo sapiens

<400> 26

tctgccttcc tggcgtcagg agagtcggac gcttggccct ttcccagccc ggcctcgtat 60

agggtccgag gagcccatag ctgcactcct agttaggtgg gaggtcggac tccgccgaca 120

aatcccaaag agaggggtgg ggaagaagga gataccagcc ctgaaagcga aaagccggag 180

aggaaagtcc tgaaaagccg ccaggaattc ccaaggagcg caggg 225

<210> 27
<211> 232
<212> DNA
<213> Homo sapiens

<400> 27

agaggccgca gtgaggggca ccgagtggcg ggctcctccg caccaccccc gggctttata 60

ataaaagtcg ccgttgttta agagccaggt cggaggcgcc gctttctggg cggtgaaagc 120

cgaagcgaga gcgggcaatt cgctgcggcg gcggccgctt cctgggcgtc ggtccagtgc 180

cccggcccgg cctgggagac ccgcaccaac cccgggaggg aggacggggc gc 232

<210> 28
<211> 214
<212> DNA
<213> Homo sapiens

<400> 28

cgccacacaa aacaagcatc cttagcctga agggccccgg ccgaagggcc ggaagtggaa 60

gtgcgcggcc ttgcccgccg cgtttcccct cctcggagtg ctttttgtgg accctgggca 120

aggggctctt gctacataga gaggagcaaa gaacaggcat cgcccggtcc ttaaagcggc 180

ggcaccctcc acgccccgca gtcgcgtctg cttg 214

<210> 29

<211> 194
<212> DNA
<213> Homo sapiens

<400> 29

cagcaccgtc atggacacac aacgcgggtg tcctgagatt tgtggacccc ggagatgcaa 60

agacgatggc agcagcgtta acaactaatt acagtagcgg cgaccgcgcc aaggctttcc 120

ttcaaccctg attccgcaga tatccggaaa actcttaccc tccctcgata tgccgccccc 180

atccgcagcc acat 194

<210> 30
<211> 346
<212> DNA
<213> Homo sapiens

<400> 30

ggcgcaggac tccacgagcg agaacagcag ctccgtgggc ggcaggtgcc ggagcctcaa 60

gaccccgaag aaacgctcca atccaggtag gcggctgggc gcagggacgc gggtgaggag 120

ggcggcaggt gggcgagctg cccaaacccc actgccaggg tgtcccgtgc cctggggagg 180

gggcgtccag gcctggcctc tccttgcagg tgggcgaact gcccaaaccc cactgccagg 240

gtgtcccgtg ccctggggag ggggcgtcca ggcctggcct ctcctttccc acggcccggc 300

accccgcggg gctccgccgg ggacatcacc gcggagggtt tctctg 346

<210> 31
<211> 211
<212> DNA
<213> Homo sapiens

<400> 31

caggcctggc ctctcctttc ccacggcccg gcaccccgcg gggctccgcc ggggacatca 60

ccgcggaggg tttctctgtg agaaactgcc ctactgcgtg ccgtcatcag gcttagggcc 120

gtgaatccct tggcgctcag caggtggaa aggcgagggc ccggatgcag gtggcagcat 180

gacccgcacc tgctcgctct gggggcagcc t 211

<210> 32
<211> 235
<212> DNA
<213> Homo sapiens

<400> 32

ggtccgcggc tgctctgcga ggcccagcgc gccccaggtc gcggctcccc ggccgccagc 60
gcacctttcc atctccaggg aaaatattta gacgggaaac tcgagtgcac cttattttct 120
ctttggttta tgagtgaccc agaaggcttc tagctcacaa tcgcacgatt tcaggacgag 180
gctgccggag aagcgctggc gaccgaggat gggggaagga caaagaggaa gagga 235

<210> 33
<211> 325
<212> DNA
<213> Homo sapiens

<400> 33

cgcatccgca tcccgagaaa actttctttg ctccgaagcc ggacgcggcc gggccaaccc 60
tgcccgagac agaagccctt tgttcccgcc taggtggctc ggaggagatg ggtgatggcc 120
accttgggcc tctttaagac ctcccctctc tcggcctcgt ggccctcgcg gggttccgcg 180
gcctggtctt ttccccgcag cgcggccaac tttgtccaga gtcggggtcc gcggcgagcg 240
ggggatcccg ctgcccgccg ccgcccgccg ccgcccgggg tctttggtac ccggtgcgga 300
ccttatcaac ttgttcttga agtgg 325

<210> 34
<211> 234
<212> DNA
<213> Homo sapiens

<400> 34

taggcggccc ctccccaggg ctgcctcccg cgcccctcgg cccatttccc ggttcgggcg 60
tgcgctcctc tgctgcgacc cacggagtgc gacgggacag ccacgttttc acatcgggcc 120
ccgtgaaatt gccgccaatg gaaaggactt ggtccagaaa aacgttagtt tcatatggtt 180
cgcccggtac ttaaatgttt tattttctcc cccaacagaa aactaaagca gctg 234

<210> 35
<211> 202
<212> DNA
<213> Homo sapiens

<400> 35

gaggaggacg cgttcggcct gggggggactg ggtggcctca cccccaaccc ggtcatcctc 60

caggactgca tgtggagcgg cttctccgcc cgcgagaagc tggagcgcgc cgtgagcgag 120

aagctgcagc acggccgcgg gccgccaacc gccggttcca ccgcccagtc cccgggagcc 180

ggcgccgcca gccctgcggg tc 202

<210> 36
<211> 222
<212> DNA
<213> Homo sapiens

<400> 36

gcctccttgg cgttcctcgg tgcgcgctct acgctgcgct cctggacgcc gggagcaggg 60

cagtgcgctg cactgggcgc ccgcaaggga aaaacgctac ctgtggaagt aatgtaggca 120

gaagaggccc agaagtaccg tcccaggtgg cccgaagagg gagaggggct gccgggcgag 180

gatccccgcg ggcaccgcga aggaaggcag ctcctgcagg aa 222

<210> 37
<211> 289
<212> DNA
<213> Homo sapiens

<400> 37

gccgtaagtg acggctgtcg cacgactgcc gcgcctgcgc agaacctccc ggcggacaag 60

aagctgggag gcgcgcagaa ccatcgtcag tccccgaggc tctctgcagt ccgcgtgagt 120

cctggccaag gtctggcctc ggaagctacg agctacaatc ttccttcatc ctacccaccc 180

acgaagagcg ccacccccagc tgcagaccaa gtgaccagcc cagttagaag gggctcggcc 240

ggatcaaacc tcggtgctgc ctcaaagcat ctgctgaagc tcttcctcg 289

<210> 38
<211> 199
<212> DNA
<213> Homo sapiens

<400> 38

gcagtcagcc tagccacctt cggaactctc cacgcctgat cccgttcccc ggagcccgcg 60

gccgttacct tggtgcctcc gagccgtaac acttcctcca gggagaaccc attctcggct 120

tcactagtat tatcctcatc ctcctcgtcc ggatcttcta ctgcctcctc ggggcgccaa 180

ggccgcttgg catggaact 199

<210> 39
<211> 244
<212> DNA
<213> Homo sapiens

<400> 39

tcagggcccg gcagtggact cgctgcgggg cctgcggcca ggacggcgcc gggttcgcag 60

tcgggatgcg ggaaacaggt agaggccaga atacgagccc caaagagaag agtgggcgct 120

tccccaaacg ggtcggcgca gcggaaaagg ctaactgaag caagcgggag gccccgcacc 180

gaggccctgc ttcccgggga gcagccaggc acagcggaca ggggggccgc gctttgttac 240

cggc 244

<210> 40
<211> 282
<212> DNA
<213> Homo sapiens

<400> 40

ccgccttggt tcggtatcag ggcctgggat cctgctggtc cagattcacc ggccccaggc 60

agactggatg aaacacactg ggccgcgatg ctcgtcagat cccgaccact cctggagcag 120

gaggcggcag acacactgac aaacagggtg acgcgcacac cgtgcgcaca acgcgcatca 180

cttgtccgca gaagtggaag acgtacctgg ctcgcagagt tgggtcccga gccggctgtc 240

gggaggtgct gcccaagggt cagaggtcca aactggactt gg 282

<210> 41
<211> 231
<212> DNA
<213> Homo sapiens

<400> 41

gcgcggggcg ctcgggacct gcgcgcacct cggaccttca ccacccgccc gggccgcggg 60

gagcggacga gggccacagc cccccacccg ccagggagcc caggtgctcg gcgtctgaac 120

gtctcaaagg gccacagcga caatgacagc tgacaaggag aagaaaaggt aagcgggcgt 180

ccgggccgat caggggggccg gtccgaggcc agggccgggc tgcgcggggc a 231

<210> 42
<211> 231
<212> DNA
<213> Homo sapiens

<400> 42

```
ctcaaacccg ctccatctat taacgttctc ttctgcccag ccacgccccc ggcgttctcc      60
gtggttgcgc acctgacgta catcgttcaa aacgtgccca gtgtttccct ccgagggcac     120
tgcgtgccgc gcaggcgcaa agggccaggt gctggaggtg ctgtcatggc ctgcctcaac     180
ccggtggtaa ggaagacctc aggctctgtg tagagggagc gccctgggac t              231
```

<210> 43
<211> 246
<212> DNA
<213> Homo sapiens

<400> 43

```
cgccgccccg ccgcgcgtcg ctcgcaggac ttcagcctga cagctgctcc ggcctcccgc      60
ccacataccc gctctcgccg cgggctggac gacgggaacg aggctcaagg ctgattggct     120
ctgacgcctg ctcatgtagc cacactttcc gcctctgcgc cttagcctac ttcctattgg     180
tcgagagaag tggagccggg ggctgattgg cttaaactct taacagctac aaccggcctt     240
cgcctc                                                                246
```

<210> 44
<211> 221
<212> DNA
<213> Homo sapiens

<400> 44

```
cgggaagccg gcgtttgcac acattttcgg acgtcatatt aaagcgcacc ggacgccgag      60
acttctggga gatgaagtcc ttgcgaaggc cccgcattga ttgggagcgc agtcgccagg     120
accctgtgcg caggcgcgtt ggtttcccga cctgaagagg cgccgtcttc ccgggtcccg     180
agcactctgt gccggaggtg agggttgtgg gtgtgtgtct t                         221
```

<210> 45
<211> 339
<212> DNA
<213> Homo sapiens

<400> 45

```
caggtccgcc tcagcaaggt gcccgcgccc accttccaac ccaaaaggcc ggcgcggaag      60
ggcagcataa aggcagcggc cacctgcgga tacccaagtg gccttacaag gtggccaagg     120
aggagaaacg ggaggcggag gaggccgaga agaggcgcca ggccaaggtg caggagaagc     180
gcccgccgcc ctggaagaag aggacgtgag agtccgcggg tgcttggcac ggggtttgag     240
ggctgggtga ggccgcgggg ctgggcacca tggcagctct cgggaccacc gggcagcgcg     300
cgtttccacg ctgtctctct aggatgctcc cagaaaggg                            339
```

<210> 46
<211> 224
<212> DNA
<213> Homo sapiens

<400> 46

```
ccgcagcagc gcgttccaag gcactggccg cgcggcgcgc acgtggcgcc ggcagaaggc      60
caggaagggg cggcagtcca gcagcagcgt gcgttccgcc tcccgcggat cccgcagcag     120
cgtgcccagc gccgcgcact ccagctcgcg cgccgcctcc agccccatgg ccaccggtgc     180
ctcttcctct tcctttcggt ctttcccgcg tcccgggctc tcgt                      224
```

<210> 47
<211> 302
<212> DNA
<213> Homo sapiens

<400> 47

```
actgaccggg cttcaaaccc cggtactgtg tgaagatgcg ccagacgccc gggttccctc      60
cttaagcaat tgatttacag tgacatctag tggagaaagg gacacacatg aactgacatt     120
ttcctatgtc cagagcaacc gagaaccacg gcccactcag gttcgcggga aggagcactg     180
cgccacggcg ttttcgggat tcgtggcctg ctcccgcagg gcacagacac tatcttctgt     240
cctttgagtg cccgggcagc attgcgcctg agtatttgta aacggagggc cggtatccca     300
tc                                                                    302
```

<210> 48
<211> 205
<212> DNA
<213> Homo sapiens

<400> 48

ccgcggatcg cagggccccg gcgcagggac gggggaggcc gcgatcggcc gggagaactt 60

aaccgtgttt attacaacta tttatgttcg cgcacaaagc tgctgtcaca ttgcataaag 120

gaggtcctcg cttcggcgaa gggagctttt gtgcccggct tcaggctctg cgccctgccc 180

cagatttaca ggactctaac agcca 205

<210> 49
<211> 221
<212> DNA
<213> Homo sapiens

<400> 49

ggtgatccag aaactacccc ggcagggagt gggggagcag cggatattcc ggggtaggaa 60

cggccatcgc aacgctgaca gaggttgttt tttaagaagc aaaactgttg gcggcgacct 120

gagcgctgga agccgaaggg gaagaggaag gagacgcgaa gccagggcgg ccggcacaaa 180

ggcggcggac tcgcggcggc agcgcctgcc cggccgggag c 221

<210> 50
<211> 266
<212> DNA
<213> Homo sapiens

<400> 50

ccggggccgc ggcagctctg ggaactccct ctcgccgcgc ggcggcggcc ggcgggggct 60

gcagctccgc gctgacttcc cctccgcctt cgacctccgc actcgcctaa aagtacggcg 120

cggcagcggg cgagactcga gcagctcggg cagccgccgc cctgcgtgcc atggacggga 180

gttcgcgcgc ggagctggcg gcgtcgccga ctctcccggc ggcagcggag gagcagcgcc 240

gccgcgtccc cacctaccgc ggcctc 266

<210> 51
<211> 251
<212> DNA
<213> Homo sapiens

<400> 51

tggcagtggg tcacgtggtg ctgcgtgcgc acggaggcgc agggcggccc ggtcactgag 60

tgcgcaggcg ccgtgggcct cgccctgccc cgcggcggga acgcggccgt gagggtgtgg 120

ggtgtaggtc cgccaggcct gcaacggctg ccgagggtcg tgggatgcgc tcgccgcgtc 180

cctgctcagg cggagggagc ccggcccgcc gccagggtct cgagtccggg tccaggcagg 240

cgtggaggtg g 251

<210> 52
<211> 243
<212> DNA
<213> Homo sapiens

<400> 52

ccgcgcgacc atgcaatggc gagcgctcgt cctggggctg gtgctcctcc ggcttggcct 60

ccatggagta ttgtggctcg tcttcgggct ggggcccagc atgggcttct accagcgctt 120

tccgctcagc ttcggcttcc agcgtctgag gagccccgac ggccccgcgt cgcccacctc 180

ggggcccgtg ggccggcctg ggggggtatc cgggccgtcg tggctgcagc cgccggggac 240

cgg 243

<210> 53
<211> 200
<212> DNA
<213> Homo sapiens

<400> 53

aacttggctc gggcatccat cgctctggcc tgaactcagg ccccgccccc ggggcgtggc 60

ctagcgatct ggtggccgcc atttcgaagc tgaagaggtt aggcgacgct gacttgcttt 120

caggagaggc actcggtaat cgagaagaac cggctttcgg ccagccagga gtggccagag 180

gcctagaagg ggagaggaag 200

<210> 54
<211> 217
<212> DNA
<213> Homo sapiens

<400> 54

cgcgcgcctg cctcggcgcc ctcgcaaccg cggccaggcc aacctcagcc ggaaactaca 60

tttcccagca ggtcgcgcgc tcctcgacag ctctccaggc agtagaaact acatttcccc 120

gaaagctttg cagtgcgcag gcgcatcaat gaggtactgc aatgtcccgg accgtatagt 180

ttccaggacg tctcgccgcg cgcatgcgca gaaacac 217

<210> 55
<211> 277
<212> DNA
<213> Homo sapiens

<400> 55

tagccaaatt accagtgccc tggagccggt tgggttgagc gcccctgccc ggggaaggta 60

gcctgcgggc tcttgccccg agcccgcgga gcaggaggtc tcttttccaa gcgcactcac 120

attattcatg caaagttaat ccccgccgcg tcacggccgc ccgcccgagg atgtgcgcct 180

tccttggcca agccccgcag tctccaccct cctccgcccc ctcctcccgg cggcggggtc 240

ccggggagcg ccggcgactg ggcgtcgcgc gagtcct 277

<210> 56
<211> 230
<212> DNA
<213> Homo sapiens

<400> 56

cgcaccaacg ccgccgcctt cgccgggagc caagcccgcc gggccggccc ggtcccaggt 60

gggcagctcg gcttgcagct ggttggaggt ggcggccgct gcagccctgg cagcgggcac 120

acccctaagc atacgcaccc aacgcctcct ccctgagcca cgaggatgga gcagccaccc 180

cggcccggga ctggcgcaag gtaaggtgca gccgcctgtt gcttttcgca 230

<210> 57
<211> 227
<212> DNA
<213> Homo sapiens

<400> 57

cctagcgtgg gcacgcgcca ctgcggccaa aagacagaaa tctttttacc ggaagagatg 60

aaatagaaag caggagccag aggctgactt acagagccag tgtgcggatc agagttcctt 120

aggagcggct gctggcgctt gacgggcgga atccttgctg aagagaacgt caaggcccgg 180

gcttcgccct gcatcgaact tactgcctaa gaataagacc caaatgc 227

<210> 58
<211> 319
<212> DNA
<213> Homo sapiens

<400> 58

caagcccgcc ttcctgaaga aaaagcccag tcccggcagc gttcttctcc ggctccgccc 60
ttcttccgct cgactttctt tgccattggc tgacaacgga gtacataagg acgtcatttc 120
ctgccgcctg tcttttccgt gctacctgca gaggggtcca tacggcgttg ttctgggtga 180
gttccgtgta gcgtccctgg cgccttccag ggctagaaaa atgagctttt cctgctcaaa 240
tgaagggtga gaagactagt gatgaaagcc ggtcagactg gatctgtctc ccgcccggcg 300
cgccccacct taggcctgc 319

<210> 59
<211> 205
<212> DNA
<213> Homo sapiens

<400> 59

agagtccagc cggtcttccg cggcacttac gcgctcgtgg gtccaccgcc gggcagaacc 60
gaaactttct ggggccaagc ggaagaggac gggaacgcgg tgctgacggc catgacgcgc 120
acaccaagag ggtggcgggg gcggactaca actcccggcg tgctccccgg ggctgaggcc 180
tgcaactccc atcggactcc cgggg 205

<210> 60
<211> 218
<212> DNA
<213> Homo sapiens

<400> 60

ggaaccactc aataaacacg tttaccccctg caagcggcac atcagagtcc gggggtaatt 60
ctcggtgtcg tggggccagg acggcgaggg gctggaagag gccgccctgt gggagctggg 120
aggctgagat aaattcccgt gattgggggc tgaaatggcc tcccatgccg gactgccgtg 180
gttctagaac tttttcctgg aacaggccgg cactccca 218

<210> 61
<211> 205
<212> DNA
<213> Homo sapiens

<400> 61

actcgcggcg gaggcaagcg gcggcgcgcg gacggttggt ccagttctcc ggcctggcgg 60

caggcaagtc tagctcggcg ctgtcggata cttggggtga gcggaaagca tggcggggac 120

ctccgcgcca ggcagcaaga ggcggagcga gcccccggcg cctcgccccg gcccgccgcc 180

gggcaccggg caccccccga gcaag 205

<210> 62
<211> 230
<212> DNA
<213> Homo sapiens

<400> 62

gagggcgggg aacgacgtgc gcgcctccga gtgcccagcg ggctcgagcc ggcacaggtc 60

gcggccccgc gcacgcgcgc gcactcacag ctcatccact attgctcagg agcccgtcca 120

cgccctaaaa gcaaaaccct taaccaatca ttgcagccgc gtggggcatc acgggaactc 180

cggggctata aaagacctgg ttgctatttt gtactcccgg agtcactcat 230

<210> 63
<211> 232
<212> DNA
<213> Homo sapiens

<400> 63

tccgaggcct gtccttatga cgcctgcccg ctgtggcact ccggttaccc ggaggagagt 60

tgctactctc gagttcaccg aggcggaaag cccctctgac tccaaagcag ggctcagcgc 120

gacgtcccag aggccgcttc tagtggcagg tgcccctcct cacagggccc tcgcagcccc 180

gccggccccc gcccggctcc ttctcagcat gatacaaccg cgtcggccct gg 232

<210> 64
<211> 316
<212> DNA
<213> Homo sapiens

<400> 64

tccacctcct ccaacgaagc ggaacacgtg acaccaccct atccacttcc ggcttggagg 60

gcatgccctc ccgtcaccag catgcacttt cgacaaaacc gtggaatcta gtattttcct 120

ctgacacctt tatattttct tcacttccct ataattctga cctatcgtgt aggtaaaaat 180

taatcttcag agtattacag acatgcgtgc ccacgcgcta ggcgaaagtg cgagtcggaa 240

gcacttcatt ccctctcttc gctagccgga agtcgcgaga tctgaatgag tcaaagccgg 300

cggcctcggc tcctca 316

<210> 65
<211> 201
<212> DNA
<213> Homo sapiens

<400> 65

ccgccgccgc ccgcggtccc tgcctgcccc aggcccgggg catcgccgcc ggccgccgac 60

tccgcgccct gcccgatcgg ctctctcctt tttaaacgga aagcagcctt tctccgccga 120

gaggatcgtc cccagcgtgg ctctgcgttc ccggtcactt tttgagattt tccgggggc 180

gctcggcggc ttcccggatt c 201

<210> 66
<211> 279
<212> DNA
<213> Homo sapiens

<400> 66

cgcctcggcc gcccaaagca ctgggataac aggcgtaagc caactggccc ggcccagaag 60

aagtttttag gatatatgga ggaaaacaca acacaacaca gctacaactc cgtgacaact 120

gagagcgtag tcttcgagta cccagttgcc tactgacagc cgtctcctct aaggcgcttc 180

ttttagccaa gaaagcgttc tttgcgtgct gtgacgtaaa aagcagcgcc ggaagggaga 240

gggcggaact tccggctgag ccccagcgac cgctctggt 279

<210> 67
<211> 306
<212> DNA
<213> Homo sapiens

<400> 67

gatcccgcag cagctgatga ccaccctggg acccctgttc cgcaactccc ggctggcaca 60

gttccacttc actaaccgag actgctgctt gctcagtggg ctctgccgca tcatgggcaa 120

cggcttcgcg ggctgcacgc tgtttcccca catctccccc tgcgagatgt gcgtgcgcat 180

gctcctgtac tcttccaaga ggaagatctt catgggcttc atcccctacg accagagcgg 240

cttcgtcagc gccatccggc aggtcatcac tactcgcaag caagcagtgg gacctggtag 300

tgtcaa 306

<210> 68
<211> 309
<212> DNA
<213> Homo sapiens

<400> 68

agagtagctg cggtgcctgc gtcgtctccc gtgcctctga gtgctctccc gggggctggg 60

cctgcggggc ctctcctgac agcggctcct gaaaggaggg tggctcggcc gccagcgtgc 120

ctcactggat gaggggacat cgcttaggct gtccctgtct gaccagtgta tgggtgaccc 180

attcagccta gagctacggt gccttccact ccacgatggg tccaactccc ttctttccaa 240

tgcccaagac tttggccccc ggtcctggag ctcctggtgg aaatcagggt aatggtggtg 300

gcttctccc 309

<210> 69
<211> 197
<212> DNA
<213> Homo sapiens

<400> 69

cctcccacct gcgcccgccg tccccgctcc tcgtgcgggt gtacaagtcc ggcccccgag 60

tacgaaggaa gctggagagc tacttccaga gctctaagtc ctcgggcggc ggggagtgca 120

cggtcagcac ccaggaacac gaagccccgg gcaccttccg ggtggagttc agtgaaaggg 180

caggtgagct tcgggcg 197

<210> 70
<211> 203
<212> DNA
<213> Homo sapiens

<400> 70

tgctgctgca cttgctccag gagccccagg aagcccagaa gatgggcacc gggcaaggcg 60
tgttctcatt acagaacctg gggtcggggg agaggcgtca atccagggag ggggaccccc 120
ttccctgacg agaggtccct gggagtgcaa gaccggaaag gacgagagtc cccgggatca 180
cgaggagaat ccaggccact ttc 203

<210> 71
<211> 409
<212> DNA
<213> Homo sapiens

<400> 71

gagggaggca gcagagcggg ccatgcggca gcgtgaccgg gaggctggcc ggggcctggg 60
ccgcatgcgc cgtgggctcc tgtatggtag gtccagttgt ctgcctgccc gagggactgg 120
gaagctggga aggagtctgg gaggggagta gaaggtgctg gggatgaccg cagtagcagg 180
tgtctcttgc ctccccagac agcgatgagg aggacgagga gcgccctgcc cgcaagcgcc 240
gccaggtgga gcgggccacg gaggacggcg aggaggacga ggagatgatc gagagcatcg 300
agaacctgga ggatctcaaa ggccactctg tgcgcgagtg ggtgagcatg gcgggccccc 360
ggctggagat ccaccaccgc ttcaagaact tcctgcgcac tcacgtcga 409

<210> 72
<211> 220
<212> DNA
<213> Homo sapiens

<400> 72

tgttttgccg gaaacgtaaa agacagggac cggaactgaa tgggagtacc ggaagtgccc 60
agacgaagcg gacgctcaca gaaactgaag attctaactg cccacagaca ctgttttgtc 120
ggaaacgtaa aagaaaggga caggaagtga atgagagtac tggaagtgcc cggacgaagc 180
cgacgctcgc aggaactgag gattctaact gcccacagag 220

<210> 73
<211> 335
<212> DNA
<213> Homo sapiens

<400> 73

actgcgacag caaagagctg cagcggcggc ggcgtagcct ggccctgccc gggctgcagg 60

gctgcgcgga gctccgccag aagctgtccc tgaacttcca cagcctgtgt gagcagcagc 120

ccatcggtcg ccgcctcttc cgtgacttcc tagccacagt gcccacgttc cgcaaggcgg 180

caaccttcct agaggacgtg cagaactggg agctggccga ggagggaccc accaaagaca 240

gcgcgctgca ggggctggtg gccacttgtg cgagtgcccc tgccccgggg aacccgcaac 300

ccttcctcag ccaggccgtg gccaccaagt gccaa 335

<210> 74
<211> 197
<212> DNA
<213> Homo sapiens

<400> 74

taggcaagac taactcggtg ttgctcctcc cggcgctgac ttcgaggccc ggctatggac 60

ggcgagagcg aggtggattt ttctagcaac agcataaccc ctttgtggcg gaggcggtcg 120

attcctcagc cccaccaggt tctgggccgg agcaagccga ggccccagtc ctaccagagc 180

cccaacgggt tactaat 197

<210> 75
<211> 235
<212> DNA
<213> Homo sapiens

<400> 75

gccctagccc accggcgcgt cccgcagttc cgaactaaac gcgccaggcc gggaaggctc 60

gggctgtctg aagagaggcg tgacctcgcc tacaccagat ccaaggcgca cataccgaga 120

cacccctcaa cctcactcag cagtgccgtt tccaactccg cggcagagac agcgcctggc 180

gaccccgggg gtagatcccc accggggaaa agccgcctta aggaggagcc aactg 235

<210> 76
<211> 320
<212> DNA
<213> Homo sapiens

<400> 76

ggagcagctc accttcaagg acctggtgtc ctgtgcctac caggtggccc ggggcatgga 60

gtacttggcc tcccagaagg tgggcagggc ggcaggtgtg ggtggagtag gctgggccct 120

gccctgagat gctgggagca gcggggagag gtggagaggc ttcagccctg cctcccaccc 180

cttccccagt gcatccacag ggacctggct gcccgcaatg tgctggtgac cgaggacaac 240

gtgatgaaga tcgcagactt cggcctggcc cgggacgtgc acaacctcga ctactacaag 300

aagacgacca acgtgagccc 320

<210> 77
<211> 263
<212> DNA
<213> Homo sapiens

<400> 77

agaaagaaaa gaaaggacta gacatgtccc cattacctgc ctgcgctccc ggcgggtcag 60

gctgtggccg ttgaggatgc gccagagcat gcgtgccgcg atcttggtgt cgatccatag 120

caggcggaag ccatggtagt agtgcttcag ctcgtccagc acccgctgcc ccagggactt 180

cttcaccacc acctctgcgg gggggctgta caccgggccg ccttcctcca gcttcttgtt 240

cttgtccttc aaggacttga ggg 263

<210> 78
<211> 214
<212> DNA
<213> Homo sapiens

<400> 78

cagggagcac atggcaacgg gctgcgggcg agctggccag cggcccgccc ggggtcccag 60

cccttcgaag aaagggacat tcacgagctt ctatctctaa ttctctgtcg caggcatttc 120

ggccgtggaa ccccaggctc ggaggactgg gtgtgagcgc tgcccgggag aggctgacct 180

gccgggaccg gagtgcccgg ggacgctgtg cccc 214

<210> 79
<211> 242
<212> DNA
<213> Homo sapiens

<400> 79

gccttctccc ctgccccctc ttccctgcat tcaaggagcc gggcggcccc gggggatatt 60

tttagttgct tctttctcct tcacttttag ctttagcagc atctcgaaac actcaaagcg 120

gagaaagact gggggttgg ggggagctgg agagggagtg ggggaaaagt taagagtcgc 180

gcaaagaaaa gccggaggga gggcggagag ggaccggggg cgcgggtgga ggaggggaag 240

gg 242

<210> 80
<211> 220
<212> DNA
<213> Homo sapiens

<400> 80

ttccgcgaaa ttgacggaag agtccgggtg tttcttcttg tgctcttccc ggcaggtctg 60

cacgaagaag gcgtacgagg acattttgcc ccgcggcttg ttggggtctc ctttacccat 120

gttgacagat ccgcgtccac ctgacggggc cgagggggga gaggggaagc cggagggtcg 180

gcgcggagcc cgcagctgcc agggcgggcg ctggcggggc 220

<210> 81
<211> 214
<212> DNA
<213> Homo sapiens

<400> 81

cggagaacca cccacggggc tttaaaaatg ttggtgccca ccacctcccc ggaacagggc 60

ccgctctacc tcggtcgggg agcgcgggac ctcagcgttc ccttaacgcc accgtccgcg 120

ggtccgcttt gcgcaggcgc ggcgccccca ctcagtaccc gctccgggcg tggcatggtg 180

cgcaggcgcg atgtccccca ctgcagcccc gctc 214

<210> 82
<211> 296
<212> DNA
<213> Homo sapiens

<400> 82

cccgcccgcg gacaggcgag acgtgggccg ccatggccgc acgagtgacc gggggccagg 60

agagccagtc tctccccagc ctccacgacg tctggggctc ccgtggggcg agcccttgct 120

agtgtcccat gctgagtggc gggtagggga atcggaattc cttgtcctag gctgggagcc 180

gtgtgggggc tccatgtttc gggctggagg agctgggcga gtgggagggg ctggacctag 240

acgtcccggg ctgggggtcc cggggagcgg gaatgcagta ccccggctgg atgagc 296

<210> 83
<211> 212
<212> DNA
<213> Homo sapiens

<400> 83

gggccctcgg cgctgcagga gctgcccaga aacttttccc tgctctcacc gggcggggga 60

gagaagccct ctggacagct tctagagtgt gcaggttctc gtatccctcg gccaagggta 120

tcctctgcaa acctctgcaa acccagcgca actacggtcc cccggtcaga cccaggatgg 180

ggccagaacg gacaggggcc gcgccgctgc cg 212

<210> 84
<211> 213
<212> DNA
<213> Homo sapiens

<400> 84

ccgtggtcct cgcacccttg cctctggtgc cgactagcac ccaacacccc ggtccacttg 60

tgcgagtgag agcgctcggc gaagtcttat atacggggag gcgggaaggg ggcggagact 120

cgatacgcga catcacgtca cggcctatgc cccgccccac ggccggccga gcgcgcgtgg 180

ccgggaagca agtcttgtcc cgccacccgg gtc 213

<210> 85
<211> 225
<212> DNA
<213> Homo sapiens

<400> 85

gcgaacgctg agcgttacgg ggtacgtggt ggcccctcag gttaccgacc gggtcggtta 60

gattccagag gacaaaaggc agggaacagc ctggtcttgg ggtctttaaa cagagaggcg 120

ccatctactg ccgagaaccc agtcaacatc tcatggacaa agctgcacta acccccggcc 180

ggctgagctc tgggccccgt tagcctttct ttgaggctcg tccac 225

<210> 86
<211> 202
<212> DNA

<213> Homo sapiens

<400> 86

actacaagcg gtcctcccgg ccaccgtact gttccgctcc cagaagcccc gggcggcgga 60

agtcgtcact cttaagaagg gacggggccc cacgctgcgc acccgcgggt ttgctatggc 120

gatgagcagc ggcggcagtg gtggcggcgt cccggagcag gaggattccg tgctgttccg 180

gcgcggcaca ggccaggtga gg 202

<210> 87
<211> 191
<212> DNA
<213> Homo sapiens

<400> 87

tcacattccg ctcccgcagg cctgcgggcc gccccagctg gtttgttccc ggcgtgttag 60

aggtcagagg ccgaggccgc actcagtgcc tgggtctcgc gcagctccag ggctctcagg 120

cgacactggg cgtttccttt ccggcttcgg aaagttttgt tttggttcaa ggaaaggcgc 180

cctgttgacc a 191

<210> 88
<211> 196
<212> DNA
<213> Homo sapiens

<400> 88

gctggaggag ttccagaaag ccaaggccgg ctccacaggc acccgacccc ggcctgaagc 60

gcaggtctcc tcctgaggcc cgacaaccac ctgccccgcc tcgtcctcgc ctgtgtcccc 120

cccgtcatcc ccgctccagg cgcccccggg gagccagcct ccgcagccac cgtcaagagg 180

aagccgaaga gccggt 196

<210> 89
<211> 216
<212> DNA
<213> Homo sapiens

<400> 89

agtgtagccg gagtgagagg aggggccccg gacgtagcct gctcttcccc ggtgtggagc 60

cattacgggg ggacattcct cctgcggcgg ggggagggag ccccctttct tttctttgag 120

aggtcagaat ccgcagtccg cccttcgcct ttaaataaaa gcctgccggc tcgagatcgg 180

cgtcgcgatg gagtgcccgt ggacttctac gcttgc 216

<210> 90
<211> 238
<212> DNA
<213> Homo sapiens

<400> 90

gggcgggccg ggggcggggg cgtggggcgg cccatttcct cctcctagcc ggactggagg 60

gagacaaagc agcgcccgtc tgcttcgggc ctctggaatt tagcgctcgc ccagctagcc 120

gcaggtaact tcgtacctcc ctcctcgcgg gcgcggtctc tcgggccagg ccgagagggt 180

ccttgggccg ggccgtccca agctgggccc cgcgagccgc gggcgggcga gcgggcgg 238

<210> 91
<211> 277
<212> DNA
<213> Homo sapiens

<400> 91

gtcgcaaaag gtatatatga ttaattgaaa gataagctgg aactatcacc gggaatgtca 60

ttaatgcgct ggggagacgt ccattggaga caggcggcgt tatccgcggc tttatcttca 120

acaacgcctc gctctcggcc cgcgcggggg aaacagatgg gagtttctgt ctgggacgct 180

cgccccatgt ttatattttg ggaagaatcg caactcgtgg gagtccccgg ctggcccct 240

gataaatgaa cattagcact ttttcggact actgtat 277

<210> 92
<211> 204
<212> DNA
<213> Homo sapiens

<400> 92

acgctttcat gggtggttac gcgacagttg ccaggaccga gggcgaaacc ggctgcaagg 60

cattgtggga cattggagga cttggaatat gcgctagaag gctcaatcca agcccattgg 120

aagaggaaga cgtacgcagc ggagacagct gatccggagt cggggcggcc tgccaggtgc 180

ccaacctctc ccagagcatg gcgt 204

<210> 93
<211> 215
<212> DNA
<213> Homo sapiens

<400> 93

ttggggaagc agctccacgg ggacccagga ggcgccggcc gggttgagcc gggttggttc 60

cgacccaaga gagctcgtcc cacgacggag caggtccctt tgcatcccgc ggggccgcca 120

ggtgcaattt tcgctgggcc gacggcgcgg agatgggcca gagtccggcc atccagaagt 180

gcctggagcg cacagcaagg ccctgccctc ggctc 215

<210> 94
<211> 208
<212> DNA
<213> Homo sapiens

<400> 94

ccgctcctcc tccctgtaac atgccatagt gcgcctgcga ccacacggcc ggggcgctag 60

cgttcgcctt cagccaccat ggggaatggg atgaacaagg taacgtcttc cctcgttcgc 120

gctgggtttg cctccgctcc gacgccctgc cgtctcgccg gcgtcggctg cccgacgact 180

cgagcccggg gtgccctttc ccgcggtg 208

<210> 95
<211> 242
<212> DNA
<213> Homo sapiens

<400> 95

caggtggaca tgcgagagcg tgtgtgtgcg tgcacacact ctgggggccc gggcgggggc 60

tggagggcac ccaaaagcag cagagcctcc tcacctcgtg gctccttggc ccgcggaggc 120

tcccgcttgc gccgtttccg agacggcttc acccatgggc tgtcttttcg ccatttcttc 180

ttggccttgc gccggccact ggaaccactc tgggaagggg gaggaggagg agttaggaac 240

cc 242

<210> 96
<211> 207
<212> DNA
<213> Homo sapiens

<400> 96

```
ccgccggctc gccccctgag gagggggctg ggccagggcc tcggctgacc ggggaggaag      60
aaggggagca gagaaaaaca tgagtcacag ccgtgtgtca ctggagcgca tttcaattcc     120
ctgcatcaca ggaggtgtgg aaggccgcct cggggaccgg gcgcgggagg tgcgcccgag     180
aaggccccgg gccggcctgc agggcgc                                          207
```

<210> 97
<211> 201
<212> DNA
<213> Homo sapiens

<400> 97

```
tggaaaggag tcggttgttt ggaggtcccc gccccgcggg gaagggctcc ggatccgacc      60
ctggtaggtg gggagcagcg gaccagccca aacgcggagc caatcccgcc aaagccctta     120
attgcacgca tctaagatgg ccgcccccgc ccggtagcgg ggcggaaacg agcccctcta     180
gcttgctagc cgagcccaca c                                                201
```

<210> 98
<211> 287
<212> DNA
<213> Homo sapiens

<400> 98

```
ccgggaggga cggggaagta gttagacaat agtcagggga ggttattccc gggacaaaaa      60
gcacctggca aagcgcgccg aggtcagtcc cttcgaatct ccacatttct gacaatggct     120
tcccagggaa tttgtttaaa cacccaagtg gcagacagca acaaacccgc accacaagca     180
acagagtgcc gtcacgggag tcgtaactac tttttctttc tctcttcccc actagcccgg     240
cgcaggccac gcgagacgcc gaagccagca ggtccccaca ctctccg                    287
```

<210> 99
<211> 245
<212> DNA
<213> Homo sapiens

<400> 99

ctgggtaagc gtccggcacc tgctcgccgc ggcgggaggg ccgcgcggcc ggggctgaac 60

ccgcgcctcc gcgcgtgggg ctttcgcgga gcgtcggtca tgggtgccgt tctggcgatt 120

gcgagagtcg cctcgggaaa ttgatgtggg atttgagagt cttggctcgg gcgtggacat 180

cagccgcctc ccctccggtc tctttcactc tccgaaacct tcctcgggaa ccgctgtcct 240

ccctg 245

<210> 100
<211> 223
<212> DNA
<213> Homo sapiens

<400> 100

acccggcccg gcttctgccg acagctgggc ggggacgcgc acgcagtccc ggcggggacg 60

cgcacgccgc cctctgaccc cgcagcgtcg tggctagagt agggtctgga gagagctccc 120

actgtggcga gtcctgtcgt tggattcccg cggcgccccg taaggctccg ccccggcggt 180

gctaccaaac cctgttcgat tttagaatcg tacattccca gag 223

<210> 101
<211> 193
<212> DNA
<213> Homo sapiens

<400> 101

gatctgggag gcttgtccct cgccgcccac cgtagccccg gcgctcggcc ggtcgccgtt 60

tccaagatgg ccgcggcgcg cacggctcct gcggcggggt agaggcggag gcggagtcga 120

gtcactcccg cacttcgggg ctccggtgcc ccgcgccagg ctgcagctta ctgcccgccg 180

cggccatgcg ggg 193

<210> 102
<211> 265
<212> DNA
<213> Homo sapiens

<400> 102

gcgccgcggg gaccccgaaa ctttccggag cgcgctgaga gcaaaaagcc ggctgggcag 60

aaagaggggg agggggctgg gcgcgccttc gagtgcgctg gggtagccgc ggcgccccaa 120

gttgaaccac agacgaaccc ctcttcatct gaaacctggt ctcggagtcg gaggtgggga 180

gggagtgggc tgtgggtgcc tcgacttcgc aaagccggcg tgggggggcg cagcgccccg 240

ccccgcgccg cttgaacctc cggag 265

<210> 103
<211> 205
<212> DNA
<213> Homo sapiens

<400> 103

gcgcaccggg gctgccgggg cgccacgtgg tgcgcacgtg gtccggcccc ggcgcggcag 60

ctcccctccc ccaccacgcg agcctggccg cagcgcccgc tggcggcgcg gcgttgcaca 120

ttgcgcgtgc gcacaactcg ccgcgaggcg ccttccggtg gcgcggcggg gtcggttgcc 180

tcctcgcgac ggctgcgccg ggatg 205

<210> 104
<211> 255
<212> DNA
<213> Homo sapiens

<400> 104

ccgtgcagcc tgggcgctac tctctgccgc gggcatggct ctgaggcccc ggctcggaga 60

agcagccgtg gccatgcagc taccgagagc gggctctggc ttaaccgcgt ttctttttaa 120

cgtctctgcc tgcggtgggg ggcattccta cgatgagcct gggaggaacc cgaggggctg 180

ctgccatggg cagaggggtc acgcccgggt cccgctgggc cggcggctca cccagcttca 240

ggagccagcc ctcgc 255

<210> 105
<211> 207
<212> DNA
<213> Homo sapiens

<400> 105

gaggcggcgg tggcggcgcc cacccccagc ggcaaggtgg accccgcgcc ggaacggcgc 60

ttcctggagc tgggcaacgg cctgggggag ggcgaaggcc cctcctccca cccgctgggc 120

ttccacttcc ccgtgcaccc caagtcctgg ctgcacccgg acagcttccc gatcctgggc 180

ctacccgact tccgagagcg gctgccg 207

<210> 106
<211> 230
<212> DNA
<213> Homo sapiens

<400> 106

gccggggagg gcccgcgctt ctcccggatg gaccgggacg ccggccttcc gggcccagga 60

ggcctcccat cccagtctcc cgcggcgcca ctgcggccct gcctcgtccg aacccctctc 120

cagggacgcg caggtcaggg acacccgtcc acctgcgtct gccatcgctt cggggcctgc 180

cggtgcccgg tcctcgttca gctgcagcgc caggcccagg cctgcactac 230

<210> 107
<211> 338
<212> DNA
<213> Homo sapiens

<400> 107

ggtctcagtt gcacagacca ggaaactgag gcccacagag gcaggtgtcc ggttgtttgc 60

aacctctcag cctgtgctaa ccccaattgt tcagagagag ccctgaaacc ctctcctctg 120

ggcgccccca ggtgactgcc ccagcctcaa gggctgcctc tgttgcagga aagacgacgt 180

cacaggctac ttcccgtcca tgtacctgca aaagtcaggg caagacgtgt cccaggccca 240

acgccagatc aagcgggggg cgccgccccg caggtaagcg gggtccccg gggctgggcg 300

gggtcgagcg gggcgcacca cgggttcgct ctgtctag 338

<210> 108
<211> 347
<212> DNA
<213> Homo sapiens

<400> 108

gcgagtcccc ggtgccccgc cgggatgggc ctgtgggccg gcaggactcc gggcggccct 60

acagcttggt gggctgctcg gggctgaggg ggccaggccc gctgggcggg ggcagcacca 120

tgggcagcga gttgctgccc ctctcgtgcc agcaggtgtc caggatgggg tagagcttgc 180

cctggaagtc ggcctggaag gtgtagagcg gccgcaggtc atcggggcgg tcggcatcga 240

agaaggtgag ttcgccctgc tcatagtgca ggtagagccc gatgcggtgg gggtggccgg 300

ccacgggcag gggtacccgg gggcaggcaa aggcttcgta cacccgg 347

<210> 109
<211> 248
<212> DNA
<213> Homo sapiens

<400> 109

accaaggcct ccggggcagc cgacggggcc gagcagagga gcaggctccc gggcgcctcc 60

cgacacatgg acagcacggt gggggacagg aggttgaggc tgaccagccg ctccccccac 120

agccaggcgt cgtccaggtg ggggtcaatg gcagagcccc gctcggggca gtagtccagg 180

ttgcactgct cgacgggccg gaagccctcc agccccgggt agaggcccat cctccgcacc 240

acctcccg 248

<210> 110
<211> 213
<212> DNA
<213> Homo sapiens

<400> 110

ccctccctag ttcccagccc ccagccctcg gtgacacttc cccgcccccc ggttgtcacc 60

ccgtctggtg ctcgcagaaa atgtcactgt ccccattttc cccgccgatc ccccaccacg 120

gccgcactcc cgcaccctct ccgctgctct tgtgggtgga ccccggaaag gtcaggcgtc 180

ctgggggca gtgcccctcc ccggaaagtt ggg 213

<210> 111
<211> 234
<212> DNA
<213> Homo sapiens

<400> 111

acgccccctt ccggccgagg cgcggaccgc gcggagctaa gtggaatccc ggttggcttg 60

gggcgcaggc ttccaacttc gtactctggc ctctggcgtc tcggctcgtc ggttgggtac 120

ccgaacccag ctactgctgc ttgaagagaa gatggatggg gactcctcgc cgtcgctgcg 180

ccgccggcct tccctgggcg gacgtacacc tttgcgaagc gtcagtgagg accc 234

<210> 112
<211> 276
<212> DNA
<213> Homo sapiens

<400> 112

gctgcttgaa gagaagatgg atggggactc ctcgccgtcg ctgcgccgcc ggccttccct 60

gggcggacgt acacctttgc gaagcgtcag tgaggaccca gggcccctcc ttggaatagc 120

tcttatttct caagcgctgc agcgtgaagc tcgctctgcg ggtccgagag gcctgcgatc 180

tgaagactga aaactgggaa gagacgctct accctgtgct cctcgccggc ttcgatagga 240

gccgcaggtg cctgggattt tctcaaactt tgtccc 276

<210> 113
<211> 192
<212> DNA
<213> Homo sapiens

<400> 113

aagcggcagc gctcgccatt gccgctggtg gcaggaggct gcgaggagcc ggcgcggtcg 60

cagtctccac ggcgcaggcc cacggtagcg cagccgctct gaggtgagtg ccctaccgcg 120

caatctccag ctatttcagc cccggcgcag ccgcaacacg ccgcccgccc gccggccggc 180

cggcccgccc cg 192

<210> 114
<211> 209
<212> DNA
<213> Homo sapiens

<400> 114

ggaagacctg gagccgagag gaggcggtgg ctgcgagcgc cacggagccc ggctgcagcc 60

gcgcgcgggc cccatgaatg ggttactacg gccctggctg cgggaggcgg agctgcggcc 120

cgataaggag cctcatttgc atggacgcgc gggattggcc ggcgcctggc aagccgggcc 180

gacgtcctcc ggcggcgcgc gccacgctc 209

<210> 115
<211> 221
<212> DNA
<213> Homo sapiens

<400> 115

ggccgggctg ggcgcttttg ccccgggccc cggaccccgg gcgtttgtcc ggtgcgctgg 60

cttaagaggc gctcagctca taccgtgatt gcacaactgg cttgctctcc gaactcggtg 120

gggaaaacgg tgacaaatac agattccagg tctccatcta gacccctgca ccggggagcc 180

ccaggcccta ggtccttcgc acacgcccct cccagagggg t 221

<210> 116
<211> 222
<212> DNA
<213> Homo sapiens

<400> 116

tacagaagca cacaggacaa atccattcat tctccagctc ctacacgccc gggaagcagt 60

tttactttac agcaagttca agagtcgcac cgcagatgct gtaaaaacga aagaagccca 120

cagttgggac gaggattcca gttttgcgtc accgtcaagg cgggaaagtt cccggctgtc 180

ggttggaggg tgggtcctcg ccccgccccg ccccctcggg cc 222

<210> 117
<211> 262
<212> DNA
<213> Homo sapiens

<400> 117

tcgccactct gtcgcagagg ggtccggcta gccacgaggc cccggccccc ggcagcccag 60

ccccttggca agcaccctcc cgccccctcc gcccctgccc gtggcgcggc gacatgggcc 120

ccatccaggt gctgcagccc ctcctccgct gcaggagaaa actctcctgt tttcccctcc 180

ctgccacccct ccgaagacct cctacctggt cccggaccac gcccccgtcc ccaactcccc 240

caacgccccg cgctctgcgg gt 262

<210> 118
<211> 219
<212> DNA
<213> Homo sapiens

<400> 118

cacgggaaat gaagcactgg gctctaccaa gagccacggg ccgggggccc ggggggggga 60

cgcgaccctt gcacacgccc cgcctggccg ccaggaccct cgacgcgccg tgccccgaca 120

cttcaaaggc agaattccgt tggagatgac cgcggaggcc gctcttcccc ggaggagtct 180

gcacaagtgc tgggaacggg cggcagcgcg cgcgccccc 219

<210> 119
<211> 221
<212> DNA
<213> Homo sapiens

<400> 119

agatagcaga aaagctcatc aaggcctttg ctgagcagat attttacacc ggcttcatcc 60

actcggaccc acatcctggc aacggtagga atttacccca ggggtggggg tctcggggtg 120

ggcgcagcgc gacctaagag gctgtatccc tagttctggt gcggaaaggc ccggacggga 180

aagcggagct ggtgctgctg gaccacgggc tctaccagtt c 221

<210> 120
<211> 255
<212> DNA
<213> Homo sapiens

<400> 120

cggggtcgtc ttcagccctc gcggctgtcc ttttggtgtt tctttccccc ggcatggggc 60

catttcttcc tgcagtgcct ggaggaacca tgatgcggtg tagacggcgg gtgcttaaat 120

aaatgcgagt tggattaggg cgcgtggact gccaggcaca gaacgtggcg tggagtgggg 180

acgtggagga gggagccaga caggccgggg ctgtaggcag gagcttgggg ttttcctctg 240

tgctccgcca gtaaa 255

<210> 121
<211> 204
<212> DNA
<213> Homo sapiens

<400> 121

tgaattagga ggttagggag cagtgcaaac aggaaacgag accctggccc ggtctttcag 60

aaacctaggc tcgagaagcc tgttcggttc tcagcatgtt tgagtgcttc tgggcgcggg 120

cggagcgaga aagcaagtgt agggtggcag gctccggagc cggaagaagc ccgttcaatt 180

cagcaacttt tcattaagca tttg 204

<210> 122
<211> 203
<212> DNA
<213> Homo sapiens

<400> 122

tgagaaagct cagactcagg gccagcaacc ccggtcccag cggagcgccc ggcacgcgcc 60

gacacttcag caccagtcgc ggtggccacc actgtgcgcg gagatggctg cgacgcgtgc 120

gcaggtaaag tccatccgtg ccttgcctcc caccggcgcc ttccaccgcc tctggttttg 180

tccccgccag cggctccgac tcc 203

<210> 123
<211> 229
<212> DNA
<213> Homo sapiens

<400> 123

ctggcctgag ggttcgcaga gggccagtcg ttgcgacagc cccgcgtccc ggcctcctcg 60

tctcttggac cttcacccca ggccagcgca gcccagcttc ctgggcaagt ttcacgctac 120

caggatccag tgcggggcga cgaagtggag agctgtatca agactccgaa gagaaattcc 180

ggcttcgcgg ggcggcgcgg agcccggagc tgcccacctc cgccgcctt 229

<210> 124
<211> 193
<212> DNA
<213> Homo sapiens

<400> 124

ccccgccagc cggaaatgtc cgcgcgctga aaagcagacg ctgcctgccc ggggaacggc 60

gagagagagc gagaaaaaaa aaagcctgaa ggcggcgcgg cagccagcga gaggggagaa 120

acagattcac acccaggggc ctccgggcag ggcccacggg actcaggccg tgtcccgctt 180

ggaatgtgga gca 193

<210> 125
<211> 192
<212> DNA
<213> Homo sapiens

<400> 125

cggccaaggc cgcgttcctc gcccgctgga gctcccgggg actccgggcc ggcttgggcc 60

cgcgtcagca cccagccctc gggagcgcag gcctcctccc cagaggtccg acttccctgg 120

gttgggcagc cgcggccact cccggccatc cctctgcggc gaagcccagg cgcccgtggc 180

ctgaggtcgc tt 192

<210> 126
<211> 453
<212> DNA
<213> Homo sapiens

<400> 126

actgcagccc cggcggatct gagcgtcatg gggggctcgg tcataaagcc gggccgcagc 60

gtgggagctg cacctcgggg acatcagggc gcccccaccc gcgggatccc tgagcgactt 120

ggggcactgg ggctgcaata cagaagagga agccgaggta gagaggagag aaaccactcg 180

acacagacaa tattcacccc agagctgaga agggaggggc tgcgtaatcc cagcgatgga 240

gcccaaggtt caggagccag caaaccagtc ctgctctgtc catcaccctc ggggacgggc 300

gctaggggcg agcgctcttt ggaacaacat attgcgggtt cccccaactg gacccccgcc 360

gagaatggta gtaagtgggg atccacgtgg tgagcgcggc cgccggagtt cactcccgac 420

gcatttccat agcgtgggga gaggctgtgt gag 453

<210> 127
<211> 241
<212> DNA
<213> Homo sapiens

<400> 127

gacgctcgcg cctcaggtac tggccgcggg ggcgcgcccg agccctggcc ggaggggagg 60

ggaggggcgg ggggtatttc tgaagcgtgc gagccacgct caggcgctcc cagacctggg 120

gccccgcgcc gccgccgaag ttctaacgcg gtctctggag ccgtctggcg cgcaaggagg 180

aggctacagg ccggggtcgt ctcgctggca gtcgctgtca gaggggtttt ctaactggat 240

g 241

<210> 128
<211> 205
<212> DNA
<213> Homo sapiens

<400> 128

ccccgccagg ccacagaccc cgcccagcgg ccagcacccg gcgcaggccc ggcagccgag 60

ctgcgcggcg gcaccatgca ggtcaccctg aagaccctcc agcagcagac cttcaagata 120

gacattgacc ccgaggagac ggtatgcgcg cgggccgggg gcaggggcag ccgcgtgcgg 180

gccgcgggga gcgccaggag ctcgt 205

<210> 129
<211> 212
<212> DNA
<213> Homo sapiens

<400> 129

acgaagagga ggctgacgct gacgaggaca cggtggcggc cactgactcc ggaggatggg 60

tcagcgaatt ggagagaata aagtccaggt ccaggagatc gttgaactcc tcggtctctc 120

tccgaggtag gggcgccagg ttgctaccgc cgcaagccgc accggctccg ccgctctcca 180

ggtctgtggc cacggtcgcc gccgccaggt ca 212

<210> 130
<211> 235
<212> DNA
<213> Homo sapiens

<400> 130

caaacaaatc ctccagctgc tggtacgggg aaggtgttca gtgacacacc ggaagcagcc 60

tagtggctga ggcgtccttc tccctaccaa cgggttgcga aggaagtagt tgctgtagcc 120

gcgccctgtg cgcctgcgcg gaggcgtcgg ctgctgacgt gtagctgggg ccagacggga 180

ctagccgggc gcgcggctga gtgctgcaga atcgctgggg tggcagagcc gccag 235

<210> 131
<211> 251
<212> DNA
<213> Homo sapiens

<400> 131

cctcccgccc tcgcgcccct tcggcccgtc ccgtccagcc cgggctgccc ggctctcgca 60

ggcccccccg cgccgaccgc gttcctatgg acagacgcac agacacctgc aggtgggtga 120

gagcccgcgc gcggggcggg gacgagtgcg ccgacgccgc gacccaggcg gggagcccct 180

ttgtgtgggg ggcggccgcg ccggggtcga ctctgcttcc ccgtcagcgg ccgcgcggtg 240

acccggctga a 251

<210> 132
<211> 223
<212> DNA
<213> Homo sapiens

<400> 132

ctctcgcccc tttgccgagt ctttgtctgg ccccagcccc gcggggcccc gggtccctgt 60

gccctcgggg gtccctagaa ggcgacaatg gctcgagtcc aggcgccgag gctggcgagc 120

gcctgcttgg cgcacagctg cccgttgagc ggcggcggct gctccgagga gtccggctgt 180

cggctcacca gccccgagaa gccggagcca aagatggaga tca 223

<210> 133
<211> 214
<212> DNA
<213> Homo sapiens

<400> 133

gtatttgagt gtgaagcgcc tagaaaacca caggacccct acggcgagcc gggaattttt 60

agatattttc ctccgagtca acgctcagtg aaatcagttc aatcagtggc ccgacaccgt 120

gcggctgaca cagcttatcc cccgaccctg agatcagggg tccccggagc ccaaggtcgc 180

ttccaaagct cagcgaggcg gaggtgcggc ccgg 214

<210> 134
<211> 430
<212> DNA
<213> Homo sapiens

<400> 134

ccagagacct gggccgggct gtgtgcaaga gctgacgggc actggtcacc gggggaacct 60

agcacccctt ggtcggcccc atcgcccccc agctgtgcct gcgatgcccc cttttttttct 120

aggggcctcc actccaaccg ctgtcccgca ctcttgcagg ccagcgtcag gccctccccg 180

ccaccctggg atctggaaac tcactctctg cagtttccat ctctgtgtcc cgcctgcaga 240

gcggcgggac tttctttgcc tggccgctgg ccctgcacgc acccccttcc tcgcgcctct 300

gccgcccttg ccgtttctgt cctcagtgtc ctgctggggc ttaccccgag tcccgcccaa 360

ggtgcagacg gcggcggccc cgggcctcgc tcggtcgcgc tcgagccccg tttccagcag 420

catcgcggcc 430

<210> 135
<211> 230
<212> DNA
<213> Homo sapiens

<400> 135

ggggctgcct ccctgcgcac cggccgcggt gactcagccc cggcccgccc gggcccgccc 60

gcgtcccttg tagttttggg aggtgatcag cgatgagcac ggcatcgacc ccacgggcac 120

ctaccacggg gacagcgacc tgcagctgga acgcatcaac gtgtactaca atgaggccac 180

cggtgaggcc ccggcccctt ccccgaccgc cctccgggga ccccgcggcc 230

<210> 136
<211> 284
<212> DNA
<213> Homo sapiens

<400> 136

cccatgagag gccggggtgg agaagccggg ggtgaagggg ggcaggggcc ggcgcgccac 60

gcggtcctta cctttccgct tggacctccg cctcctcctc ctcttggact tgcatttgag 120

ttgactgctc agccctccag accctctgcc gctccccagg accgaggcca tggccgagac 180

ccaggcgcgg agcccagagc cgcctccctc ctgcacctgc acctgcctcc ttcccggcag 240

ccgccgccgc cgctgctgcg aagcttgagg ttgcaaaccc ggga 284

<210> 137
<211> 226
<212> DNA
<213> Homo sapiens

<400> 137

ggcatccgta gaaccccccgc ctttgaccta gcttcaggag attactttcc gggtcagcgt 60

gcgtttaggg cgaagacgga gttgtaaact tcttaaaatt cctctctcga cacttcggta 120

attcctcttt cgagactaaa gctcttttttg tatgcgtgtg tgtcaagcgt atgccccggg 180

attctcctcc gcttcctttt ctcggtcttc cttcttgctt taggga 226

<210> 138
<211> 197
<212> DNA
<213> Homo sapiens

<400> 138

ccagcgcgcc cccggccgct cctccgcgcc gcgctcgtcg gccatggccc gggagaacgg 60

cgagagcagc tcctcctgga aaaagcaagc tgaagacatc aagaagatct tcgagttcaa 120

agagaccctc ggaacgtaag tggggaccgg ggaggcgagg gtggaggtgg ccgcggcagg 180

ggctgcacgg gcagctc 197

<210> 139
<211> 232
<212> DNA
<213> Homo sapiens

<400> 139

ggcctcagcg gctgaggagt gcctgtgagg cagaaggcgt ctcgcagtcc gggttcgatc 60

ccagctgcga gccgtcaggc ggcaggacct ggtctgctgc ctgcctgcct cagtttccac 120

gggagtgtgt gtgggtgtgt gtgggtgtgt gagggtgtgt atgggtgttg gcctgcgcac 180

accggagggg gggtcggtat acagtcggcg cctaatgcgc gcagcgcctc cc 232

<210> 140
<211> 241
<212> DNA
<213> Homo sapiens

<400> 140

gctttcagag agcttcgggg cctcgccggg gtcgctgggc ccgggccgcc gggccgcctg 60

ctgggggcgt aggccgactt gatgtccaga gagaaggagg gcttgaggcg gctggtgtcc 120

tgcaggtggt ccgaggagag gcgcaggctg cgcaggccct gctgcagcgt gctggtggcc 180

gtgggggcgc ccgggggctt cctgcccgcg ctcgggccgc cctccctggc agctgcactc 240

c 241

<210> 141
<211> 377
<212> DNA
<213> Homo sapiens

<400> 141

caagcccggg cggagcgcgc taagccacag gtccccgcgg cgttcctccc ggtcttcggg 60
gatgctaatg gcagcgaaaa tatttgctga agtgcaggca taaatcagcc gcctccccgc 120
tcccccaccc ccaccccttt tgtttcctct tccttctctc caaaagcgct gggctgctgg 180
gcgccagaat ttaccttttc catcttagcg gcctgattag catcgccgcc gcttgtgggg 240
ctggattcta ccctctcctg cgtcccccac ccccagtccc catctccacc tccctcccca 300
gccgagctaa atcggtctgc ggagccccgg gagctctaac gccaccgcct agcgagtgag 360
tgcacggagc cgccctc 377

<210> 142
<211> 333
<212> DNA
<213> Homo sapiens

<400> 142

agccacactg cctggagtag gatgttctca gggcccgggt tatttctccc gggttgcccc 60
aggagtgtgc agttcccaga aactcatggc aaagtcctct ataatctctt tccgagcagg 120
ggctctgaga agctctgcgc ctgtttaagg gttgggtggg tttggagggg tgaagaggaa 180
aaagagggag agagactggt ggacagttgt cctattctgg aattagtatt ttgccttgga 240
atttcatagc tgtaacgttg ccgacctcgc tgttggcgtt gcccgggctt ttcaaatatg 300
tagataagac tcaaggggc atccaggcct cag 333

<210> 143
<211> 254
<212> DNA
<213> Homo sapiens

<400> 143

ttggccctgc cctctagctc ccgcgctcgc tcccgccctc ctggctctcc gggcgcgcct 60

ggcggggcag ggcggacatg ggcaccaagc agaggagccg cacgcgcacg tactggggct 120

cggatctacc ttccagtagc aacggaggag ccaatgggac cgcgggcggc ggcgggggcg 180

ctcgggccac cgcggggcgg ttcccggctc aggtgcccag tgggcaccag cccagcgcct 240

cgggcggcgc tgct 254

<210> 144
<211> 221
<212> DNA
<213> Homo sapiens

<400> 144

acggtccggc cggggcgcgc ggagcctggc cccgggcgat ccatcctgcc gggttttcac 60

ggcggccaag ggggggcggg gctaggtggt ctctgagaac cgagcttgac tccgacgccg 120

cgaaccgacc tggagcccga ggggaaagat gctcgactct cttgggggca ccggagcggg 180

cgcaggagag gcctgcgggg tgcgtcccac tcacagggat c 221

<210> 145
<211> 299
<212> DNA
<213> Homo sapiens

<400> 145

cttgaatttc cgccggtaca gcgtggaaat gggcaggcaa agcgacttcc ggctcactcc 60

gcttcaagta cccgccgctt cttgctttgg tggggagagc aggcaccaga gacacgcatg 120

cgcatgtgcc agctggcctc aggcgcggcg ttcaaagtgt gcggggagga ggtcgagagc 180

ggagcatgcg cactggcaga cccttgctct ttcgctggag actatgcgca ggcgcggtgc 240

actgcgcgcc ggcacgaggg tgttttcgac gttggctgtg gggaaacagc aagcggcgt 299

<210> 146
<211> 233
<212> DNA
<213> Homo sapiens

<400> 146

gcccgcccgc ccgcccgcag cgtcagccct ccacttcctt cttccagccc ggcgcgcccg 60

cccaattcaa acgaaaacga agattcaaat tctggcgcta agcgctgcga ttgaacggga 120

aagcgcggca tgggaccaat aggaagggag cgcagggcgt gggcggggag ggggcgggga 180

aaccgggctc gcggggcggg gctggcgtgt ccgcgggcga gccactcacg cat 233

<210> 147
<211> 200
<212> DNA
<213> Homo sapiens

<400> 147

ccgaggggag ggcagacccg ggcagcgccc cgcacccccg gcggggaacc gggggcatct 60

ttcagccaca gaaagctgga gaagacagag gagctcctgg gaagcaggga ctgagcgaca 120

ggaaggggcc gagaagcggc gcgggagacc cggagaggga aaaggcactg gggctgaggc 180

ccccggcctg gtccgcgacc 200

<210> 148
<211> 285
<212> DNA
<213> Homo sapiens

<400> 148

ctagtgaagc tcaggggcag tagaatcaga caaaaaggac ccctggcccc ggcctagtgg 60

ccaggtcctc tccttcccag cgcatggagt taagacgctc catatgaagc ctagcctggg 120

atatccgtgg gtcttacatt tgctctcttc tgagctgctt cccccacact tccccgctcg 180

ctcgaaagac tccctatggc tgcgtcccag aacttgagtc catccgcccc caggccgggg 240

cactgggagg cccatcaccc tgtgcaatgt ccccgctcca ccttg 285

<210> 149
<211> 197
<212> DNA
<213> Homo sapiens

<400> 149

cccggggcac cggtgctggg gagcctcggg tgcctctgag ggcactgccc ggctcccaca 60

ggggcagcgg ctcctcgctc cgccacacac gttgtccttg ggctgttgag gggtggggga 120

gaggtccttg tggggacact gcgaagccgg tcccagacat ccagcctggc agtgtccaga 180

ctgcggggga ccggggt 197

<210> 150
<211> 255
<212> DNA
<213> Homo sapiens

<400> 150

cggcggcgct ggggaacgca ggccccgtgc gggcggctgc gcgcgaagcc ggctttgcag 60

acgcagcgga aggagccgct ggtgttcacg cagcgctcgc tcttgcacag cagcccgcgc 120

tggttcagct ctcggcactc gtcgatatct gaaggtgagg gcgacaggtg cggcttcgct 180

gagcctccag gcggctcctc accaccggcc ccgcccctgc ccccaccccc gaagcccggc 240

tcacccacgc agcgg 255

<210> 151
<211> 207
<212> DNA
<213> Homo sapiens

<400> 151

agcgggtcgc cctctcctgc cccggagttc tccttgacgc tcctcagccc gggcgctcga 60

gctcttccta gagtcgctgt tgaagaaggc ctgccaggtg acccagtcgc ggaacgcgaa 120

gaccatgacc acatcccacc tgtgagcggc gaggagccgg gaggggccgg cgggtttggc 180

gcggggagtt cacacactga ggaaggc 207

<210> 152
<211> 364
<212> DNA
<213> Homo sapiens

<400> 152

gagacaggag atggtcattg aagtggtaag aagtgtccca gtatctgtcc ggctgtcccc 60

acctcctcac tttgtcctct cttctgctca caccctctac ctgcaggtct ccctgctctt 120

tcaccccgag gggagccctg gtcgagtccc cactctgctc tgactctctg ggaagctggg 180

gaagagccct cactctccct gggtctcagt gtccccttgg gcactatggg caggggggtg 240

ggcttctgga ctgtgtccac ccagcctgaa ttctgccttg ggctccacag aaggcgatcg 300

gtggcaagaa ggaccggtct ctcttcctgt tcacggacct catcgtctgc accactctga 360

agcg 364

<210> 153
<211> 191

<212> DNA
<213> Homo sapiens

<400> 153

ggctgggacg ggcagggcag ggcaggcgcc ggcggggagg cgcggcggcc ggagccccgc 60

ggggaagtct ttccaggtcc cgactcggct ctctctgcaa aacggggcga acctcggcgc 120

ggcggccgac tcccgagctc ccggccgcgg ctccacttcc tcctcttccc ctgctccctc 180

gccgctcgct c 191

<210> 154
<211> 241
<212> DNA
<213> Homo sapiens

<400> 154

tgggggaaaa ggggttgtgt cgggtccctt gtcccctcgg cggaatcccc gggctgcacc 60

ttcgctgacc gttagcttca cctctgcagt gggtcatgca gtgctggcca tcaatggcat 120

ggacgtgaat ggcaggtaca cggccgacgg gaaagaggtg ctggagtatc tgggtaaccc 180

tgctaattac ccggtgtcca ttcgatttgg ccggccccgc ctcacttcta atgagaagct 240

t 241

<210> 155
<211> 271
<212> DNA
<213> Homo sapiens

<400> 155

ctggtgcgag aatggggtgc tggcccccct ccctgggggt ggcccggtcc ggaagcggcg 60

cgcggtggta gttccccgcg ttgctgcagc tgctgcattg cgccattttc cgtcccgcag 120

aatcgggagc ttggggcgag agttgtcaag gaggtgccga gcccccacac cagagtcaac 180

tcacattttt ctgacagaga gagtgagggt ctgttcctct ccggaggctg cggcgagaca 240

cccggtgagg gacgctgcgg ctgaagtgga c 271

<210> 156
<211> 238
<212> DNA
<213> Homo sapiens

<400> 156

ttcggggcgc cactggggcg gggccccaaa cttgggggt ggtcgtcccc ggcggcactc 60

tgcaaaactc catttcatcc ccaactcctc tgagaaccct cagaggcgaa ccgcgaggtc 120

tcaccattcc tagactgcca gatccaacaa tttctccgcg gcgacggtag cagccgcagg 180

gccaaagccg gcttccgtga ggtacgtcta cttccggtta acgaaccagg gggctggc 238

<210> 157
<211> 243
<212> DNA
<213> Homo sapiens

<400> 157

agcgccactg cccctgctcg cctatctgct ggcactggcg gctcccggcc ggggcgcgga 60

cgagcccgtg tggcggtcgg agcaagccat cggagccatc gcggcgagcc aggaggacgg 120

cgtgtttgtg gcgagcggca gctgcctgga ccagctggac tacagcctgg agcacagcct 180

ctcgcgcctg taccgggacc aagcgggcaa ctgcacagag ccggtctcgc tggcgccccc 240

cgc 243

<210> 158
<211> 243
<212> DNA
<213> Homo sapiens

<400> 158

tcgcctcaag agagcgggga gggggctcgg gggccgcggc ctgccctccc ggcgggcggc 60

tgagggcgag ggaggccctc ccttctggcg aggggaggga gggtgggtca ggagccccca 120

acccgccctg cggagctcgg ggccgcgcga ggggcggttg tctgggggag ggggcgcggg 180

gtgattcagc gcccggcgag gcggaagcgg ccgcaagagg aggaggggag agcccgtccg 240

cgc 243

<210> 159
<211> 249
<212> DNA
<213> Homo sapiens

<400> 159

cgggcaggcg gacgccctgg ggcggcgggc ggcggctgcc ggggctcgcc ggggctcggc 60

cgcccgctgc ccatcgatag gtattaggaa ttgatctcgc cgctgctctt tgttcggttc 120

agtcatcgat tagctgccgc gaccatggag aagcgctagt gagccctcgg tcggcggagc 180

ggccgagcgg ccaggcggcc gggcgaggag cggggaaggc aggggctacg gcctcgggca 240

gggggctgc 249

<210> 160
<211> 201
<212> DNA
<213> Homo sapiens

<400> 160

agccgctgtc gagtcccagc ggaaagaagc ccaaggtgcc ggagaagccc ggtcgtggac 60

gttgttgctc cctcatcttc gagacccgca cgccctgcgg agaggaaatg acaggcacgc 120

cgcgaccgtg ggcggggct cgcacgagct ccgggtttcc agaggcaggg gccggggccg 180

tagaaggcag agtcgcgcgg g 201

<210> 161
<211> 245
<212> DNA
<213> Homo sapiens

<400> 161

gggctccacc ttctgggtgt gtgggcctcg tagcgtgcgg cccattgccc gggcaaccga 60

cgccgtgagc ggtgcattgt ggtctgcaac aaaatgcagg agaaagatcc tgagggaacc 120

atggagtcaa tggaccaatg gaagaatgcc gtgggctgcg tcaagccaat agggtcgcgc 180

ggcgaggggc ggggccggcg ggagcagggc ggggcctgag cactaggcgg cggcggctgg 240

cgtgg 245

<210> 162
<211> 207
<212> DNA
<213> Homo sapiens

<400> 162

ggcctggcgt ccgtggggcg ctgtgcgtct ccgagaacga agcggcctcc gggttcgtgt 60

gctccgagcc cgagtactgt ttgcggccgt cgtcccgtcc tggagcagga gtgaggaagc 120

gcctctcact cccccgcgaa cccgcagaac aggccgccgg cgctatggag accggcggag 180

cttcccgctc cttcaccaag gggccct 207

<210> 163
<211> 223
<212> DNA
<213> Homo sapiens

<400> 163

ccccgcgcgc cccgcgacct gcttggctgc ccgcggctgc tgcacaagcc ggtgacactg 60

ccctgcggga tcacggtctg tgagcgctgc gtggagctgg ggcccgcgcg gccacaggcg 120

cgggcgcggg cgcgtgaacg tagtgctgag ccgcctgctg gagaggttct tcccggccga 180

gtgcccgctg cgcaggctgg agggtcaggc gcggagcctg cag 223

<210> 164
<211> 203
<212> DNA
<213> Homo sapiens

<400> 164

tggggcccgc ccgggggggg ctcgccgttg gcctgcgccc tcgcctttcc ggaggaggca 60

gggagctctg cggcggccgc ggcagcagta aatggcgtca tgtggatccg aggcggaaca 120

gagcagttgt tgtcccagag gatatatcgc atccggtccc agctcattgg ctccgcgggg 180

ctacattcac tcacactcca gcg 203

<210> 165
<211> 218
<212> DNA
<213> Homo sapiens

<400> 165

gccagcctct ccccaccacg tcccgggatg gtgggcgtcg ggtgggagcc ggcccaggac 60

atccaggtgg gatcagccac cgctcaggcc ctgtcccaag cggacgcggc cagaggcagt 120

ctcagcacca cgcaggccgc gggttcagtg ggcaaagaga ggggcccccg gtttagacca 180

aaaacgtgtg ttctccaacc tcctacttaa aagcccct 218

<210> 166
<211> 219
<212> DNA
<213> Homo sapiens

<400> 166

```
ttccgggtca ctgtctggct ccacccccc cccccccccc actgggttcc ggaaaggctc     60
agggttgctc ccgcgtttcg gttcagtgac gtcgtaaatt ggaatgaggc atccagttta   120
gcaacagcag agatgacgac tctgcgattc tgagagtccc tggcgagccc gggctagcga   180
aaagtggggg cagaacgaac tacatctccc atcgtgcca                          219
```

<210> 167
<211> 251
<212> DNA
<213> Homo sapiens

<400> 167

```
acaacaaata aaacaccctc tagcttcccc tagactttgt ttaactggcc gggtctccag     60
aaggaacgct ggggatggga tgggtggaga gagggagcgg ctcaaggact ttagtgagga   120
gcaggcgaga aggagcacgt tcaggcgtca agaccgattt ctccccctgc ttcgggagac   180
ttttgaacgc tcggagaggc ccggcatctc accactttac ttggccgtag gggcctccgg   240
cacggcagga a                                                        251
```

<210> 168
<211> 214
<212> DNA
<213> Homo sapiens

<400> 168

```
gcggcgcgcc gccgccccgt aggccccacg cgccgccccg ctcctccgcc ggatcgtctg     60
tgggtgagtc tcgagccagg aggctctgag ccagtggcga ttggctgacg cggtggctgc   120
gcactcggcc tgagaaactc ggcaagcgcg cagtgtcgac tccccggtct atgccaggcg   180
catctcaggt aaacggttgg gcagggaggt ttgg                               214
```

<210> 169
<211> 208
<212> DNA
<213> Homo sapiens

<400> 169

cggcgggctg cgcagagagc tggaagtgcg gccggaggcg ctgggctgcc gggcaggggt 60

ggagtccgcc ccgccgcgcc agcagctttc aacgcgcacg aaagtttggg gcccgccccc 120

tcgggcggtg ccgtccccgc tgcactccca ccgcgtcccg gcccctgccc caggccggag 180

ggcagaggaa gtgcccggcc cctcccga 208

<210> 170
<211> 246
<212> DNA
<213> Homo sapiens

<400> 170

tcccgggagc ggattgggtc tgggagttcc cagaggcggc tataagaacc gggaactggg 60

cgcggggagc tgagttgctg gtagtgcccg tggtgcttgg ttcgaggtgg ccgttagttg 120

actccgcgga gttcatctcc ctggttttcc cgtcctaacg tcgctcgcct ttcagtcagg 180

atgtctgccc gtggcccggc tatcggcatc gacctgggca ccacctattc gtgcgtcggg 240

gtcttc 246

<210> 171
<211> 199
<212> DNA
<213> Homo sapiens

<400> 171

cgctggacgc cgggcccagg ggactgggtg gagtctctgg gggagtagcc ggctgtctaa 60

ataatgctgc ctctctttgg tgtgactggt tatactttgt cttcttcata cggagtggtt 120

ccgttttgcg cctaggggcg taacccgccc gggagggaat ctggctgcgg cggaccaggg 180

ggcgcggctc gggatgcct 199

<210> 172
<211> 241
<212> DNA
<213> Homo sapiens

<400> 172

cagtaagaga attcaaaaca aaacaaaaca aaacaaaacc cagtttcccc gggcaggcac 60

tgaattccag ttttcgcgaa ctctggccaa aagtttgcag caagaagttt gcaaactcct 120

gcgcccgatc ggcaaaagcg gagggggag ttgcaagagg agcaaagttt gaggaaggtg 180

aattaaccaa ccggctccat gggctaagga ccgcagtggg cggcagaggc cggagcgagc 240

g 241

<210> 173
<211> 205
<212> DNA
<213> Homo sapiens

<400> 173

ggatcgccag ggcgcccagg ctgagaagcc ccaagccgcg catcccgccc ggctcttcca 60

ccacagggag acaggagctg ctggcatggg gactccgcag tcacctgtga tggctttgag 120

cggcaggttc tcagttctca ctcctgtgca gccgccggga catctgaccc cggttctgcg 180

acgctggcag cgcgaccggg ctcgg 205

<210> 174
<211> 236
<212> DNA
<213> Homo sapiens

<400> 174

cgccgagccc ctgggccggg cgctgaggcg ggcccctctg ggcagggccc gggcggggct 60

gggtgggccg cccctgctgc tgccgtccat gctgatgttt gcggtgatcg tggcctccag 120

cgggctgctg ctcatgatcg agcggggcat cctggccgag atgaagcccc tgcccctgca 180

cccgcccggc cgcgagggca cagcctggcg cgggaaagcc cccaagcctg ggggcc 236

<210> 175
<211> 252
<212> DNA
<213> Homo sapiens

<400> 175

gggcggccga tccccgaagt ccgggcttca ggacccgggc cggcagcacc ggctgcgagg 60

gttgccgaag gcgcacggat ctgggcgctg aaaaagccag gatttggcaa tggctttgct 120

gtgtggcctt gggcaagtca ctctgcgtat ctgggtttca cttccttccc aatccgaaaa 180

cgggattggg ttccttgcag cccgggcatt cctgaggtct cctttacccc ttctcctccc 240

ccaagccccg gg 252

<210> 176
<211> 227
<212> DNA
<213> Homo sapiens

<400> 176

ccagggcccg cagggcccac accacgtcgg gccggccaga ccgagcggcc ggcggactag 60

ccccccaaac gcctgagatt gggggacca aattaaaagc agctgtttaa catacttcc 120

gagccaggcg aggagtcctg aatgggggg aacgcccccc acggctatt ggctccccgg 180

cgctcacgtc agtcaccctg ggcccgcctc cgcccttcgc cccgcct 227

<210> 177
<211> 261
<212> DNA
<213> Homo sapiens

<400> 177

atactacagc tttccaagct cctctcggat ctcagagtgt tggtggggcc gggaaggaga 60

agggagaggg ggggcaccca gggaggcagg cgagcgctgg ggaaagaggg tggggagggg 120

ttccccttc ccccagcacg gtgcgcacta gcagcgcccg ctgcctttaa gaaaagatgc 180

ggatggcctg agtgaccgcg gtgtggcaga ccgggcactc gggctcgctc ttctcacaga 240

tgcgattggc gcactccatg c 261

<210> 178
<211> 288
<212> DNA
<213> Homo sapiens

<400> 178

tccagaccta ctccacacgg ccccgagagc aacaaccacc cgcaaacacc ggcgatgtca 60

ctccgcgtag ccgctccgcg agccgttgag ccccgcaaaa tttcaaaccg cgccacgggg 120

cgaagcctcg tcgcgattgg cgtcgcgtcc cgtcgctcca agaaaatccc cgcccctcgg 180

gctgaagtcg cgggtgcgca atcggggtgg ggactcgcga gcagggcctg ggaagcgccg 240

gagtggcggc gggggatgac gtcactgtct gtgagtggcc gggcgtgg 288

<210> 179
<211> 196
<212> DNA
<213> Homo sapiens

<400> 179

agacctgtgc gttgcggctt ctctgagggt ttcgattacg gaaaaagtcc gggtgacgta 60

caattaggct gccgcaggct gggaggcttg gcggcgtcat tccagtttct gcagcgaggt 120

ggcgctccaa ggtacggcag cgcgcccggg ttttcgccac cgagcgcatc cgccgccgcg 180

tccacgcagc gaggca 196

<210> 180
<211> 359
<212> DNA
<213> Homo sapiens

<400> 180

ccacccgccc cctaccctgc cactcaccat gttgtggttt tgttacctcc ggctgttacc 60

cgcccagtgg ggtattagtt gggccaggaa cacctgccct cagtgtgtgc cctcagtgtc 120

tgccctcctg ccctcctcac aggagcccgt gctcttaccc tccccctgtg ccaggagggc 180

atgacccctc acctcccagc gccctcactc ctcgctgaga ggggcaagtc tacgcaggaa 240

ggggatgggc ttgccctccc acatctcagg ctccagcggg caagggcaca agaatagtgt 300

agggttggcc gggtgcggtg gctcacgcct gtaatcccag cactttggga ggccaaggc 359

<210> 181
<211> 208
<212> DNA
<213> Homo sapiens

<400> 181

agcagtcgga ggacgcggaa ggcctgcgag agtcgcccgc gcccagcgcc ggccttcggg 60

tcccaccttg cgggtgatgt tgtgcacgta ggggcacgtg ttgcaggaga agcggtggca 120

gcgttgtccc tcctccacga tcagcccgtt cccgcagccg gggcagaaca gcagcatggt 180

ctcgaactcc gcaggctcca actcccgg 208

<210> 182
<211> 379
<212> DNA
<213> Homo sapiens

<400> 182

ccggcatctg ccttctcctt gccagtaaaa aagcaacact gcgggcctcc ggggatggca 60

gggagctgga acggggacac actgacttct gcaactcagc aagtatcacc tctgtcacct 120

ccgcctcccc gttggattct gcccagcccc tgggttctaa gattgctgtc ccattccaca 180

ggcaagccat gagactgggg ccatgcccag ctctgcgtct cccacccctg gtggcccagt 240

gagtagtgag ttcccagtcc ctgaggctac cccttccatc gcccacagag gtgaatgccg 300

atactcgccc agctgttcct gacgggcccc ggggtctctc tctgcccctc ttccctgcct 360

gccgcactca cagcatcag 379

<210> 183
<211> 191
<212> DNA
<213> Homo sapiens

<400> 183

ctcccacggt actgcctttc gcaggctgca cttgggggcc ctgctccccc ggagcccaca 60

ccccacgtcg agggccactc acgtatttca ggactgtctt gagccgtgcc atgcacagga 120

tggtgaccca gatggccgcc ccggagccca agaagtcgca gtactgcagc gtgtcgtagc 180

tgaggatgca c 191

<210> 184
<211> 215
<212> DNA
<213> Homo sapiens

<400> 184

gcgcgcgagg tcaggggaag gcggccttgg gcggtcgggg cagacgggcc ggtgaacagg 60

acgcgacctc cgcgatcagg ggcgcacgta ctgctgcacg aactccgcgt aggtgaggtc 120

ggcccgacgc tccaccgtgc agcgctcctc ctccgccacg gcccccggcc cgcccgggcg 180

cctgcgggca cagctgggtc agcccgcgcc ccgcc 215

<210> 185
<211> 269
<212> DNA
<213> Homo sapiens

<400> 185

gccttccttc aggtccgcag atgcccctcc atccggagtg ggccttgccc ggggttctgt 60

tgccgagtcc agattcgcag ctgtcttttc ctctagagtc aggagaattt ctggatttgc 120

gggcgccttc tcccctgtag aaatggtgac ctcaaggctt ctaggtcgca tctttcccga 180

gggcaggtac acttcgaagg gcctgcactc cttctgcccc ggggcgcccc ccgccagccc 240

ctgcagcctc cccgcggagc tggcgtttc 269

<210> 186
<211> 201
<212> DNA
<213> Homo sapiens

<400> 186

atttattaat aaattctagt ctctagcttg caaggaagac tacgtttccc ggcgtgcagc 60

gtgctggccg taagcattac gtcctccatc gcgggcgcgc aatgcacctc ggggatggta 120

gtgttctggt ggcccaacgg ttttaacagc ccgggaatca accaccggaa gtgccggccc 180

tgagaggtgg agtcggggtg g 201

<210> 187
<211> 234
<212> DNA
<213> Homo sapiens

<400> 187

agaatatgaa tggtgccgaa cccgcccccc gagctctttt ccccttgtcc ggcggctcaa 60

ctcaccacat gctccgcctc caggtcccgc tgcagcttct cgcggaggta ttcggcgctg 120

agttccatgg cggcagtcca gctggaacgg cagcccagca gggacacaac cccagctcgg 180

gcgccggcca cgctaccttg ctgccttaca ggagccactt ccgctggaaa actc 234

<210> 188
<211> 228
<212> DNA
<213> Homo sapiens

<400> 188

cccaactccc ggccccgcgc tcgagcccgc tttgtcgcag tgctgcatcc gggcactcgc 60

ggcgcgcacg cgctctgcgg gcccctcccc cttcgcggcg cgggtacccc ttccccgcct 120

cgtgttggtt cagctttctg tcgcgagacc cttcgcggaa gactcggcgg cgcgcgtccg 180

gtgtgagcct tgtccctcag tggtccttcg cgaatgggcg ggatcgct 228

<210> 189
<211> 305
<212> DNA
<213> Homo sapiens

<400> 189

cccttgagcc caggctctgc tctctggggg ggtgggggc gctccaagcc ggggagccgt 60

gccagccgag tcgtgcgggc tgtggcaggg aaggggccac catgggatgt actctgagcg 120

cagaggagag agccgccctc gagcggagca aggcgattga gaaaaacctc aaagaggatg 180

gcatcagcgc cgccaaagac gtgaaattac tcctgctcgg taaggaccgc cgctgctacc 240

cccatccccc gaccccggcc actccgcacc ccctgccacc agctccccca ccccactcgc 300

gcccg 305

<210> 190
<211> 246
<212> DNA
<213> Homo sapiens

<400> 190

agggcttact ccaaagccag tgggtcccca gtcctaagcc aagcagtccc gggggaaaac 60

gcttctcatc gccgtgggag cgcggatctt ggaagtggct ctgggctttc ttgggcgagg 120

ctctcacaga gtagatcgga aatccattct gctggcccgc cccacctcgg aggacggact 180

aacggacctg agttcccggc cctatcttac tcttctcagc ttctcagctt ggctcagctc 240

agagga 246

<210> 191
<211> 245
<212> DNA

<213> Homo sapiens

<400> 191

tttatagcac tcgtgacgtc aggtccaaat gagaaattga ctggcactcc gggccaatgg 60
gaggcggcga gagcggccgc gattagcata atagtataga aacaaggaaa cttttcggag 120
ctgtcaatca gggcccaatc agctgactgt cagctgggac gggctggctt aacccttcct 180
gcgccgctgc ctacccggga gggaacgagg cccggcgcaa agtttggtgt aagaggggaa 240
ggagt 245

<210> 192
<211> 228
<212> DNA
<213> Homo sapiens

<400> 192

cacgaagaca gcaatacaga tgttacctcg gctggtctca gcgcagcacc ggcgctggga 60
aacgacgctg cctgggatgg cgggcgcgtg ggaaggcccc gcggagtggg ggtgtgaagt 120
ggccgcgggg tgtcggcaga gacaggcgga agtggcgtcc cgatgaggac gaggtgcccg 180
ggcagccggg gacgggggtg cgaggggcgt gtgcttctcc tgttcctg 228

<210> 193
<211> 220
<212> DNA
<213> Homo sapiens

<400> 193

gttggagtcg gagagctcca gcacgggctt ggggtgccag gggaactccc ggcagtcctc 60
gtcgttcagc acctccaccc gcccctgccg cgtgatcacc tcgccctgcg ggtccagcat 120
gatgagcgtg gggatgcctg cagggaagag ggggtgcttg gccgctggcc cgggagatgc 180
tggccctgcc tggcaggagg ctcgcggggt ccagcccggg 220

<210> 194
<211> 221
<212> DNA
<213> Homo sapiens

<400> 194

cccggccagc gcgccccccg gtccccgagc ggacggagcc gcactcctcc ggctcctcgg 60

acttcctgcc tgggcatggc tcctccctgg ggcccacatg ggccttccta gggggcctct 120

caggggaggc cgcccgctcg ctcagggagg tgaagagcga atccgcgtcc ccggtgcccc 180

cgatggagtc ctttagggtg agccgcttcc gatagctcag g 221

<210> 195
<211> 215
<212> DNA
<213> Homo sapiens

<400> 195

cttgaaatcc tcccttaatg aagtcacacc ttctccgtcc gcttccaccc ggtcggccca 60

tggcagagcc cccatgtagc ccaggaatcg gcttggccct tcggggaggt cagcgtcctt 120

actgtcccca ctctccagtg cggcacattc gttcccctag gactccggcc ggttgccggc 180

cccaggcggt gcttctcccc accaccgccc agctc 215

<210> 196
<211> 241
<212> DNA
<213> Homo sapiens

<400> 196

ggccttggtc atggtaacgc cggctgagcg ggccccggct ccggggttcc ggcgggtgaa 60

aggagctgaa atgtcgcttg tcagttcagc ggcccccac ccaaacgacg acgcctgagc 120

cctccgtcgc cgccgatgcg agaccgcctc cgcccgctgg ccgcactccg agcctcgcgc 180

ctagctccca ccggtgccgc gacgacgacc acttccgtgt ccacgtcacc ttccgcgacc 240

a 241

<210> 197
<211> 208
<212> DNA
<213> Homo sapiens

<400> 197

tccgggccca tgaggcgacg aaggaggcgg gacggctttt acccagcccc ggacttccga 60

gacagggaag ctgaggacat ggcaggagtg tttgacatag acctggacca gccagaggac 120

gcgggctctg aggatgagct ggaggagggg gtgaggcccg gggtccccgg gggcccgagg 180

tgacagggcc ggggcggcgg cgcgggct 208

<210> 198
<211> 231

<212> DNA
<213> Homo sapiens

<400> 198

ctggggcggg ctggggaggg ggcccagggc tggggccggt tcggcctccc gggtggcgcg 60
cgggccgagg agctaggagg accgccgcgc cgcttgtcct ttggataaac cttggcggtt 120
cctcctctgg tgtccgtgga ccccgccgcg gcgttctcca gggccgcgga cctttgccca 180
ccggttgcgc ccgctgcccc gggcagaaag gacccccctc ctcccggacc g 231

<210> 199
<211> 257
<212> DNA
<213> Homo sapiens

<400> 199

ttggctcgcc cggcccgcgg gctccacctc aggttttcac ttttcgctcc ggagcgagaa 60
cgaaacgaca aaaacgcaag aaaacaataa aacgctagaa agcgaagtga acggcgctgt 120
gcgctctgtg ggtcgcgcgg ggctggggcg ctgcacgcgc gcaaggaggg acgagaggcg 180
gggacaaaag gctgcgcgcc cctcgcccgg cggcagctgc accccgggga cggcagggcg 240
gcacggcggc agggcgg 257

<210> 200
<211> 229
<212> DNA
<213> Homo sapiens

<400> 200

ccctagggat gttcttctgc cctccggaga cgtcggtctt ccctggggcc ggggctcaag 60
gccagggtgc cccaagccca ggctccaggg tgtgcgggga aaggctcggt cctgctcggc 120
gcggctcgcc ctgggcacta ggggtcgccc ctgcccagcg gaagccacgc gggcgcgccc 180
ggtcgccgca ggtttccccg gaccgggaaa agcgaggcgt ctccggggc 229

<210> 201
<211> 201
<212> DNA
<213> Homo sapiens

<400> 201

acagttctga cagtccaggt ggaggacccg cgctctgccg ggaaatcccc ggcttcccgc 60

tggggacaaa cggtggccgc cgcagggagt gctgtgtttg ggggtcgggg gaggcaggga 120

gccccacttt ccctcgcgct gcctcagtgc ccggcctgcg tgcgcgcgcc agcctggggt 180

aggaaggtac ctctgacatt t 201

<210> 202
<211> 202
<212> DNA
<213> Homo sapiens

<400> 202

gagccgagcc ggagtctggc tgctcccacc gcccgcacgg tgcccacgcc gggcgctgca 60

caagcagaaa gacggcactg agttcaagag catctacagc ctggacaagc tctaccccga 120

atctcagggc tcggacaccg cctggagggt cccggtgagc cgggaaggac tggacggaag 180

gggcgttttg tcaggctgct gg 202

<210> 203
<211> 227
<212> DNA
<213> Homo sapiens

<400> 203

tgactgtcgg ggtttggaca agtggctgcg acgacactaa ggtggagacc gggatgtagc 60

gaggactccc agagttggga gacgttgtac gcttactggg acctaaatcc cgcacgagtt 120

cccacccaag tgccgccgcc ccagcctacg cacgcgcatt cgcaccgtct ccagccccgg 180

ggtcctcctg cccctcccgc cgtctccggg ggccttttct ccgctcg 227

<210> 204
<211> 213
<212> DNA
<213> Homo sapiens

<400> 204

caccccaggt ctcccagcaa aggccgctcc tctggccagg gtcaattacc ggggtgtttc 60

gggcaccaag ttcccacact agtgccccat tgttacccgt ggccccctag tggtcggttg 120

cggacgtggc ctctttggtt ttgttttctc agcgggcggc ccccggggcg tgctcccgcg 180

gccctgccgc gtgctggtgc tgctgaaccc gcg 213

<210> 205

<211> 210
<212> DNA
<213> Homo sapiens

<400> 205

gctccccagg cccccctccga gagcgaggac gcgcccaggc ccgctctgcc ggagccgcca 60

ctggggggcg tagcgcggac gcgcaccctt gcctcgggcg cctgcgcggg aggccgcgtc 120

acgtgaccca ccgcggcccc gccccgcgac gagctcccgc cggtcacgtg acccgcctct 180

gcgcgccccc gggcacgacc ccggagtctc 210

<210> 206
<211> 203
<212> DNA
<213> Homo sapiens

<400> 206

acccctccgc tcccgggagc agagaagctt tggtttcatt tcccggcccc ggccgcggtg 60

gctgcctggg cgctgagcgg ggccgcgcgt cccgccagcc ccccaagctg cgtcgccgcg 120

gcttagagag gcccttccct ggccaccctc ccccgggggt ctcggagggg cagccccgga 180

gccggctgaa attaaagcct ttc 203

<210> 207
<211> 210
<212> DNA
<213> Homo sapiens

<400> 207

gactgggctc cctcccgggc ctcagttgcc tcattgctcc cgaaacaccc ggcattccac 60

atccaaatgc acacacacac gcacactcgc ggacacaagc acacgcgcgc tggcaaagcc 120

gcccgcgccg ctagtcgcac cgagcgctcg tttctttgtc cggatcgggg gcgctcccgc 180

cgccggggcc aacctgcagc ggaacaggcg 210

<210> 208
<211> 342
<212> DNA
<213> Homo sapiens

<400> 208

aggccccaga gctgacaggt ccttcccacc aagactccat ctccacgccc ggctgccctc 60
ctccgcccag gactcagagc gggtctctgt tggaagcgtg ggttcagccc cgaggcgcgg 120
caggaaggtg ggcacaggct gggctccttc cacagcctca gggtcggcct cgtccaagct 180
gttccaggga tgcgcgtggg tgaaggcccc gcctgctcct cactcccagc cgaggtgttc 240
gcctctgcgc gttccccctt ctcctgggtt cctgcccccg cctttctttc cccggagccg 300
tccacccccct cgccgggcgc cggctttcaa gcctcaaact tg 342

<210> 209
<211> 193
<212> DNA
<213> Homo sapiens

<400> 209

ctggacggcc gacccttgtg cttttcgggt tgaacctgtg gacgtgctcc gggcgcgctg 60
ttagttgccg ccccagacct aggaggcgag tcgggtcggc tcaggggagc ggggtttggg 120
ctcggagtct ccagtggacc tgccggactc gctcctcacc cctcaccctt tgccgtctca 180
cgcgtggcgt cat 193

<210> 210
<211> 296
<212> DNA
<213> Homo sapiens

<400> 210

ccaccggggg ctcctccgcc ggggcatcct gctccggggc atcgttcgcc ggtcttgtcg 60
ctgcggtgga agtgtcgtgt tcccagccag gctgggagtg gtcgctggcg aggcctggcg 120
tgggcccgcg gtgtctgtca gggtgccctg ggccagcccc gctgattcga tttagagcta 180
acgagagtcc accaacggca attacaggaa cctaatggcc ttcctgccgc accgctccgt 240
ggcacccggc tcgctgtgat gaattgctca ttatgtgcag tgttggaata gcctcc 296

<210> 211
<211> 210
<212> DNA
<213> Homo sapiens

<400> 211

```
ctgcaccggg tctttcaagc ccatttctca gacttggaaa cggcggctcc ggagagtgaa      60
gtcactctct cagggtctct ccgtctaagg ggcgaaggtg aaactccgag tccacgaggc     120
ccagcgtagg tgctccgtaa gaagggaaga gacttggtcc cggatctggc tccgatgcct     180
ccggccagcc cgcccaggcg gagccccagt                                      210
```

<210> 212
<211> 211
<212> DNA
<213> Homo sapiens

<400> 212

```
cccaggaaag gctttcccgg ctccctgtcg ggggctgacg gactcagccc gggcaccaac      60
cctgccaacg gctgcacctt cggcgggggc ctggccgcgg acctgagttt acacagcttc     120
agtgatggtg cttctcttcc ccacaagggc cccgaggcgg ccggcctgag ctccccgctg     180
agcttcccct cgcagcgcgg caaggagggc t                                    211
```

<210> 213
<211> 195
<212> DNA
<213> Homo sapiens

<400> 213

```
gccacctcct ccagcagcgg ccagttgtca atctccaggt gcagcagccc gggcagcctg      60
cggaagttct ggtcctccag ggaggcgatg gccaggtggc gcagccgcag ggcgcccagg     120
ctgcgcagat ggcccagcga ctccccggac agagccgtga ggttgcagcg ctccagggtc     180
agctcctcca gggcc                                                      195
```

<210> 214
<211> 227
<212> DNA
<213> Homo sapiens

<400> 214

```
cccggcgccg cgtcctcctc atcctccagg cgacaaggtc aggaggggcc ggggcggcgc      60
cccttccctc agcccccagc ccccagcccc ctacctgggt cttcccattc catcccgtgg     120
gggagggggc tggcgaggag ggcaaggggg agggggcggc accagggagg gcgggaccgg     180
gcgggcgggc ggggcgggga gctccggcag cgcccagccc cgccctc                   227
```

<210> 215
<211> 241
<212> DNA
<213> Homo sapiens

<400> 215

```
gtgcgcccat cacgtgcact cagcacacgt caagtgagca gccgtgatcc gggctacagc      60
ggggtgccct ggaatcccaa gggaggccac agagaggaga ggagcaggcg atggacatgg     120
atgcggaggg gctgctgctt cagagaggat gcttgttggg gggctcgggg ctccagctcc     180
tcagcgtcac ccggggacgc ctggcctgag cgctggcggt tcccgggagg gacacgcagg     240
a                                                                     241
```

<210> 216
<211> 348
<212> DNA
<213> Homo sapiens

<400> 216

```
cgcgaccaga gagaaaggca gcctgggagg gccagcccgg ccaacccacc gggcctgctg      60
atggccgagc ccacgctcga ggggctgacc agagttcaat gcgcctcaga aaactccata     120
aactatcccg tcttatttgg caaaagctca tatttttttc aaagtttgaa gacttgaatg     180
aaagagagga agaggagcct atgggaggag gccgagcccc agggcggctg atgcgagtct     240
gtccgcggag actgttggcg ctagtctgag tagagcccga gagcagactg gtggctcccg     300
ggcgccctga ggactcgctg ccctttgtag tgttggctga cccccccg                  348
```

<210> 217
<211> 190
<212> DNA
<213> Homo sapiens

<400> 217

```
ggccaggctc tgctgtctcg cgagggtccg gcctcaatgc tccaggcccc ggcgttgggc      60
cgcgcctcct cgtgggcctc ggaaggccag gatagccgag gtctccgcgt aggtcccccc     120
cagggaaagt gagcggctcc cgggccggcc cgccttctcc ccgcccccgg cggcccggtt     180
tcctccctct                                                            190
```

<210> 218
<211> 210
<212> DNA
<213> Homo sapiens

<400> 218

```
tcccctgccg gaaaatttgg gagagaggat cagggaaacc gccaatgccc ggaggccagg      60
cctttcacag gaaacagatt tcccgcgggt ttcgggggcg gtggcttttt gtgcgaggct     120
gtttctctgc gaatagctct tggagccaaa tcttgggacc cggaaccgcg tgggcttggc     180
gcatgcgtgt tgtaattcag gcgccgaccg                                      210
```

<210> 219
<211> 410
<212> DNA
<213> Homo sapiens

<400> 219

```
tgcaagcccg gcaggcggcg ggcgccgagc tcctgtccac ggtcagcgcc ggcccctcct      60
cggtcgtgtc cttgcaggag cgcggggggg gttgcgaccc caggaaggcc ctggccgcca     120
tccttttcgg cgccgtgctg ctggcggctg tggccctagc cgtgtgcgtg gcgaagctga     180
gctgacggac acccgacggc cgcctgctgc tgccgctccc tcccctgaga aaagactcgg     240
gatgggtgtg gggtctggcc tgtgcaaggg gagtggtcct aaaaccccgt gtgtgcatgg     300
gtacacgcgc gtttccagtg cacatctgcc tgggcaggac acggtttcct cttgctggcc     360
cgggaggagt taactttgcg ccggccgtca gggcattacc gctaacgtct                410
```

<210> 220
<211> 206
<212> DNA
<213> Homo sapiens

<400> 220

```
tgtagccccg ccccgggcct tgggctccac ctctcggccc cacttccgcc gggcaagcgt      60
ttgtaggcgg cgctgccgta aatcaggcgg tctgcttgcc gcatcacgca agatggcggc     120
cgcggcggtg aacggggcgg caggcttctc gagctccggg cccgcggcaa cctcgggcgc     180
tgttctgcag gccgcgaccg gcatgt                                          206
```

<210> 221
<211> 256
<212> DNA
<213> Homo sapiens

<400> 221

tctacttgga ggacgtgggc aactgcgagc agctgcgggg ggcagaggcc ggctcggtgg 60

tggatgagcg cggcttcgta tgggagaagg cggtcgaggg cgactttttc cgcgtgcagt 120

acagcgaaag caaccacttg cacgtggacc tgtggccctt ctacccccgc aatggcgtca 180

tgaccaagga cacgtggctg gaccaccggc aggatgtgga gtttcccgag cacttcctgc 240

agccgctggt gcccct 256

<210> 222
<211> 195
<212> DNA
<213> Homo sapiens

<400> 222

cgtcgggggc ctctctggcc ctacccaggc cgtgttctcg atagctttcc ggaagaaagg 60

gatctgggag cgagatgcgt gtagctagca cgatgcgtcg cgcggtgacg ctctggcccg 120

acgccgacgg cctctcagtg gctcccggag gacccggcgg gcccagtgtt ggaggtgagg 180

aggcggggct ggcag 195

<210> 223
<211> 212
<212> DNA
<213> Homo sapiens

<400> 223

tttgtttaga ttaagtgttc gcttagcggt gccctcacgc ttctgtaccc gggatgtggg 60

gggcggtaca gaccttgaaa tccccgcacc gctctctcca ccccgagtaa attcatgcgt 120

cccgtcagag tgttaaaatc gccctagggc tggttctgtc tccggtcgtt ctgccgtagg 180

gctgcgtagg cagctgcttt cgcgccgttt tc 212

<210> 224
<211> 356
<212> DNA
<213> Homo sapiens

<400> 224

gaggtgcggc ggtgtgcttt ttctctaggg tttgggttgg atggtggccc gggccttccg 60

agtttccatg agtaagctaa agacgttagg aaacagagca gggtggttga acgggagtgc 120

agcacggttg tgggggcaga tactgactat gagagcgttg gaggttattc tcgcgagatc 180

ggatctgggc tccgcgaggt tttggcgtag ttgtgggact gcgcaggcgc cgtttggagc 240

ccttacgctc acacttctct cccgcgcagg cgcagacggg gaagcggagc caacatgcca 300

gtggcccgga gctgggtttg tcgcaaaact tatgtgaccc cgcggagacc cttcga 356

<210> 225
<211> 219
<212> DNA
<213> Homo sapiens

<400> 225

gcagtctcgg tctcccggag ctcttgggag cctggccccg cccctttccc ggtgattgat 60

gtgtgctgcc tctttctggg tgatttacat gtaatgcccc gcccctttcg ggatgattac 120

gtgtgcctcg acctttaggt gatttacatg tagtgccccg ccccgttccc ggtgatttac 180

atgtggtgcc ccgccccttt cctggtgatt tacgagtgc 219

<210> 226
<211> 308
<212> DNA
<213> Homo sapiens

<400> 226

ggaggggaga gggatggcgg tgcgcgcgca ttcaccgcct ccctcccgcc gggtctggct 60

ttctccctcc tgtggccgaa gctttcctcg gagaaataga agagggaggc cgcgactcta 120

tggtgatgga cggaggcctt acccaatgga aagaggagct gtcccaaggc caggcaatca 180

tatacgacta ctggagctgg cagagcccgc cctctttcca cttggacctg aataacccga 240

cccaaaccga gtttcgcccg gagagactgc gctttcggcc aatgagtgcg tcgatttcga 300

gccccagt 308

<210> 227
<211> 261
<212> DNA
<213> Homo sapiens

<400> 227

cctgcgcggg tctcggaatg tccaggggtc cctactgggg atcacaaccc ggcgaaatgt 60

cgcatttgcc accacctatc tcgactgcgt aatcggcttt cagccgcaga cgctcaggtc 120

ctgcagcacc cgcggggatc cgtgtaggaa cctgctgccg ctggatggac tgagcgattg 180

cttcgcgggt gtcacgcacc agacgccgtg ccgggagcgc cgggaagggg gtacgcgccc 240

caaccccggc cgggggaggg c 261

<210> 228
<211> 227
<212> DNA
<213> Homo sapiens

<400> 228

tctcttcctc cggaccttcg gccacagagc cctcctcatc gttggtgtcc ggcaggtcta 60

agatgtcctt gaccgaaaac cccgtctttg tgttggtcag cgacatggtt cgagacccca 120

aaatttatgt cgcaaagttg tagcttcact tggtcaattc gtggcgctcc cctgccccgg 180

cgggcggggg aggggggagt tggggggagg gactggggga ggggagg 227

<210> 229
<211> 200
<212> DNA
<213> Homo sapiens

<400> 229

ggcgcggtca ctgaaacagg gccgatttct cgaaaaccac cccccacccc ggtcgctaaa 60

gacctccaaa agcgcccctc agcccaaaat ggagccaaac ctcgagaaag ctgagataat 120

acgcgggtcc gcaacgcccc gtacctgttc cggccttcgg gcgcctgtgg tgctcgtgtt 180

cgggaagaga ttgctgctgc 200

<210> 230
<211> 201
<212> DNA
<213> Homo sapiens

<400> 230

tccccggggg cgcagggtga gccagacccc ggccgcgcgc gctcgccgcc ggaggggtta 60

aagtggctgt tgtttgatat tctctccttt tctttttcca gatcgaagcg gatttccgat 120

tgaatggtga gtgtgccccc tgcgccgact ccggggcccc ccagccgccg gccgcctccc 180

ccgcgccctg ccggtgccgg g 201

<210> 231
<211> 249
<212> DNA
<213> Homo sapiens

<400> 231

gcggtgcgcc agtgggtagg tctagcagtg gcgcagcaat agagcgctcc ggagcgtctc 60

attggctgga tcaaacccaa gcgagccatt gattggtcga cgcccccaga gggttacaat 120

tcaaacgcgg gcgggcgggc ccgcagtcct gcagttgcag tcgtgttctc cgagttcctg 180

tctctctgcc aacgccgccc ggatggcttc ccaaaaccgc gacccagccg ccactagcgt 240

cgccgccgc 249

<210> 232
<211> 203
<212> DNA
<213> Homo sapiens

<400> 232

tgctggcctg ggttgtttcg ctctgggctg cgcattcatt tttagattcc ggcctcctcc 60

ctccccgcca tctcccgctt ctattcttag aaagcaaact ggggtgtgct tgggcttcgc 120

agggaagtca gacagaagtt tgcccacgtg ctccggccgc ctgagatatg gagttaatta 180

aaaaggaggc tttgagcggc agg 203

<210> 233
<211> 220
<212> DNA
<213> Homo sapiens

<400> 233

agggcgctag gcaggaccgc ggagctcggg cctctggaca tggccccgcc ggccgtcgga 60

agatgaggca gggatgagag acgacctccc gaccccgcga cagctccctc cgtgctcaga 120

agccgcagag agtccaccgt atctgcaacc tgcgggggac ctctcgggcc cggtgcgccc 180

taccettggt tcgccacctt gctcctcgag tccttgtcca 220

<210> 234
<211> 310
<212> DNA
<213> Homo sapiens

<400> 234

cgcactgccg ccaggacgca gtgcacctga gggcggagca cccacgcccc gggccacctg 60

actcactgtt tgtcaactct gctctctgca gccacgccct ttcctccttg cctccctccc 120

cttttggctc aggggcggca taaccccgcc tctctcctgc tgtgactggc tgtccttgcc 180

gtcactccgc tgcgtgggcg gggctctgag ggggcacagg tagaggccct tcgcgaaggg 240

attagcatag tcccaccccc cggcgcgagc ctcgggccgg gcgctgattg ggcaggcagc 300

ggccgacgcg 310

<210> 235
<211> 345
<212> DNA
<213> Homo sapiens

<400> 235

cagctgattc tgagcggacc gacggaagga gattcgcaga gccccttccc ggaagtgact 60

gttgtcgcgg caactgccaa aacacagacc aagtcgctga aggggagggc ggacctccca 120

atcgactggt ctggggcccg agatttccac tcagggagat acgtgggctg agatgggagt 180

tactagatgg ggaacgggaa atacggcggg aaccacaatc gcgcagttcc gcagctgccg 240

ccccacaacc gctccacatt ccagcgtccc ttccgccgct ttcagtctcc acttccggca 300

acgtttccac gctgtttccg gcctgggaaa gttcggcggc agcga 345

<210> 236
<211> 192
<212> DNA
<213> Homo sapiens

<400> 236

tggcacaaat ctgtaggttt tcctctgggg gtgggcggag gctccaaacc ggacggtttt 60

ctcctggagg actgtgttca gacagatact ggtttcctta tccgcaggtg tgcgcggcgc 120

tcgcaagtgg tcagcataac gccgggcgaa ttcggaaagc ccgtgcgtcc gtggacgacc 180

cacttggaag ga 192

<210> 237
<211> 290
<212> DNA
<213> Homo sapiens

<400> 237

gggccacgaa gacaaaggcg caaatgccgg gtcaggtggg cacagctccc gggagggtga 60

gggggacggc agggacatgg gggctgcagc tcacagtgaa ggtgggggag acagggcgag 120

agggcgttgc tggaggtggg gtgtgaggga cggcttgcga taccctggga ctgcggggta 180

gaaagcgcag ttctagggga ggtcggggtc agaggtgaag ctcgtgggga gggcgagacc 240

cgggcgcagg tgctcacagg cgagggcggg gttcaggttc cagctgggct 290

<210> 238
<211> 389
<212> DNA
<213> Homo sapiens

<400> 238

agggagcctc cagccagagg caccaggccc tagcggggga gaggcagccc gggcagaagt 60

gcctctggct gcaaccaatg agaaggtagc cctaaccaag tccactcctg gccagtaaga 120

ggcggtcttt aagcggaacc ctcccatctt tggccaatga gacgctgtct ggtcggagcg 180

ctccataact cggccaatgg ggagggagtc gcccgctaag cgcctgtcag gcctcgacca 240

acgggatggg cccttcccgc cagagcacac tgctccaatc caggagcggc tgcaggacct 300

gagccaatga gacgcaacct ccgctagccg cgcggtgccc ggccaatagg aggccgcccg 360

tgcccggtag cgtgggaggt gtggggtgg 389

<210> 239
<211> 345
<212> DNA
<213> Homo sapiens

<400> 239

ggctgctgac cgtgcccatc ctgtctccag cccactcccc caccgccccc ggtgccaccg 60

accaaacagc agtacctatg ccagccgctc ctggatgccg tcctggccaa catccgctca 120

cctgtcttca accattccct gtaccgcaca ttcgttccag ccatgaccgc cattcacggc 180

ccacccatca cgtatgtcca gctgggctgg gctttgcgga gggcggccag ccctgggccg 240

cgtgtgccag gtgtggtcac catgccgcct ccccagggcc ccagtggtgt gcacccggaa 300

gcgcaggctt gaggatgatg agcggcagag catccccagt gtgct 345

<210> 240
<211> 287
<212> DNA
<213> Homo sapiens

<400> 240

gttgggctta cagcgcggcc gatccggcgt ggacccggga tggctggacc gggcagcacg 60
gggggcaga tcggggctgc ggccctggca ggcggcgcgc ggtccaaggt agccccgagc 120
gtggacttcg accatagctg ctcggacagt gtcgagtacc tgacgctcaa cttcgggccc 180
ttcgaaacag tgcatcgctg gcggcgcctc ccgccctgcg acgagttcgt gggtgcccgg 240
tacggtgggc ttcatggggt cctgaggaca ggaagggcga tctgcga 287

<210> 241
<211> 290
<212> DNA
<213> Homo sapiens

<400> 241

ctccatggcc tcctgatccg cagtgtcatc tctccctgca gggtggaccc ggtgcctcac 60
gatgccccca aacctccagg ctacacccgc ttcgtctgcg tctctgatac ccactcgagg 120
acggacccca tccagatgcc gtacggcgac gtgctgatcc acgctgggga cttcactgag 180
ctggggctcc cgagcgaggt gaagaagttc aacgagtggc tgggtaggtc cctcctgccc 240
cgggcgggcg gctgtgctga gcaggaaggg ggctcccgcc agctcccgag 290

<210> 242
<211> 196
<212> DNA
<213> Homo sapiens

<400> 242

cgctcttcgc ttgcgcagtc agaacgtcct ccgcggccgt gtgaacatcc gggtcacagg 60
cgcataagga ccaatgagcg aaggcatcgc gagccagggg gcgcggagaa ggcggggaat 120
catggccgcc cccagtgttc cgcgtccggg ggtttgtggg agttgccttg acctgcagct 180
ccgccaccgc ggaccc 196

<210> 243
<211> 223
<212> DNA
<213> Homo sapiens

<400> 243

```
gcggcaacaa atccttgcag agttgcccgg gcccggccca attgcgcccc ggcatgccag      60
gcggagccct tggcgggttc ctgaggctct ccaggaggcc catcaggagc tactcctccc     120
accccgacgg ccactcacca gacacagaca cctccatgag cgcctccagc ggccggttgt     180
tgtccaggtc gcggctgagc tgcagttcgc ccgtggcggg gtc                       223
```

<210> 244
<211> 295
<212> DNA
<213> Homo sapiens

<400> 244

```
ccgaaacgcc acacacccgc tgctgcacat caacacgctg tacgaggccc gggaggagga      60
ggacgggggc ccccgcctgc cgcaggacgt gggggacctc atcgccatcc ctgccccaca     120
gcagatcctc atcgccacct tcgacgagcc gagaacggtc gtgagtactg tggagttttg     180
agggatggca ccgtccaggc cgccgagagc ccctctgcct gtgtcgtgtg gcctggccag     240
cctcccggtg gacaccagcc ctgcgtggac gtggcctgtg cttcgcccgc actgc          295
```

<210> 245
<211> 229
<212> DNA
<213> Homo sapiens

<400> 245

```
gtgggggaac ccagcccagc tgaaccaagc cgaggccgcg gcgccttccc ggagcggggc      60
cgcgcgactc acgttctcgt ggcgcatgtg cttgagcagg cgcagctcgc ggtaggcgcg     120
cttggcgaac agctcggact ggaaaggccg atacagcttc ttgatggcca ccttagcgcc     180
ggtgcggccg tccacggccg agctgcgggg cggaccgctt agcgggaag                 229
```

<210> 246
<211> 297
<212> DNA
<213> Homo sapiens

<400> 246

tgaaaaacaa ctcaggccat gacaggaaga ggggatctcg ggccgtgacc ggaagaggga 60
gtctcgggcc gtgacaggaa gagggagtct cgggccgtga caggaagagg gggtctcggg 120
ccgtgacagg aagagggggt ctcgggccgt gacaggaaga gggggtctcg ggccgtgaca 180
ggaagaggga gtctcgggcc gtgacaggaa gagggggtct cgggccgtga caggaagagg 240
gggtctccgg ccgtgacagg aagagggggt ctcgggccgt gacaggaaga ggggatc 297

<210> 247
<211> 359
<212> DNA
<213> Homo sapiens

<400> 247

acgacctgta cttgaacgtt gccaagtagg tgtgcccgtg ggcagaggcc gggctgtccc 60
tggtgcgggt gccatcggcg tggggtcgtc ccccaacctt accgcttacc gcagcaggcg 120
tcaacagtac gagtgtcttc actacaaaac ggatgctcag ggaacacgta tcgggtgtcg 180
tttcgatgac atctctcgac tctccagcgg ttctcaaagt tcccacatcc tggtgcgggg 240
caggagcgca gccttcggta tcccctgcac agataagttt gtcgtctttt cacagattgg 300
tgagtagccc gggacactcc ctcccaccct cagttctgtg ataccacggc tttagcgcc 359

<210> 248
<211> 448
<212> DNA
<213> Homo sapiens

<400> 248

ctcagttgtg ggctcctgcc gcaaccaagc taacgccagg cccagtagcc gggcctcggg 60
cacccgcgcc acgtcggggg cacccagtac agccctcagc gatgtagggt tgagctccag 120
gagtcccgcg gggcccgcgc cccgcgccag cagctcctgc aagtggctca cgatgcagcg 180
ctcggccgcg tccagcgtgt gagccaggcc aaagcgcgct gccacgttgg cggcgaagca 240
gcagttctct ggagccagct ggcgctccaa gtagcgccca cagagcccca gggcctcagt 300
gacctgcagg tagctggcgg cctccagagt gtcctctaca gtgtccatgg aaagcgacag 360
ccaggcagtg tagatgaagt ccagcaggcg ctgcaggccg gctgccgatg gcacgtgcag 420
gtggatcacg cgcgcccggg attcctgg 448

<210> 249
<211> 211
<212> DNA

<213> Homo sapiens

<400> 249

tcccacctca cggcccgagg cggggcttct ttgcgcttaa aagccgagcc gggccaatgt 60

tcaaatgcgc agctcttagt cgcgggccga ctggtgttta tccgtcactc gccgaggttc 120

cttgggtcat ggtgccagcc tgactgagaa gaggacgctc ccgggagacg aatgaggaac 180

cacctcctcc tactgttcaa gtacaggggc c 211

<210> 250
<211> 204
<212> DNA
<213> Homo sapiens

<400> 250

ccccacctcc gtcccaccct ccccgactct cctggcccag ggcccggccc gggaaggcgt 60

tgttttgcca gcgggagccg agcgagtgca cagagaaaag cctccctact ctaatgagag 120

tgcacacagc gctgcagcga ggaggatgat tacccggcgg gcgggggcg cgggctcggg 180

ccgcgggcgg cgtgtgcggc ggcc 204

<210> 251
<211> 216
<212> DNA
<213> Homo sapiens

<400> 251

ctcggcgagc ttgcccatct gccccggcag cagcaggcgc acagccatcc gggtctccca 60

ggcagtgatg gtctggcgct tggtggagcg ggccaggcga ccagcctcgg tggcgatgcg 120

gtccaatatg tcatgaacca aagaatccat gacactcacg gcctcccggg aaaggctgag 180

gccctggtga acctgcttca gcacccggcg gaaata 216

<210> 252
<211> 206
<212> DNA
<213> Homo sapiens

<400> 252

```
tcccgtgcca tgctcggccc aaggagtggg ccggccgagc acggggcccc ggtggccatc     60
acagtcatgg tggtgctggt cgcgctggtc tggcgggttt ggcactggga cttagagccc    120
tgggctgcct ccagcgcccc agtggccaca ctgatccgga tcacggcagg ggtgccagct    180
gcacaggccc gacgggcatc tcgctg                                         206
```